# EUROPEAN PATENT APPLICATION

(11) **EP 1 450 163 A2**
(43) Date of publication of application: **25.08.2004**
(21) Application number: 04010902.7
(22) Date of filing: 24.03.1993
(51) Int. Cl.: G01N 35/10

(54) **Syringe with bubble flushing**

(30) Priority: 27.03.1992 US 859218; 20.07.1992 US 916556; 20.07.1992 US 917253; 20.07.1992 US 915168; 20.07.1992 US 915166; 20.07.1992 US 917634; 20.07.1992 US 916425; 20.07.1992 US 916551; 20.07.1992 US 915164; 20.07.1992 US 915167; 18.03.1993 US 27269; 18.03.1993 US 27482
(62) Divisional of application: 93908571.8
(71) Applicant: ABBOTT LABORATORIES, Abbott Park, IL 60064-6008 (US)
(72) Inventor: Clark, Frederick L., Plano TX 75023 (US); Clift, Gilbert, Mesquite TX 75150 (US); Hendrick, Kendall B., Southlake TX 76092 (US); Kanewske, William J. III., Dallas TX 75208 (US); Lagocki, Peter A., Park Ridge IL 60068 (US); Martin, Richard R., Irving TX 75063 (US); Mitchell, James E., Lake Barrington IL 60010 (US); Moore, Larry W., Plano TX 75075 (US); Pennington, Charles D., Lake Zurich IL 60047 (US); Walker, Edna S., Chicago IL 60618 (US); Smith, Jane B., Vernon Hills IL 60061 (US); Tayi, Apparao, Grayslake IL 60030 (US); Vaught, James A., Euless TX 76039 (US); Yost, David A., Poolesville MD 20837 (US); Rumbaugh, William, Carollton TX 75006 (US); Winter, Gary E., Hanover Park IL 60103 (US); Hance, Robert B., Evanston IL 60202 (US); Stanton, Alyn K., Barrington IL 60010 (US); Oleksak, Carl M., Fort Worth TX 76118 (US); Watkins, William E. III., Cedar Hills TX 75104 (US); Vickstrom, Richard L., Algonquin IL 60102 (US); Cloonan, Kevin M., Round Lake IL 60073 (US); Wohlford Robert A., Irving TX 75062 (US); Hills, David B. Jr., Plano TX 75025 (US); Schrier, Paul R., Carrollton TX 75007 (US)
(74) Representative: Modiano, Guido, Dr.-Ing.

(57) **Abstract**

A bubble flushing aspirating and dispensing syringe having precision and volumetric accuracy, particularly for use in an automated, continuous and random access analytical system, is disclosed. The syringe comprises a piston (124) within a bore (128), the bore having seal means at a first end and a closed end (130) at a second end, wherein the piston forms an annulus (138) with the bore wall and seal means, and is capable of reciprocating therein. Fluid inlet (134) and fluid outlet (136) means are in communication with the annulus (138) and bore, the fluid inlet and fluid outlet means being located between the seal means and the bore closed end. A fluid source is in communication with the inlet means and a fluid conduit is in communication with the outlet means and an open-ended release tip. A drive means is connected to the piston for reciprocating the piston within the bore. As a result, fluid from the inlet, when connected to a fluid supply, flows through the annulus (138) around the sides of the piston and out through the fluid outlet to the open-ended tip, thereby creating a cross-flow pattern in the annulus (138) around the piston as it reciprocates in the bore to flush bubbles through the outlet.

## Description

### Field of the Invention

The present invention relates to an automated analytical system and methods for the analysis of liquid test samples. In another aspect, the invention is related to a continuous and random access system which is capable of simultaneously performing a plurality of assays, particularly heterogeneous and/or homogeneous immunoassays. In yet another aspect, the present invention relates to the various components incorporated into and utilized by such system.

### Background of the Invention

Although various known clinical analyzers for chemical, immunochemical and biological testing of samples are available, clinical technology is rapidly changing due to increasing demands in the clinical laboratory to provide new levels of service. These new levels of service must be more cost effective to decrease the operating expenditures such as labor cost and the like, and must provide shorter turnaround time of test results to reduce the patient's length of stay in the hospital as well as improve efficiency of outpatient treatment. Modernization of analytical apparatus and procedures demands consolidation of work stations to meet the growing challenge placed on clinical laboratories.

Generally, analysis of a test sample involves the reaction of test samples with one or more reagents with respect to one or more analytes wherein it is frequently desired thatthe,analysis be performed on a selective basis with respect to each test sample. However, due to the high demands placed on clinical laboratories regarding not only volume throughput but also the number and frequency of various analyses, there is a need to provide an automated analysis system which is capable of combining accurate analytical results, high throughput, multiple test menu versatility as well as low reagent consumption.

Typically, analysis of a test sample involves forming a reaction mixture comprising the test sample and one or more reagents, and the reaction mixture is then analyzed by an apparatus for one or more characteristics of the test sample. Reliance on automated clinical analyzers improves the efficiency of the laboratory procedures inasmuch as the technician has fewer tasks to performed. Automated clinical analyzers provide results much more rapidly while frequently avoiding operator or technician error, thus placing emphasis on accuracy and repeatability of a variety of tests. Automated clinical analyzers presently available for routine laboratory tests include a transport or conveyor system designed to transport containers of sample liquids between various operating stations. For example, a reaction tube or cuvette containing a test sample may pass through a reagent filling station, mixing station, reaction forming station, detection stations, analysis stations, and the like. However, such transport systems are not flexible in that transport is in one direction and the reaction tubes or cuvettes, once inserted into the apparatus, must pass through without access before analysis occurs.

Automated immunoassay analyzers have been provided such as the Abbott IMx® analyzer and the Abbott TDx® analyzer (Abbott Laboratories, Abbott Park, Illinois, USA) which utilize procedures involving a variety of different assay steps but typically rely on detection and measurement of optical changes in a reaction mixture during the assay process. For example, a number of well-known techniques using single or multi-wavelength fluorescence include fluorescent polarization immunoassays (FPIA) employing homogeneous immunoassay techniques, microparticle enzyme immunoassays (MEIA) employing heterogeneous immunoassay techniques, and the like. The MEIA technology, such as that used on the Abbott IMx® analyzer, is used for high and low molecular weight analytes requiring greater sensitivity, and FPIA technology, such as that used on the Abbott TDx® analyzer, is used primarily for lower molecular weight analytes. A front surface fluorometer is used to quantify a fluorescent product generated in the MEIA assays, while a fluorescence polarization optical system is used to quantify the degree of tracer binding to antibody in the FPIA assays. The test samples are automatically processed in the Abbott IMx® analyzer and Abbott TDx® analyzer by a robotic arm with a pipetting probe and a rotating carousel which positions the samples for processing. These instruments are compact table-top analyzers which offer fully automated, walk-away immunoassay testing capabilities for both routine and specialized immunoassays. These nonisotopic methods eliminate radioactivity disposal problems and increase reagent shelf life while meeting the diverse requirements of a multitude of different assays.

instead of loading the test sample into a container and obtaining sequential testing, such as one direction only systems as described above, the Abbott lMx® analyzer and the Abbott TDx® analyzer, often referred to as batch analyzers, permit the analysis of multiple samples and provide for access to the test samples for the formation of subsequent reaction mixtures. However, such batch analyzers permit only one type of analysis at a time. In a random access analyzer, not only can multiple test samples be analyzed, but multiple analytes may be analyzed from each test sample. Another common feature of presently available sequential and random access analyzers is the inclusion of various reagents within the apparatus itself or placed near the apparatus for pipetting purposes. Liquid reagents, in bulk form, are selected for the various types of tests which are to be performed on the test sample, and are stored in or near the apparatus. The reagent delivery units, such as pumps and the like, along with valves, control and pipette mechanisms, are included in these automated analyzers so that different reagents can be mixed according to the type of test to be performed. The Abbott IMx® analyzer automatically performs all the steps required for analysis of test samples and includes numerous checks of the subsystems to insure that the assay can be run to completion and that results are valid. Quantification of the fluorescence intensity in the MEIA method and polarization in the FPIA method, as well as the final data reduction, are also fully automated on the analyzer. Results are printed by the analyzer and can be accessed through suitable means for automatic data collection by a laboratory computer.

Automated analytical apparatus for performing homogeneous assays, the detection of precipitate formed by reaction between antigens and antibodies in a test sample-cell to form light scattering centers, and methods and apparatus for detecting immunological agglutination reactions are also known in the art. Such apparatus and methods include, for example, the steps of measuring light absorption of the liquid medium with antibody before and after the antigen-antibody reaction by using light which is absorbable by the antibody, and calculating the difference of the absorptions. In this way, the presence or absence of agglutination can be detected based on the fact that the agglutination reaction reduces the concentration of antibody, which affects the light absorption of the liquid medium. As is typical of methods and apparatus for performing homogeneous assays, these procedures do not require separation of a solid phase from the reaction mixture for further analysis.

Heterogeneous assays are also known through the use of a sample analyzer for quantitating relatively small amounts of clinically significant compounds in a liquid test sample by focusing a light source onto the sample so that, for example, fluorescent particles in the sample cause fluorescent conditions, the intensity of which is the function of the intensity of the light beam and the concentration of fluorescent particles in the sample. A detector senses photons forming the fluorescent emissions of the particles when excited by the light beam. The introduction of a solid phase material into the sample requires subsequent separation of the solid phase from the reaction mixture for further analysis and before the fluorescent emissions can be detected and measured.

Recently, apparatus and methods have been proposed for performing, selectively on the same sample, various homogeneous and heterogeneous assays concurrently in a random access fashion. Such apparatus and methods provide for the analysis of a plurality of liquid samples wherein each sample is analyzed with respect to at least one analyte utilizing both homogeneous and heterogeneous assay techniques.

The precision and accuracy with which the fluidics within an automated analytical instrument can be performed during assay procedures is closely related to the precision and accuracy with which fluids can be aspirated and dispensed by such instrument. Although a syringe or similar device within the instrument can provide such aspirating and dispensing steps, performance of such syringes previously described is often severely degraded by the presence of air bubbles in the syringe. Existing construction and designs of such syringes generally have no efficient means of removing such bubbles. For example, various relatively ineffective and cumbersome manual techniques and manipulations, such as abruptly tapping the syringe, and the like, are used to flush bubbles out of the syringe. Accordingly, there remains a need for a fluidics system which includes a syringe or similar device to provide precise and accurate aspirations, dispensing, and bubble flushing steps while avoiding the problems previously encountered by automatically flushing the bubbles completely from the fluidics system.

Fluorescent lamp life within optical assemblies of analytical systems previously have not had such demands as a requirement of quick lamp turn on times as well as long periods of shut off standby because much of the prior art has been batch versus automatic systems. However, in the present usage within continuous and random access analytical systems, light source means must be capable of quick turn on functionality in order to be responsive. Previously, warm up times for such light source means have been up to one minute or longer which is intolerable within a multi process automated continuous and random access analytical system. One past alternative has been leaving the light source means on during standby which significantly reduces the life of the lamp source, however, full shut off of the lamp cannot be tolerated within an automated, continuous and random access analytical system if the light source means cannot be reactivated within a very brief cycle. A solution has been developed which provides the light source means within the optical assembly with a heated environment during shut down periods.

Prior analyzers using tungsten filament lamps within optical systems generally turn the lamps off completely during nonuse periods since the systems utilized batch processing. Automated continuous and random access analytical systems require rapid accessibility to the optical system including performance of the tungsten filament lamp; however, if the tungsten filament lamp is left on full time, the life of the lamp will be very short. Turning the tungsten lamp off requires substantial warm up times, in order to ensure for example FPIA lamp stability by imposing a long warm up time prior to FPIA reads. Since this wait time occurs only once per batch, lamp life is not generally drastically affected. However the continuous access nature of the automated, continuous and random access and analytical systems demands that the FPIA optical reader be available on short notice. Without a change in methodology, the lamp would by necessity stay on full time, diminishing its life to just a few days. Accordingly, an alternative to these prior methods has been proposed.

Various functions of electronic device control stepper motors have utilized BIT control for the simple motor movements. However such control has necessitated a PAL-type device which is at the expense of not having ramping and error detection. These simple motor movements utilized in the past necessitated additional microprocessors or special purpose motor controllers and/or integrated circuits to be able to provide complex movements and ramping and error detection. Presently these more complex movements such as ramping or error detection are performed by microprocessors or special purpose motor control integrated circuits that require a parallel data bus or serial port.

Prior methods for reducing evaporation of costly reagents from system containers have utilized manual operations to cap reagent containers as well as use of various other reclosing container caps which are held open during liquid access cycle and then allowing the caps to reseal by removing the opening force. Apparatus and methods are now presented wherein computer-controlled robotic devices replace the need for manual intervention, said devices having the capability of minimizing reagent evaporation.

Presently, the diagnostics industry still utilizes several systems routinely which require hand loading of cartridges, reagent packs and sample containers into batch and semi-automatic instruments. Individual manual loading of any of these items is further complicated because of volume and reliable requirements of automated, continuous and random access analytical systems diagnostics. An automatic feeder is demanded by such diagnostics which incorporates the general principle of feeding tubular parts such as cartridges and orientating the cartridges with an open end up. An automatic cartridge feeder hopper of multiple cartridges saves substantial operator time and error since multiple cartridges can be loaded into the hopper feeder system directly from the cartridge packaging systems, eliminating hand feeding of the cartridges individually and assuring reliability within the automated diagnostic systems. Moreover, there is a need to provide various handling and loading means to facilitate the handling and loading of reaction vessels which are utilized with such analytical system.

Although analytical instruments previously described have employed voltage to frequency converter methods, such methods cannot read zero signal and provide only moderate noise rejection. In particular. such instruments require complex circuits to implement ratiometric measurements. Accordingly, since such previously described automated analyzers do not contemplate an automated analytical system for simultaneously performing both homogeneous and heterogeneous assays in a continuous and random access fashion utilizing a commonality of various process work stations and transfer means and a data acquisition system to implement ratiometric measurements with improved noise performance, there is a need to provide an automated analytical system having these features and sufficient flexibility to meet the growing needs of the modern clinical laboratory.

Accordingly, since such previously described automated analyzers do not contemplate an automated analytical system for simultaneously performing both homogeneous and heterogeneous assays in a continuous and random access fashion utilizing a commonality of various process work stations and transfer means, there is a need to provide an automated analytical system having these features and sufficient flexibility to meet the growing needs of the modern clinical laboratory.

### Summary of the Invention

The automated analytical system of the present invention is capable of simultaneously performing two or more assays on a plurality of test samples in a continuous and random access fashion. In particular, the automated immunoassay analytical system apparatus of the invention can be viewed as a microprocessor based system of integrated subassemblies with different groups of assays being run through separate and changeable software modules. The microprocessor based system uses robotic arm pipetters with two degrees of freedom and bidirectional rotating carousels to process samples. Critical assay steps such as incubations, washes and specimen dilution are performed automatically by the instrument as scheduled.

According to the invention, automated, continuous and random access analytical system capable of simultaneously effecting multiple assays of a plurality of liquid samples is provided, and enables performing a method wherein various assays are scheduled for a plurality of liquid samples. Through kitting means the present system is capable of creating a unit dose disposable by separately transferring liquid sample and reagents to a reaction vessel without initiation of an assay reaction sequence. From the kitting means multiple, kitted unit dose disposables are transferred to a process area, wherein an aliquot is mixed for each independent sample with one or more liquid reagents at different times in a reaction vessel to form independent reaction mixtures. Independent scheduling of such kitting and mixing is achieved during incubation of the multiple reaction mixtures, simultaneously and independently.

The system of the present invention is capable of performing more than one scheduled assay in any order in which plurality of scheduled assays are presented. The incubated reaction mixtures are analyzed independently and individually by at least two assay procedures which are previously scheduled.

The automated, continuous and random access analytical system apparatus of this invention is comprised of a front end carousel assembly inclusive of a sample cup carousel, a reagent pack carousel and a reaction vessel carousel mounted concentrically and serviced by a transfer pipetting means suitable for kitting and/or mixing reagents with a sample. The kitted and pipetted reaction vessels are transferred through a transfer station which provides means for transferring the kitted and pipetted reaction vessels to a processing work station 4 which includes a controlled environment for maintaining temperature and provides timing for mixing of reagents and incubation. At least two assay procedural apparatus are provided which are scheduled for the various samples and kitted reagents in a unit dose disposable means for analyzing the incubated reaction mixtures. The unit dose disposable reaction vessels are removed from the process carousel by operation of the transfer station, which includes means for removing the disposable reaction vessel from the system.

Additional advantages and novel features of the invention will be set forth in part in the description which follows, and will become apparent to those skilled in the art upon examination of the following or may be learned by practice of the invention. The objects and advantages of the invention may be obtained by means of the exemplary combinations more particularly pointed out in the following specification and appended claims, including all equivalents thereof.

### Brief Description of the Drawings

FIGURE 1 is an isometric view of the automated analytical system illustrating the system cabinetry, exposed front end carousel, computer screen and keyboard.
FIGURE 2 is an isometric view of the automated analytical system apparatus frame and cabinet.
FIGURE 3 is a top plan view of the automated analytical system in section with component covers removed to show the automated analytical system apparatus in detail and relative position.
FIGURE 4 is a front elevational view of the automated analytical system in isolation and partial section of elements of the front end carousel.
FIGURES 4A and 4B represent a perspective side elevational view and partial end view of a reagent pack and reagent pack cover means for use with the automated analytical system.
FIGURE 5 is a top view in isolation and partial section of drive and guide elements of the front end carousel of the automated analytical system being removed.
FIGURE 6 is a cross-sectional side view of a process carousel of the automated analytical system in isolation with two reaction vessels in place, one of which is in position for an FPIA read.
FIGURE 7 is an isometric view of the probe, probe arm and pipettor of the automated analytical system in isolation.
FIGURE 8 is a schematic side view of the probe arm wiring and sensor means of the automated analytical system.
FIGURE 9 is a cross-sectional side elevational view of an automatic bubble flushing syringe apparatus of the automated analytical system.
FIGURE 9A is a sectional side view in isolation of the syringe bore end portion of the automatic bubble flushing syringe with the reciprocating piston near the end of travel toward the bore end portion.
FIGURE 9B is a sectional end view in isolation of the piston and bore of the automatic bubble flushing system syringe taken along line 9B-9B.
FIGURE 9C is a partial cross-sectional side elevation view of an automatic bubble flushing syringe apparatus of the automated analytical system.
FIGURE 9D is a sectional side view in isolation of the syringe bore end portion of the automatic bubble flushing syringe with the reciprocating piston near the end of travel toward the bore end portion and a phantom position within the bore illustrating the piston withdrawal to the outward extension.
FIGURES 10 and 10A represent a top plan view of a reaction vessel and a side view of the reaction vessel for use with the automated analytical system, respectively, with reaction vessel compartments labeled where appropriate for FPIA processing.
FIGURES 10B and 10C present a top plan view and a side view of the reaction vessel, respectively, labeled and presented for MEIA processing.
FIGURE 10D is an isometric view in section of the reaction vessel loading device illustrating the device holding to vessels and means for mounting other vessels.
Figure 10E is a top view of the reaction vessel loading device presented in an arc which matches the radius of the reaction vessel carousel, the loading device having mounted thereon ten reaction vessels.

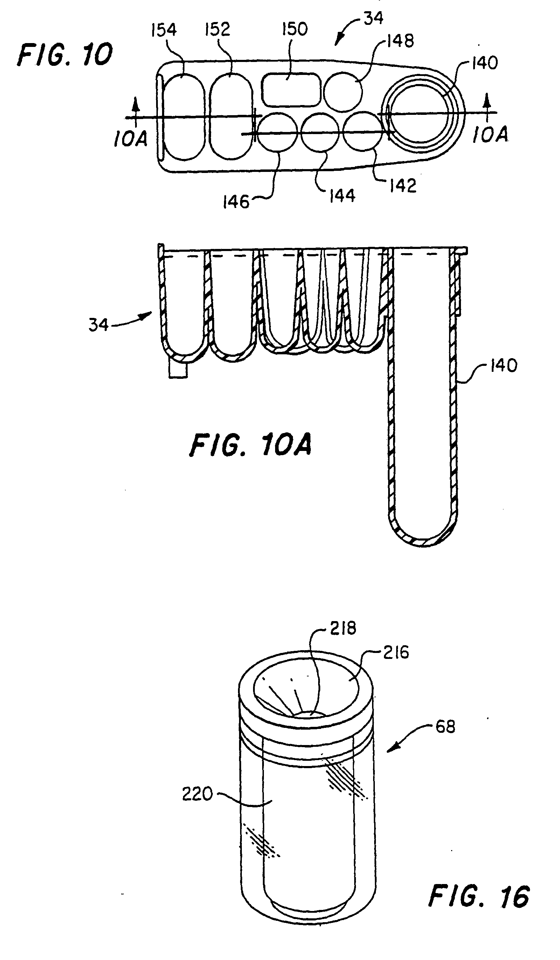

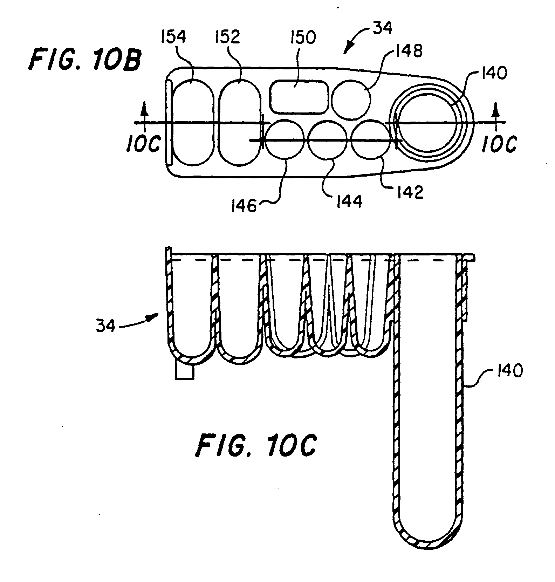

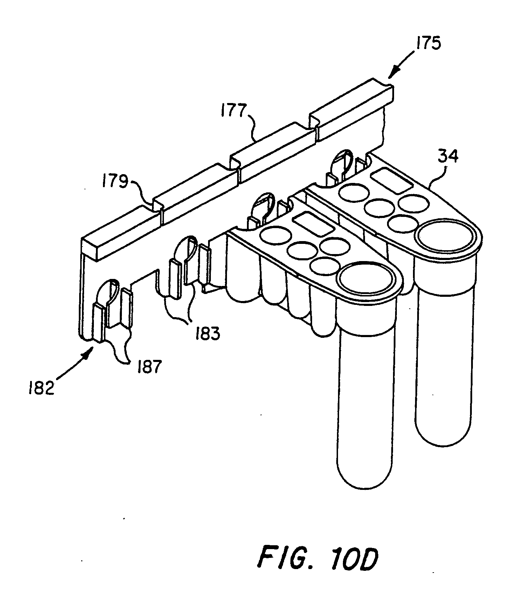

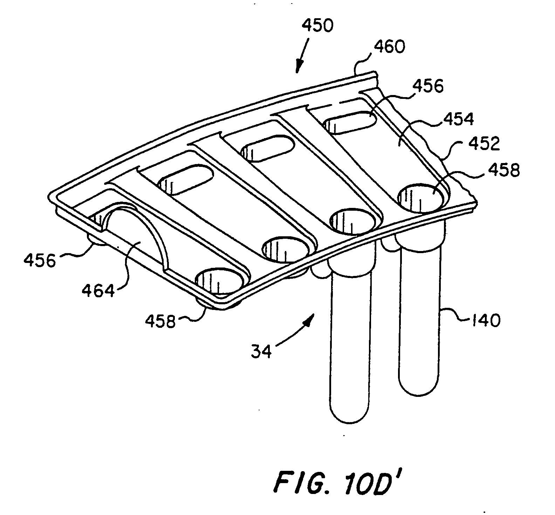

FIGURE 10D' is an isometric view in section of the reaction vessel loading device illustrating the loader mounted with two reaction vessels and means for mounting other reaction vessels.

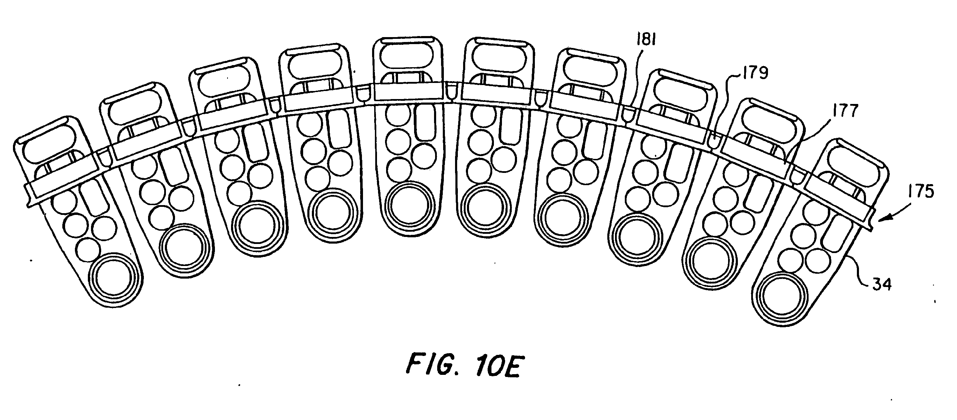

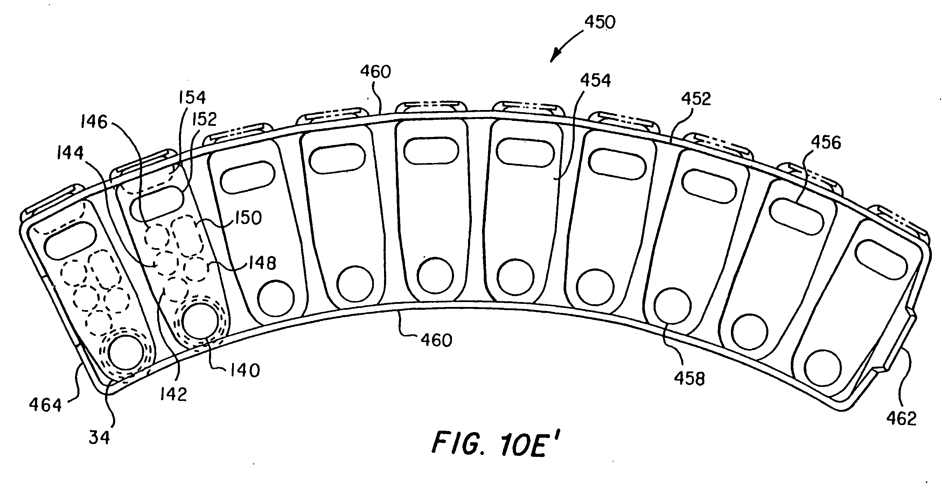

FIGURE 10E' is a top view of the reaction vessel loading device, the reaction vessel loading device having arced linear dimensions which match the radius of the reaction vessel carousel, the loader having mounted thereon two reaction vessels and the capability of mounting eight additional reaction vessels.

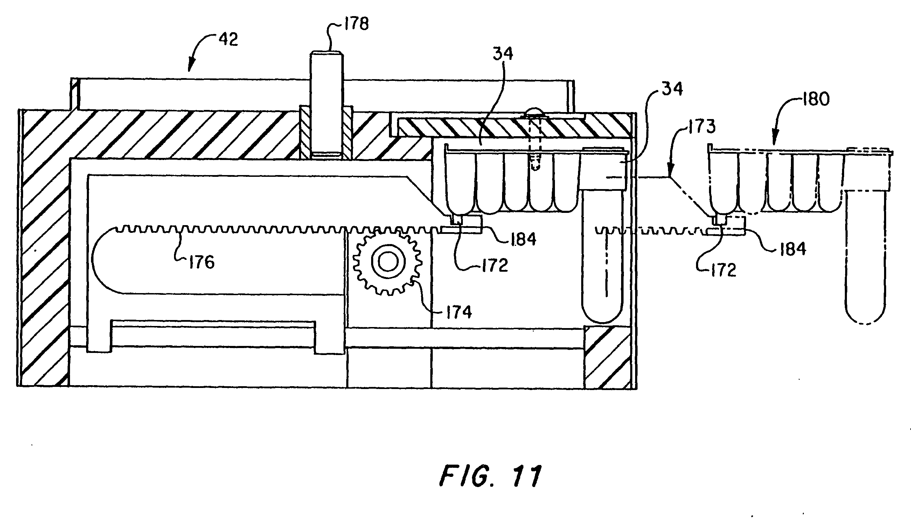

FIGURE 11 is a sectional side view of the transfer element of the automated analytical system engaging a reaction vessel for transfer from the main carousel into the transfer station.

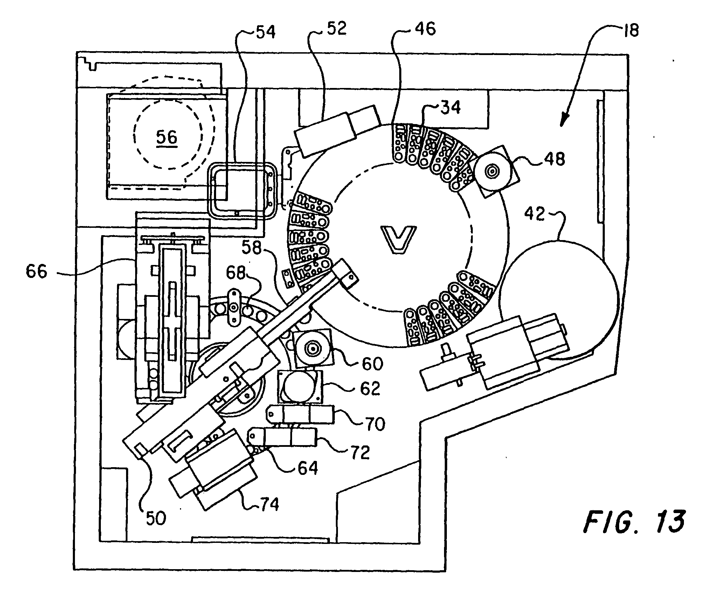

FIGURE 12 is a perspective side elevational view of the transfer station of the automated analytical system.
FIGURE 13 is a top plan view in section illustrating in isolation the controlled environment portion of the automated analytical system.

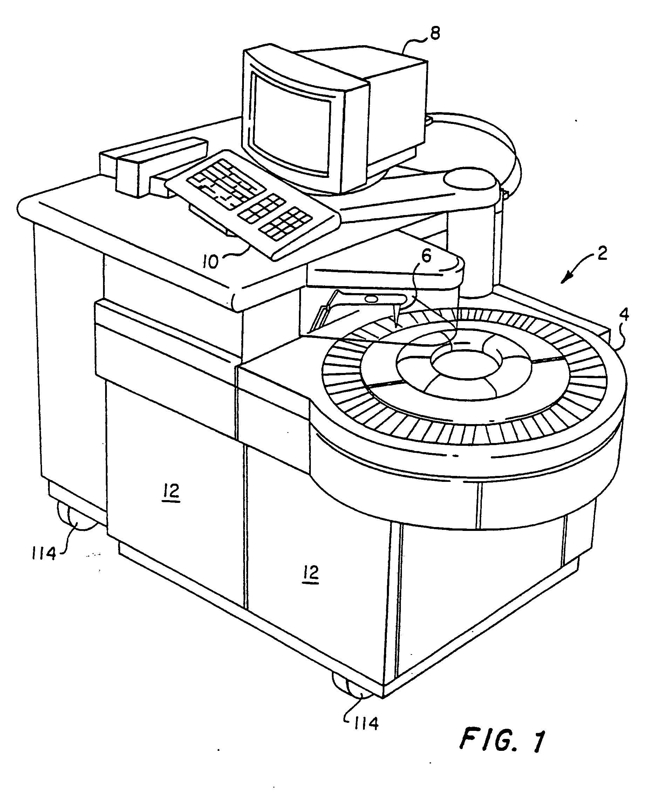

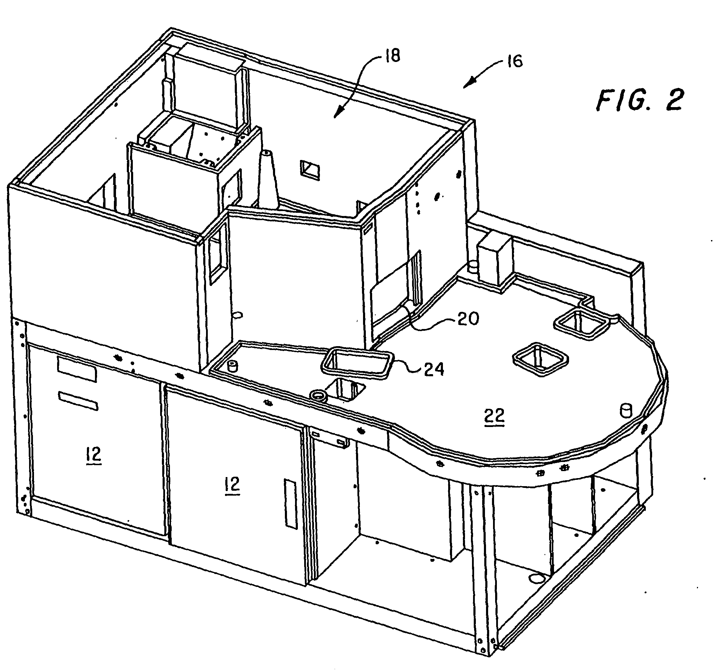

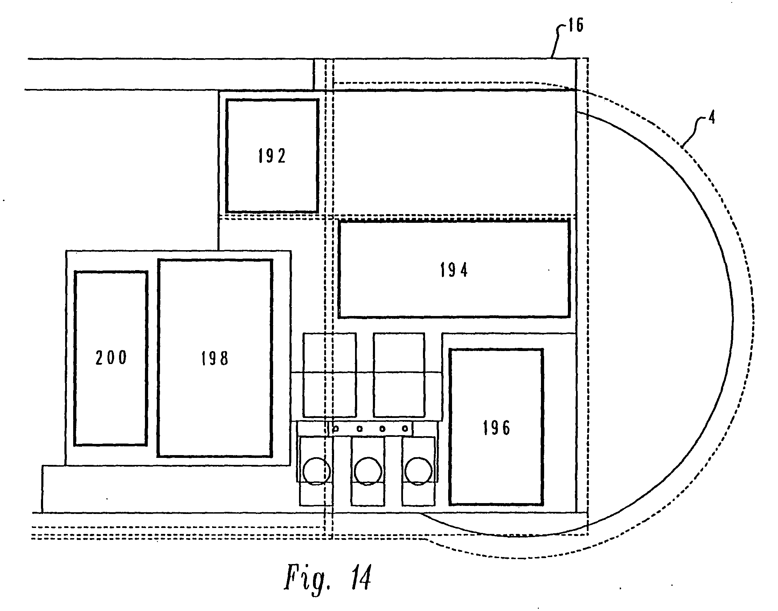

FIGURE 14 is a top plan view in section of the lower cabinet of FIGURES 1 and 2 illustrating water and/or buffer supply station as well as liquid and solid waster containers of the automated analytical system.

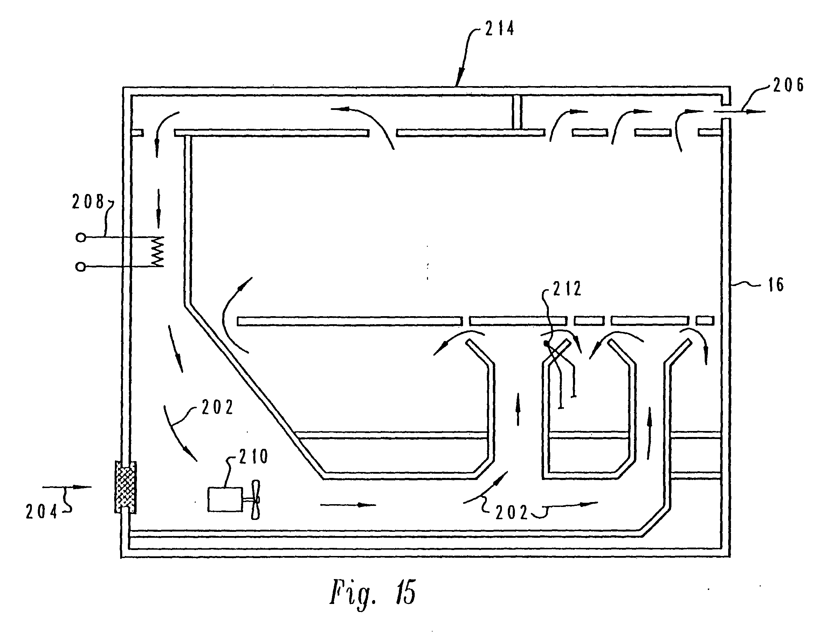

FIGURE 15 is a schematic view illustrating the system control environment airflow and temperature control of the automated analytical system.
FIGURE 15A is a schematic view which illustrates another embodiment of the environmental air flow and temperature control of the automated analytical system where no air is recirculated.

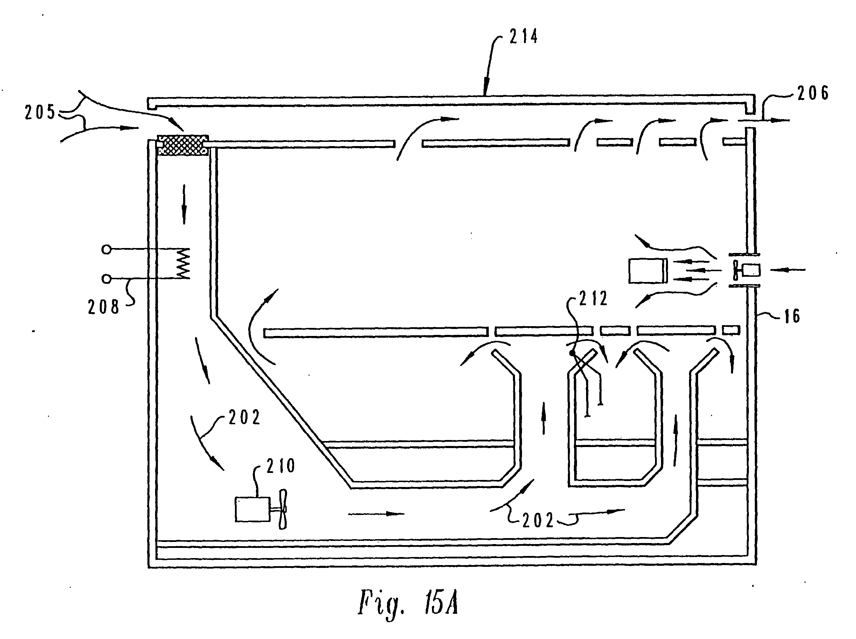

FIGURE 16 is a side elevational view in partial section of a MEIA cartridge for use with the automated analytical system.

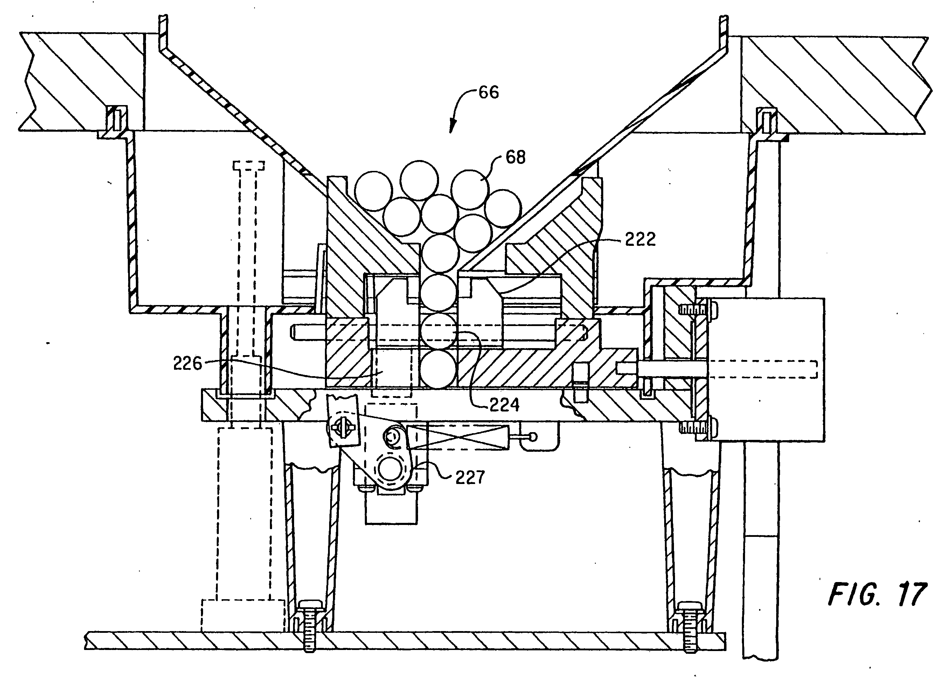

FIGURE 17 is a side elevational view in section of a MEIA cartridge feeder of the automated analytical system.

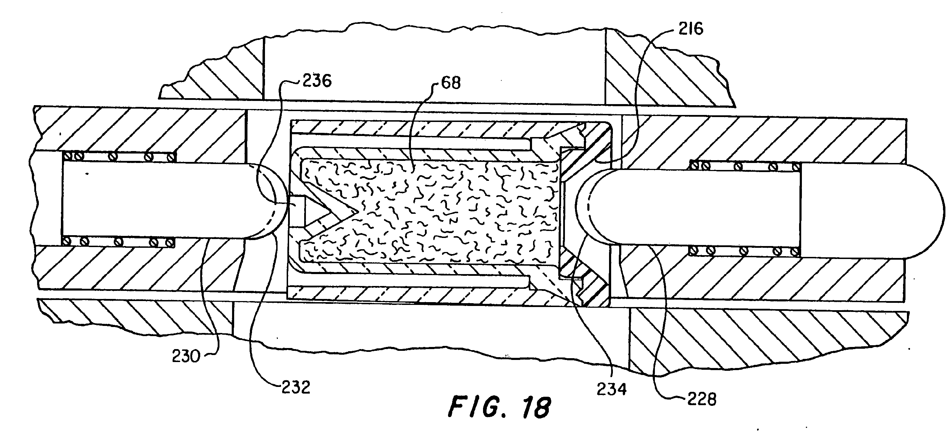

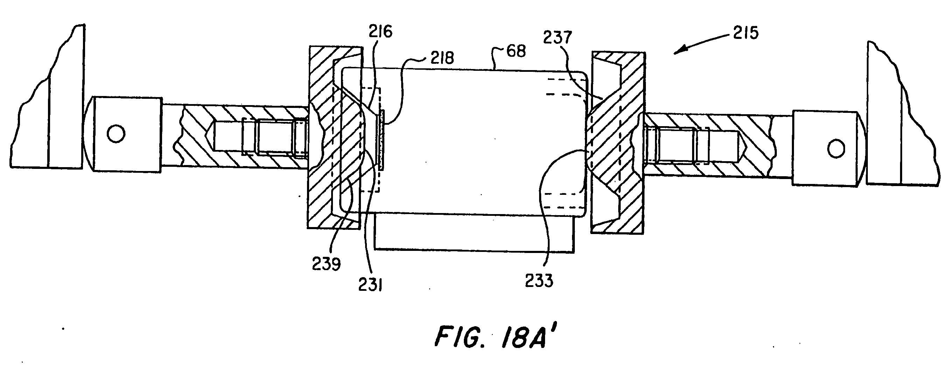

FIGURE 18 is a side sectional view in isolation of the MEIA cartridge feeder-cartridge orientation pin mechanism of the automated analytical system.
FIGURE 18A' is a side cross-sectional view in isolation of a second embodiment of an MEIA cartridge feeder/cartridge orientation mechanism of the automated analytical system.

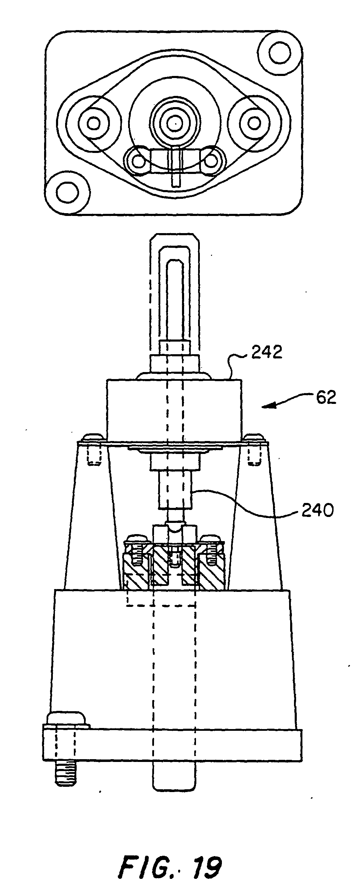

FIGURE 19 is a side sectional view in isolation of the MEIA cartridge ejector of the automated analytical system.

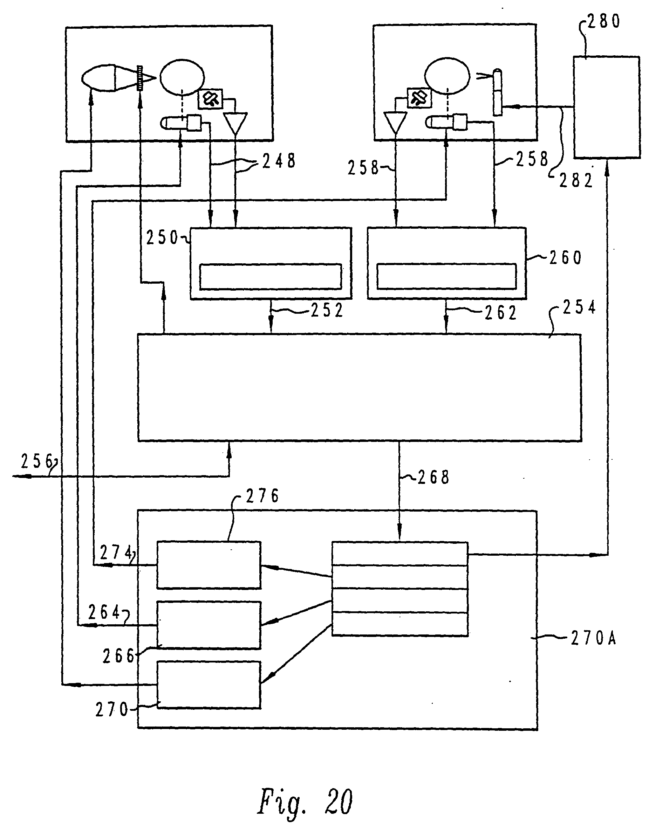

FIGURE 20 is a box diagram of the optics signal processor of the automated analytical system.

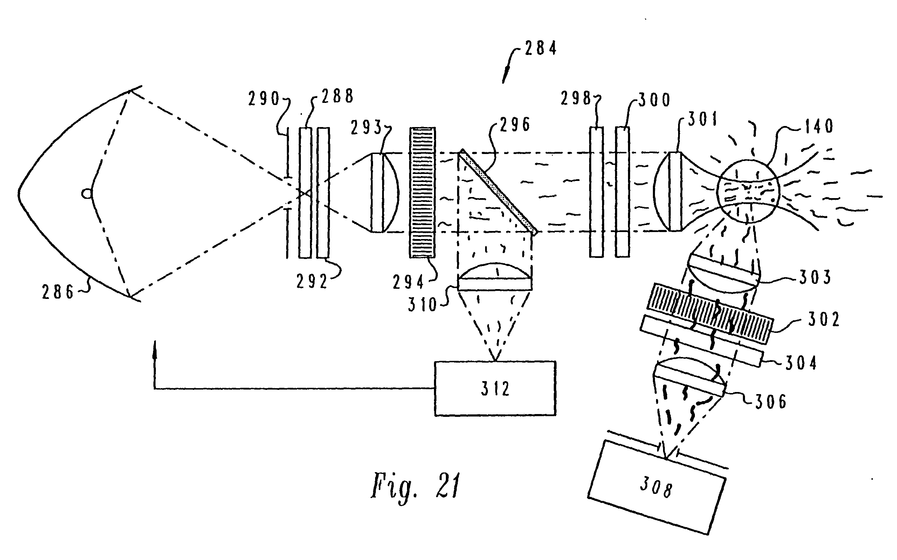

FIGURE 21 is a schematic of the FPIA optical system of the automated analytical system.

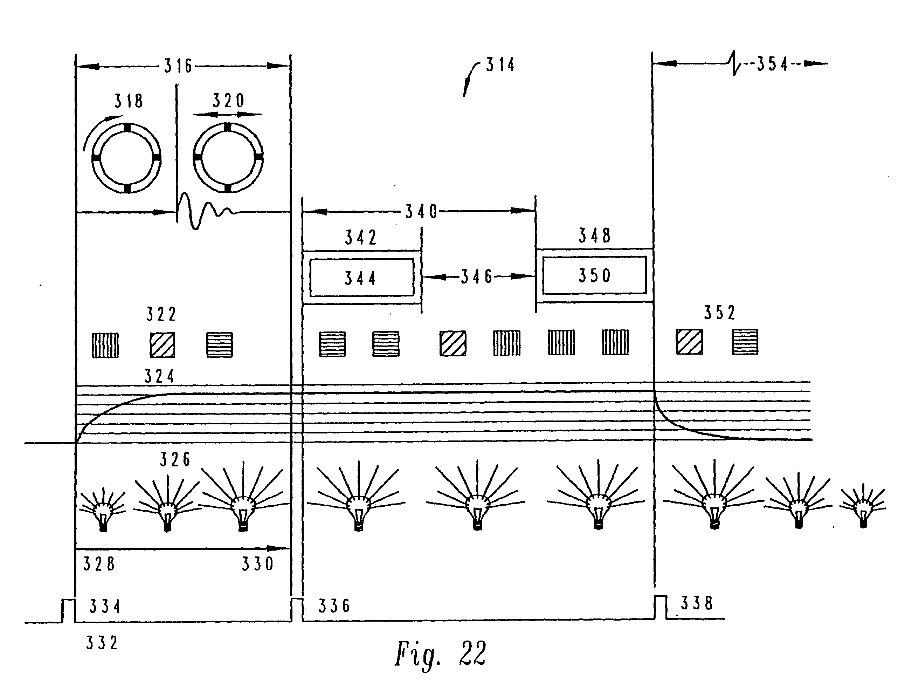

FIGURE 22 is a schematic of the FPIA read[er] sequence of the automated analytical system.

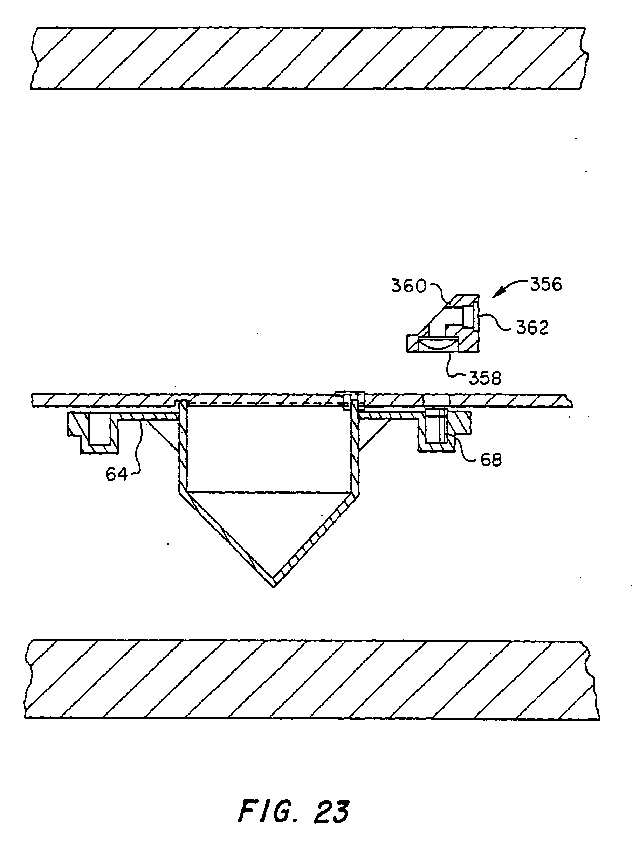

FIGURE 23 is a side sectional view in isolation of a MEIA cartridge carousel of the automated analytical system, MEIA cartridge and MEIA reader.

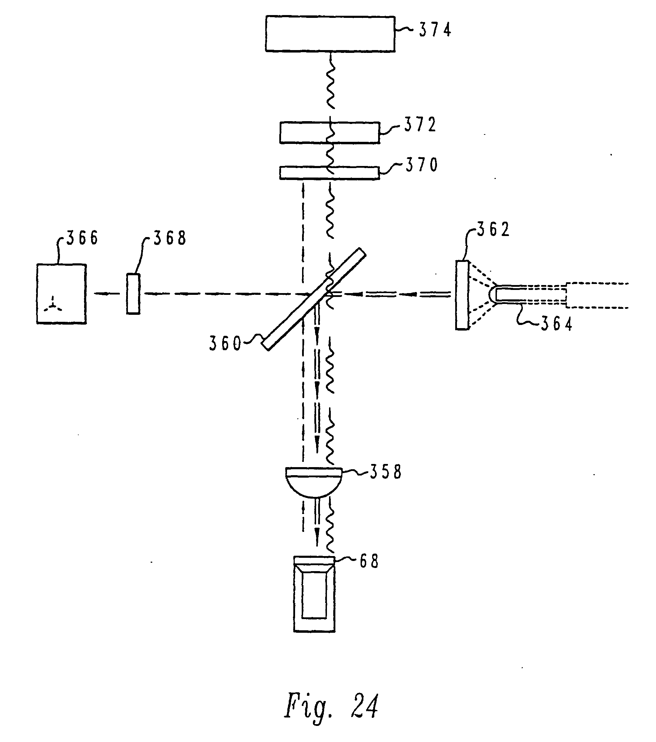

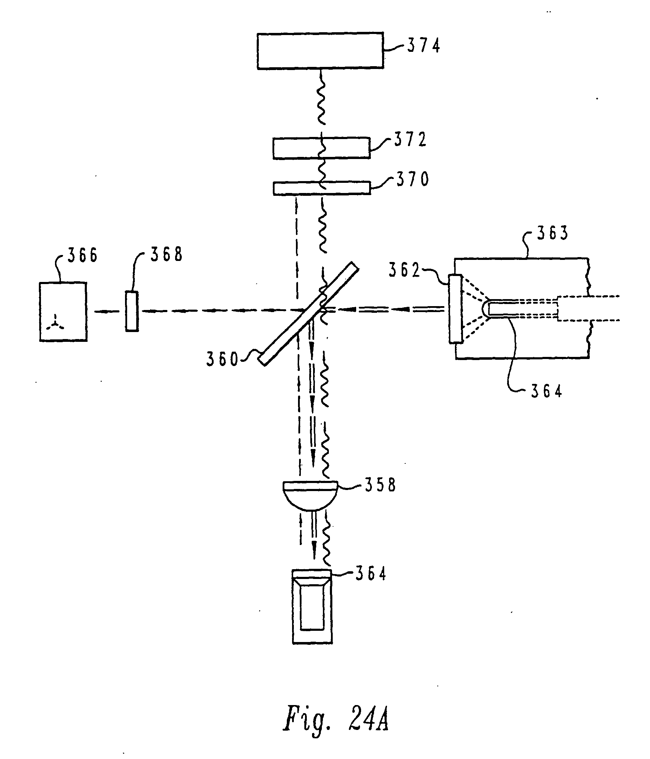

FIGURE 24 is a schematic of the MEIA system optical assembly of the automated analytical system.
FIGURE 24A is a schematic of a MEIA optical assembly of the automated, continuous and random access analytical systems wherein the light source is maintained by heating means at a constant minimal temperature during shut off periods.

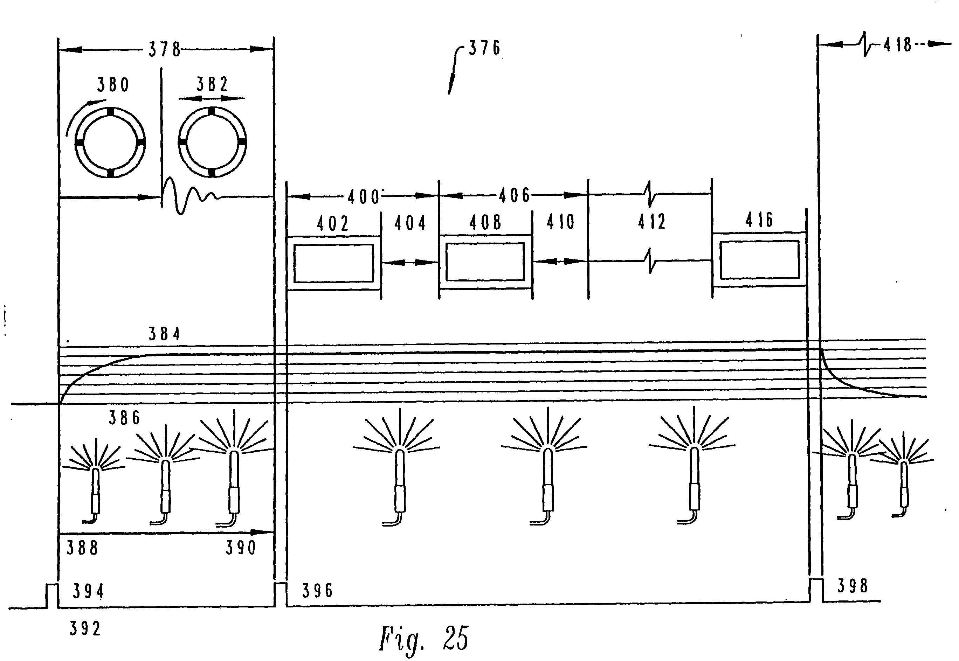

FIGURE 25 is a schematic of the MEIA read sequence of the automated analytical system.

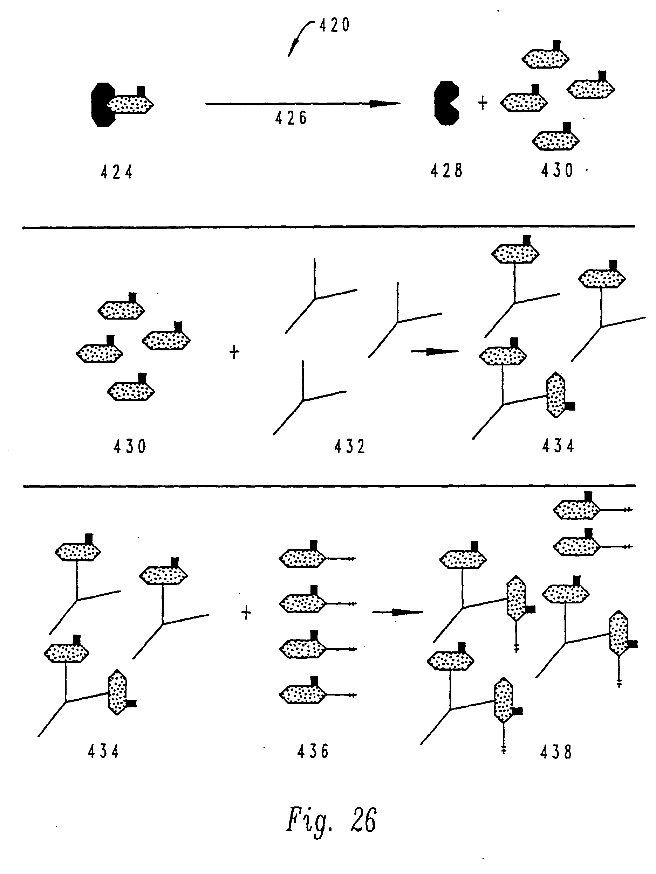

FIGURE 26 is a schematic reaction sequence of a FPIA for T4 performed on the automated analytical system.

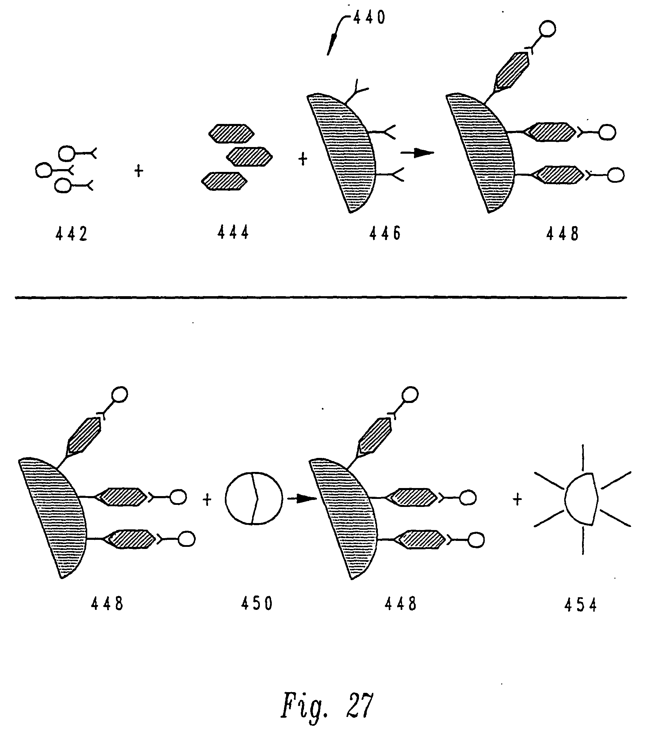

FIGURE 27 is a schematic reaction sequence of a one-step sandwich MEIA performed on the automated analytical system.

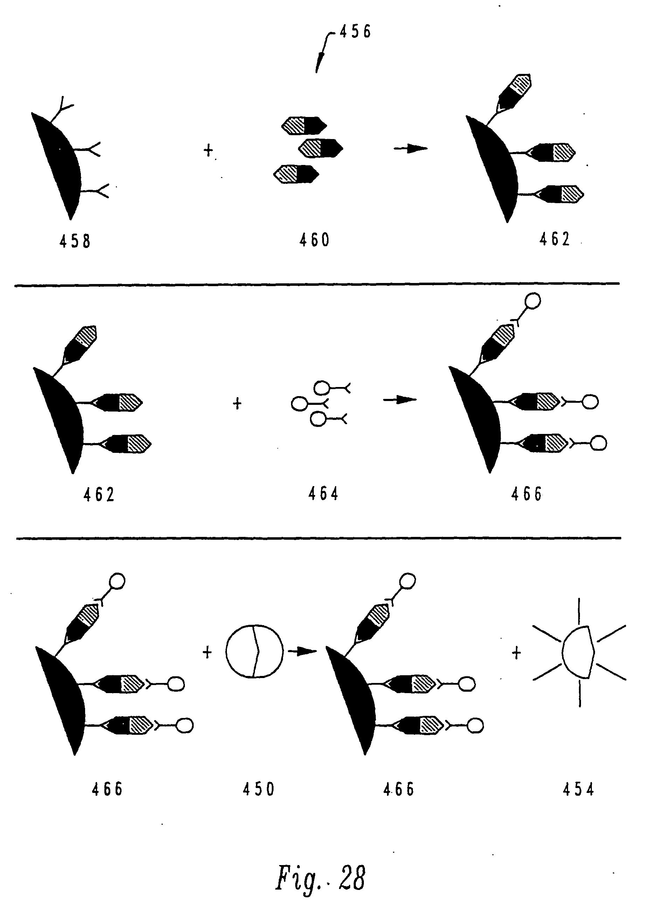

FIGURE 28 is a schematic reaction sequence of a two-step sandwich MEIA performed on the automated analytical system.

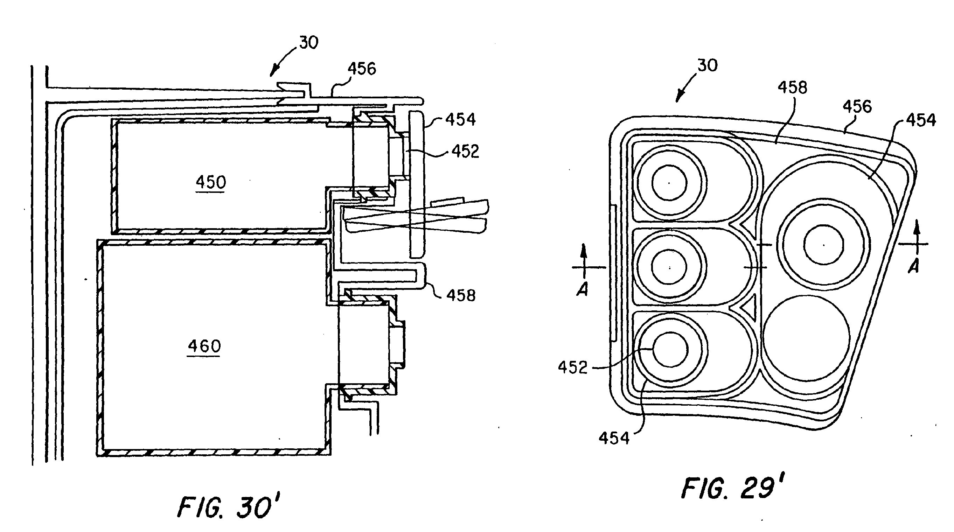

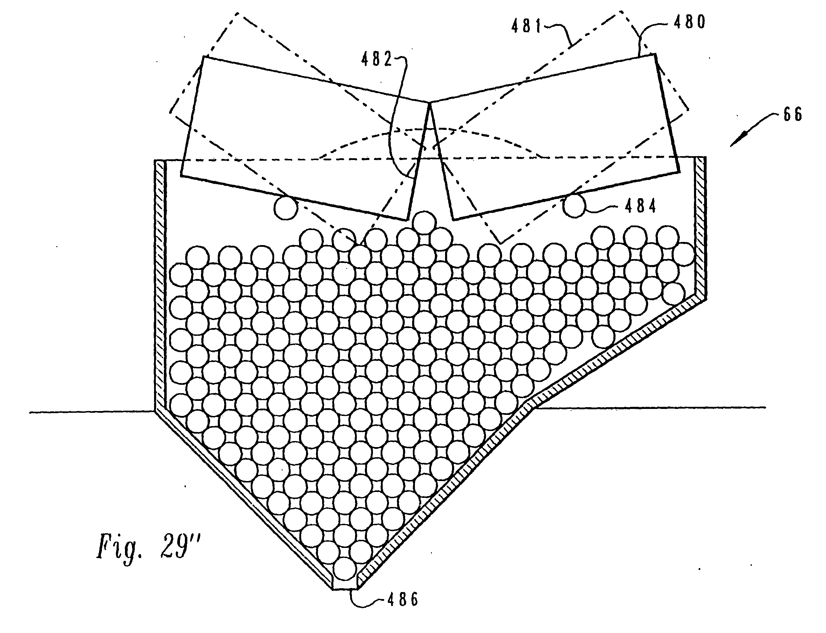

FIGURE 29' is a top view of a reagent pack having the reagent containers covered.

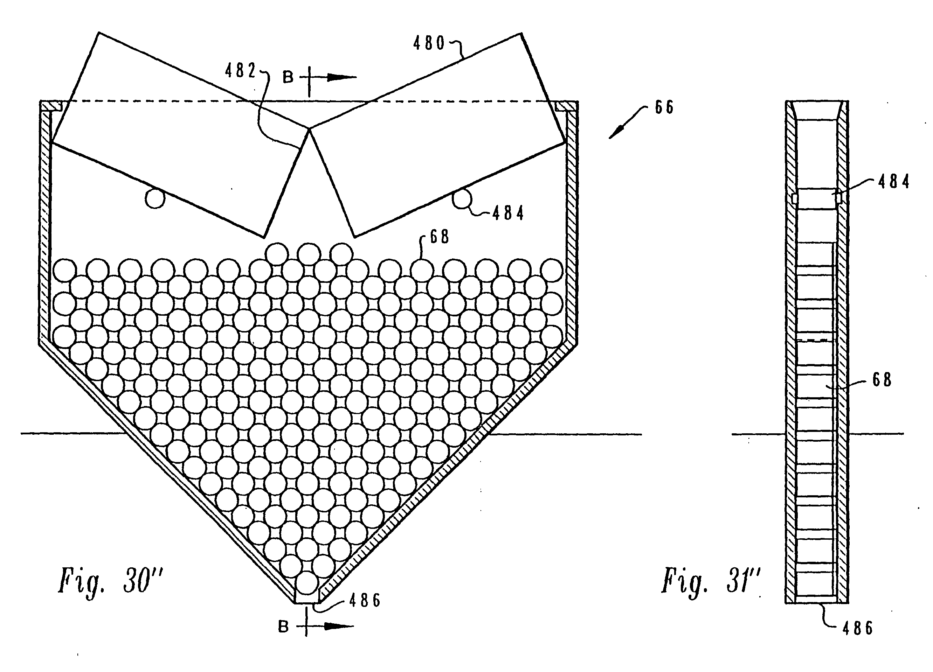

FIGURE 30' taken along section A-A of Figure 29 presents a side view in section taken along the fine A-A of Figure 29 illustrating a cover means in various open and closed positions.

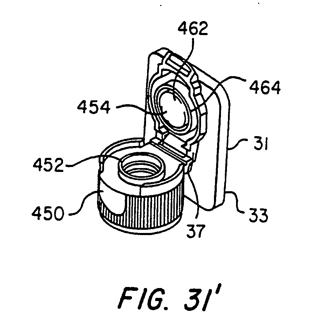

FIGURE 31' is an isometric view of an open reagent vessel capping means.

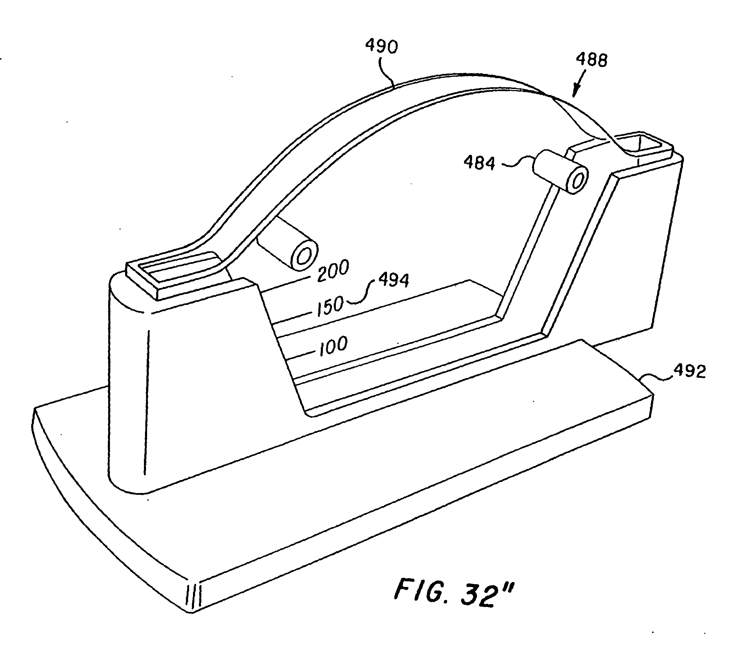

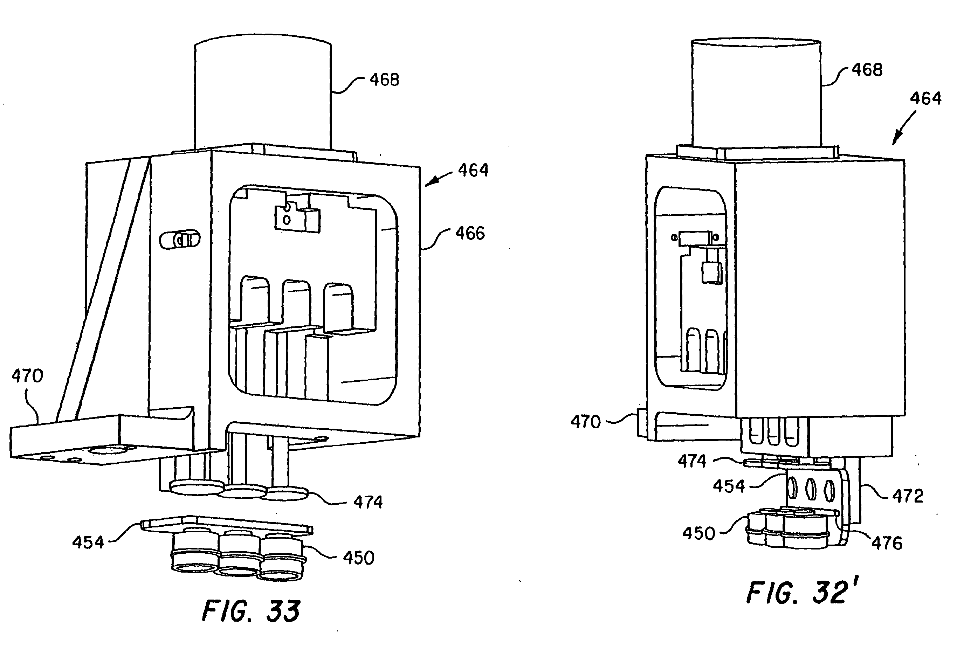

FIGURE 32' is a perspective side elevational view of a reagent container lid opening and closing station with the reagent containers in the reagent pack having the lids opened.
FIGURE 33 presents a different perspective side elevation view from that of Figure 32' wherein the reagent containers of the reagent pack are below elements of the opening and closing station with the reagent pack lids being closed.
FIGURE 29" is a side cross-sectional view in isolation of a split open cartridge carton shown in various open positions in phantom as engaged in cooperation with a cartridge hopper containing multiple cartridges.
FIGURE 30" is a side cross-sectional view in isolation of another embodiment of the cartridge hopper with a split open cartridge carton positioned for dumping cartridges into the hopper.
FIGURE 31" is a cross sectional end view in isolation of the cartridge hopper of Figure 30".
FIGURE 32" is an isometric view of another embodiment of a free standing cartridge hopper showing the cartridge hopper in a detached mode suitable for loading cartridges from a cartridge carton.

### Description of the Invention

### Definitions

The following definitions are applicable to the present invention:
The term "test sample", as used herein, refers to a material suspected of containing the analyte. The test sample can be used directly as obtained from the source or following a pretreatment to modify the character of the sample. The test sample can be derived from any biological source, such as a physiological fluid, including, blood; saliva, ocular lens fluid, cerebral spinaf fluid, sweat, urine, milk, ascites fluid, raucous, synovial fluid, peritoneal fluid, amniotic fluid or the like. The test sample can be pretreated prior to use, such as preparing plasma from blood, diluting viscous fluids, or the like; methods of treatment can involve filtration, distillation, concentration, inactivation of interfering components, and the addition of reagents. Besides physiological fluids, other liquid samples can be used such as water, food products and the like for the performance of environmental or food production assays. In addition, a solid material suspected of containing the analyte can be used as the test sample. In some instances it may be beneficial to modify a solid test sample to form a liquid medium or to release the analyte.
The term "analyte" or ''analyte of interest", as used herein, refers to the compound or composition te be detected or measured and which has at least one epitope or binding site. The analyte can be any substance for which there exists a naturally occurring binding member or for which a binding member can be prepared. Analytes include, but are not limited to, toxins, organic compounds, proteins, peptides, microorganisms, amino acids, nucleic acids, hormones, steroids, vitamins, drugs (including those administered for therapeutic purposes as well as those administered for illicit purposes), virus particles and metabolites of or antibodies to any of the above substances. The term "analyte" also includes any antigenic substances, haptens, antibodies, macromolecules and combinations thereof.
The term "analyte-analog", as used herein, refers to a substance which cross-reacts with an analyte-specific binding member, although it may do so to a greater or lesser extent than does the analyte itself. The analyte-analog can include a modified analyte as well as a fragmented or synthetic portion of the analyte molecule, so long as the analyte-analog has at least one epitopic site in common with the analyte of interest. An example of an analyte-analog is a synthetic peptide sequence which duplicates at least one epitope of the whole-molecule analyte so that the analyte-analog can bind to an analyte-specific binding member.
The term binding member", as used herein, refers to a member of a binding pair, i.e., two different molecules wherein one of the molecules specifically binds to the second molecule through chemical or physical means. In addition to antigen and antibody binding pair members, other binding pairs include, as examples without limitation, biotin and avidin, carbohydrates and lectins, complementary nucleotide sequences, complementary peptide sequences, effector and receptor molecules, enzyme cofactors and enzymes, enzyme inhibitors and enzymes, a peptide sequence and an antibody specific for the sequence or the entire protein, polymeric acids and bases, dyes and protein binders, peptides and specific protein binders (e.g., ribonuclease, S-peptide and ribonuclease S-protein), and the like. Furthermore, binding pairs can include members that are analogs of the original binding member, for example, an analyte-analog or a binding member made by recombinant techniques or molecular engineering. If the binding member is an immunoreactant it can be, for example, a monoclonal or polyclonal antibody, a recombinant protein or recombinant antibody, a chimeric antibody, a mixture(s) or fragment(s) of the foregoing, as well as a preparation of such antibodies, peptides and nucleotides for which suitability for use as binding members is well known to those skilled in the art.
The term "detectable moiety", as used herein, refers to any compound or conventional detectable chemical group having a detectable physical or chemical property and which can be used to label a binding member to form a conjugate therewith. Such detectable chemical group can be, but is not intended to be limited to, enzymatically active groups such as enzymes, enzyme substrates, prosthetic groups or coenzymes; spin labels; fluorescers and fluorogens; chromophores and chromogens; luminescers such as chemiluminescers and bioluminescers; specifically bindable ligands such as biotin and avidin; electroactive species; radioisotopes; toxins; drugs; haptens; DNA; RNA; polysaccharides; polypeptides; liposomes; colored particles and colored microparticles; and the like.
The term "continuous access", as used herein, refers to the ability to add additional test samples or reagents to the automated analytical system of the present invention without the interruption of assays which are being performed by the automated analytical system of the present invention at the time of such addition.
The term "random access", as used herein, refers to the ability of the automated analytical system of the present invention to simultaneously perform more than one scheduled assay in any order in which such plurality of scheduled assays are presented into the automated analytical system of the present invention.
The term "simultaneous", as used herein, refers to the ability of the automated analytical system of the present invention to independently perform two or more scheduled assays at the same time.
The term "kitting", as used herein, refers to the ability of the automated analytical system of the present invention to create a unit dose disposable by separately transferring test samples and reagents to a reaction vessel of the present invention without initiation of an assay reaction sequence.
The term "quat" refers to a polycationic material solution for assays which use these materials which are not an antibody or antigen to capture the analyte from the sample on the matrix of, for example, MEIA cartridge. In the present inventive system, quat is dispensed to the matrix during test processing, prior to the transfer of the reaction mixture from the reaction vessel.
The term "flexible protocols" refers to the variety of different assay protocols capable of being processed in accordance with the inventive system. Examples include MEIA formats configured in 1- and 2-step sandwich and competitive assay formats; order of activity processing, including the ability to initiate sample processing for both MEIA formats and FPIA formats on the front-end carousel prior to transfer onto the process carousel; variable incubation periods: optical read formats and wash sequences. This contrasts to some prior art, known random access systems which force all assay protocols to adhere to a strict "lock-step" format, in which assay configuration (i.e. 1- versus 2-step formats), activity order, incubation timing, and other similar protocols are fixed by the instrument.

### Scheduler

According to the present invention, a system scheduler generates and optimizes the workload for the system's mechanical resources from all the tests ordered to run on the system. The main goal of the scheduler is to keep the system's resources from sitting idle while there are tests remaining to be processed by the system. Keeping each of the resources busy also minimizes the time required by the instrument to perform the tests.

A high-level view of the scheduling process can be broken into two steps: (1) proper scheduling of each of the activities in a test is ensured before the test is kitted, and (2) an attempt to perform each test activity prior to its original scheduled execution time, to minimize resource idle time and increase test throughput in the system.

To enable scheduling a test in advance of its performance in the system, each test's assay protocol contains several timing parameters used in the scheduling process. Each activity of the test contains time values which the scheduler uses to determine which resources the activity requires and the time period that these resources are needed. Also,-each activity in the test can be tied to other activities by incubation periods. These incubation periods, which are dictated by the chemistry of the assay, help the scheduler determine the amount of time that must elapse between the execution of two activities. Each incubation period in the assay protocol provides for the minimum and maximum time that may elapse between the execution of each activity. These limits are referred to in the scheduling process as the incubation window for the activities.

In the inventive system, the operator chooses the order that tests are prepared to run on the instrument by selecting the placement of samples on the instrument. The sample placed closest to the pipette station is the first sample prepared to run on the instrument. To guard against evaporation, a test will not be prepared until the scheduler ensures that all resources used by the test's activities will be available at the required times set forth in the test's assay protocol. Preparation of a particular test will be postponed whenever an activity of another test already in the instrument has a resource scheduled at the time it is needed by an activity on that test. The sample preparation area of the instrument will remain idle until the test can be scheduled without conflicting with tests already in the instrument. When proper scheduling of the test can be achieved, the test will be prepared and transferred into the process area.

The second step in the scheduling process is to optimize the workload for each system resource to minimize both the resource's idle time and the time required to perform the resource's workload. once tests are transferred into the process area, the scheduler optimizes the existing schedule for each resource. At predetermined intervals, the scheduler examines the next interval of work for each resource. If there is any idle time in this interval, the scheduler attempts to minimize the idle time by rearranging the resource's workload to eliminate idle time, providing the activities remain within their allowed incubation windows. When optimization of this interval is complete, this section of the workload is performed by the resource at the designated times.

The scheduler continues to prepare samples as long as there are samples on the instrument that have tests ordered to be run. optimization of the resources' workloads will continue until all tests transferred into the system have finished processing.

### Stat Procedure

The inventive system allows special priority handling of specific samples identified by the user as being stat samples. A stat sample, as defined by the inventive system, is a sample that must be processed by the instrument in the shortest amount of time possible. Special handling of stat samples occurs both in the front sample entry area and in the processing area of the instrument.

In the inventive system, the operator chooses the order that tests are prepared to run on the instrument by selecting the placement of samples on the instrument. The sample placed closest to the pipette station is the first sample prepared to run on the instrument. This pattern of sample preparation is interrupted whenever the user places a stat test on the instrument. Whenever a stat test is ordered, the system will finish preparing the test on the current sample, and then move directly to the stat sample to prepare all its tests. To guard against evaporation, sample preparation will not begin for a test before proper scheduling of the test's activities in the processing area is ensured.

The system scheduling algorithm is also modified for stat processing. The scheduling algorithm used for normal tests attempts to maximize the number of tests processed in the instrument each hour. This occurs by allowing sufficient time between test activities to enable other tests' activities to be performed in these gaps. The scheduling approach used for stat tests attempts to process this one test in the shortest amount of time possible. Each activity of a stat test is scheduled at the earliest possible time of execution as defined in the test's assay definition. When all activities of a test are guaranteed proper scheduling in the instrument, sample preparation of the test will begin. After all tests on the stat sample are prepared, the system will return to the sample it was working on before it serviced the stat.

Stat tests receive special consideration in the processing area when there is idle time in a resource's workload. At predetermined intervals, the scheduler examines the next interval of work allocated to each resource in the processing area of the system. If there is any idle time during this interval the scheduler attempts to minimize it by rearranging the resource's workload. Test activities scheduled for this resource that can be performed earlier than they are currently scheduled, as defined by their assay protocols, are moved forward to fill the idle time. Stat test activities are the first candidates to be pulled forward in the workload, thus further decreasing the amount of time needed to process the stat test in the instrument.

The system stat test handling algorithms have been shown to allow stat tests to be processed in the minimum amounts of time possible, without having a negative effect on the instrument's overall throughput of tests per hour.

The automated analytical system of the present invention is capable of performing various assays employing various detection systems known in the art and include, but are not intended to be limited to, spectrophotometric absorbance assay such as end-point reaction analysis and rate of reaction analysis, turbidimetric assays, nephelometric assays, radiative energy attenuation assays (such as those described in U.S. Patent No. 4,496,293 and U.S. Patent No. 4,743,561 and incorporated herein by reference), ion capture assays, colorimetric assays, fluorometric assays, electrochemical detection systems, potentiometric detection systems, amperometric detection system and immunoassays. Immunoassays include, but are not intended to be limited to, heterogeneous immunoassays such as competitive immunoassays, sandwich immunoassays, immunometric immunoassays, and the like, where the amount of a detectable moiety employed therein can be measured and correlated to the amount of analyte present in a test sample.

Generally, in a spectrophotometric assay, such as those performed on the Abbott Spectrum clinical analyzer and the Abbott Spectrum Series II clinical analyzer (Abbott Laboratories, Abbott Park, IL, USA) the interaction in an assay solution between the analyte to be determined and a reagent system specific for the analyte produces a detectable change in the transmittive properties of the assay solution. The change in the transmittive properties refers to the amount of light absorbed or scattered by an assay solution within a particular wavelength band when a beam of light of known intensity is passed through the assay solution. The change in the transmittive properties of an assay solution is measured by passing monochromic light having a known intensity though the assay solution and determining the ratio of the intensity of the transmitted or scattered light to the intensity of the incident light. Nearly all analytes either absorb energy of a specific wavelength or interact in an assay solution with a particular reagent system to produce a detectable change in the transmittive properties of the assay solution, characteristics which have resulted in the development of numerous specific spectrophotometric assays.
Spectrophotometric assays which rely upon the measurement of the change in the transmittive properties of an assay solution as a measure of an analyte in the assay solution include, for example, assays wherein there is a change in the color of the assay when there is a change in the turbidity of the assay solution, that is, turbidimetric or nephelometric assays.

In a colorimetric assay, the change in the transmittive properties of an assay solution is generally referred to as the absorbance of the assay solution and is dependent upon the change in the color of the assay solution due to the interaction of the analyte to be determined and reagent system specific for the analyte. The absorbance of the assay solution is related to the concentration of the analyte in the assay solution. A colorimetric assay utilizes a chromogenic reagent system capable of interacting in an assay solution with the particular analyte of interest, to produce a detectable change in the transmittive properties, specifically the color, of the assay solution. Numerous chromogenic reagent systems useful in the determination of specific analytes have been developed and are commercially available.

The principle of turbidimetric assays is to determine the amount of light scattered or blocked by particulate matter as light passes though an assay solution. In a turbidimetric assay, the analyte of interest interacts with a reagent system specific for the analyte to form a suspension of particles in the assay solution. As a beam of light having a known intensity is passed through an assay solution, the suspension of particles formed by the interaction of the analyte reagent system blocks or scatters the incident light, thereby reducing the intensity of the light transmitted through the assay solution. The change of the transmittive properties in a turbidimetric assay refers to the decrease in the intensity of the light transmitted through an assay solution, is related to the amount of incident light that is scattered or blocked by the suspension of particles, and depends upon the number of particles present and the cross-sectional area of such particles.

A nephelometric assay is similar to a turbidimetric assay in that the analyte of interest interacts with a reagent system specific for the ligand to form a suspension of particles in the assay solution. In a nephelometric assay, the change in the transmittive properties of the assay solution is also related to the amount of incident light scattered or blocked by the suspension of particles, but unlike a turbidimetric assay wherein the intensity of the light transmitted through the assay solution is measured, the scattered or blocked light is measured at an angle to the light incident to the assay solution. Therefore, in a nephelometric assay the change in the transmittive properties refers to the difference in intensities of light incident to the assay solution and light scattered at an angle to the incident light. Turbidimetric and nephelometric assays are utilized in the analysis of blood, urine, spinat fluid, and the like, for the determination of analytes such as proteins wherein there is no comparable colorimetric assay due to the lack of an effective chromogenic reagent system. Yoe and Krimman, Photoelectric Chemical Analysis, Vol. II: Nephelometry, Wiley & Sons, Inc., New York, 1929, describe various nephelometric assays. various reagents and reagent systems which can be employed for performing spectrophotometric assays on the automated analytical systems of the present invention include, but are not intended to be limited to, those for the simultaneous determination of glucose and urea, such as described in U.S. Patent No. 5,037,738 and incorporated herein by reference. The simultaneous determination of calcium and phosphorous; the simultaneous determination of cholesterol and triglycerides; determining isoenzymes; determining blood ammonia levels, and the like, can be performed on the apparatus and by the methods of the present invention.

Typically in a fluorometric assay, an analyte in an assay solution is chemically or immunologically transformed into a fluorescent complex or conjugate thereby producing a detectable change in the fluorescent properties of the assay solution. The change in the fluorescent properties of the assay solution is measured by exciting the fluorescent complex or conjugate properties produced with monochromatic light of a wavelength within the excitation wavelength band of the fluorescer, and measuring the intensity of the emitted light at a wavelength within the emission wavelength band of the fluorescer. The fluorescent intensity of the emitted light is related to the concentration of the analyte. However, the intensity of the fluorescence emitted by the assay solution may be inhibited when the ligand to be determined complexes with nonfluorescent interferences such as protein or phosphates present in the sample, or when the sample containing the ligand to be determined has sufficient color so as to act as a filter and thereby reduce the intensity of the emitted fluorescence. It is well recognized that in order to maximize the sensitivity and specificity of a fluorometric assay, these inhibiting factors, if present, must be overcome either by removal of the nonfluorescent interferences or color producing material prior to the analysis, or by compensating for the presence of such factors using an internal standard added to a second aliquot of sample and carrying out the entire assay procedure using the aliquot containing the internal standard.

Generally, homogeneous and heterogeneous immunoassays depend upon the ability of a first binding member of a binding member pair to specifically bind to a second binding member of a binding member pair wherein a conjugate, comprising one of such binding members labeled with a detectable moiety, is employed to determine the extent of such binding. For example, where such binding pair members are an analyte and an antibody to such analyte, the extent of binding is determined by the amount of the detectable moiety present in the conjugate, which either has or has not participated in a binding reaction with the analyte, wherein the amount of the detectable moiety detected and measured can be correlated to the amount of analyte present in the test sample.

Homogeneous immunoassays typically are performed in a competitive immunoassay format involving a competition between an analyte from a test sample and a tracer for a limited number of receptor binding sites on an antibody to the analyte. The tracer comprises the analyte or analog thereof labeled with a detectable moiety wherein the concentration of analyte in the test sample determines the amount of the tracer that will specifically bind to the antibody. The amount of the tracer-antibody conjugate produced by such binding may be quantitatively measured and is inversely proportional to the amount of analyte present in the test sample. For example, fluorescent polarization techniques for making such determination, such as in fluorescent polarization immunoassays as described herein, are based on the principle that a fluorescently labeled compound when excited by linearly polarized light will emit fluorescence having a degree of polarization inversely related to its rate of rotation. When a molecule such as a tracer-antibody conjugate having a fluorescent label is excited with a linearly polarized fluorescent molecule it is constrained from rotating between the time light is absorbed and emitted. When a "free" tracer molecule (i.e., unbound to an antibody) is excited by linearly polarized light, its rotation is much faster than the corresponding tracer-antibody conjugate and the molecules are more randomly orientated, therefore, the emitted light is polarized. Accordingly, when plane polarized light is passed through a solution containing the aforementioned reagents, a fluorescent polarization response is detected and correlated to the amount of analyte present in the test sample.

Various fluorescent compounds which can be employed for performing fluorescent polarization assays on the automated analytical system of the present invention include, but are not intended to be limited to, aminofluoresceins, such as described in U.S. Patent No. 4,510,251 and U.S. Patent No. 4,614,823, incorporated herein by reference; triazinylaminofluoresceins, such as described in U.S. Patent No. 4,420,568 and U.S. Patent No. 4,593,089, incorporated herein by reference; carboxyfluoresceins, such as described in U.S. Patent No. 4,668,640, incorporated herein by reference; and the like.

Heterogenous immunoassays typically involve a labeled reagent or tracer comprising an analyte, an analog of the analyte, or an antibody thereto, labeled with a detectable moiety, to form a free species and a bound species. In order to correlate the amount of tracer in one of such species to the amount of analyte present in the test sample, the free species must first be separated from the bound species, which can be accomplished according to methods known in the art employing solid phase materials for the direct immobilization of one of the binding participants in the binding reaction, such as the antibody, analyte or analog of the analyte, wherein one of the binding participants is immobilized on a solid phase material, such as a test tube, beads, particles, microparticles or the matrix of a fibrous material, and the like, according to methods known in the art.

Heterogenous immunoassays can be performed in a competitive immunoassay format as described above wherein, for example, the antibody can be immobilized to a solid phase material whereby upon separation, the amount of the tracer which is bound to such solid phase material can be detected and correlated to the amount of analyte present in the test sample. Another form of a heterogeneous immunoassay employing a solid phase material is referred to as a sandwich immunoassay, which involves contacting a test sample containing, for example, an antigen with a protein such as an antibody or another substance capable of binding the antigen, and which is immobilized on a solid phase material. The solid phase material typically is treated with a second antigen or antibody which has been labeled with a detectable moiety. The second antigen or antibody then becomes bound to the corresponding antigen or antibody on the solid phase material and, following one or more washing steps to remove any unbound material, an indicator material such as a chromogenic substance which reacts with the detectable moiety (e.g., where the detectable moiety is an enzyme, a substrate for such enzyme is added) to produce a color change. The color change is then detected and correlated to the amount of antigen or antibody present in the test sample.

For example, a heterogeneous immunoassay which can be performed by the automated analytical system of the present invention, in either a competitive or sandwich immunoassay format, is a microparticle capture enzyme immunoassay, such as that described in Clinical Chemistry, Volume 34, No. 9, pages 1726-1732 (1988), employing microparticles as the solid phase material.

In addition, the use of sucrose in microparticle diluent has been found to achieve neutral density of the microparticles. The methodology entails the determination of the optimum sucrose concentration which will eliminate the settling of microparticles. The sucrose concentration required to achieve neutral density is assay specific and microparticle lot specific. The principal involves dissolving sucrose in solution to increase the density of the diluent. When the density of the diluent and microparticles are equivalent, the microparticles will be in a suspended state. Density neutralization can also be achieved by using other materials such as metrizamide and/or metrizoic acid.

Separation of the bound and free species is accomplished by capture of the microparticles on a glass fiber matrix of an MEIA cartridge, a process that relies on the high affinity of glass fibers for the microparticles, wherein the microparticles adhere to the surface of the fibers irreversibly, and nonspecifically bound material can be effectively removed by washing the matrix. The matrix also provides a precisely located mechanical support for the microparticles during the optical quantification phase of the assay protocol as described herein.

When performing a sandwich immunoassay, microparticles coated with antibody to the analyte in the test sample are incubated with the test sample containing the analyte of interest to form a capture complex with the analyte from the test sample. A conjugate comprising antibody to the analyte labeled with a detectable moiety, preferably an enzyme, is then incubated with the capture complex to form the second of a sandwich complex. When performing a competitive immunoassay, microparticles coated with antibody to the analyte in the test sample are incubated with the test sample containing the analyte of interest and a conjugate comprising the analyte or analog thereof labeled with a detectable moiety, preferably an enzyme. Removal of unbound conjugate is accomplished with the glass fiber matrix of the MEIA cartridge and, where the detectable moiety is an enzyme, a substrate for the enzyme capable of providing a detectable signal is added and the signal provided thereby is measured and correlated to the amount of analyte present in the test sample. Preferably, the enzyme-substrate system employed by the competitive and sandwich MEIA formats is alkaline phosphatase and 4-methylumbelliferyl phosphate (MUP), although other enzyme-substrate systems known in the art can be employed as well.

The MEIA cartridge which is employed by the automated analytical system of the present invention comprises a reaction well for retaining and immobilizing microparticle-analyte complexes. The reaction well has an entrance port and means for holding a quantity of sample and assay reaction mixtures positioned over a fibrous matrix which retains and immobilizes microparticle-analyte complexes as described above. The fibrous matrix is composed of fibers having an average spatial separation greater than the average diameter of the microparticles. Preferably, the average fiber spatial separation is greater than 10 microns.

The reaction well further comprises an absorbant material positioned below the fibrous matrix to enhance the flow of sample and assay reaction mixtures through the fibrous matrix. Preferably, the absorbant material is a fibrous material whose fibers predominantly lie in a plane perpendicular to the lower surface of the fibrous matrix. The absorbant material is in fluid communication with the fibrous matrix. Generally, the absorbant material is in physical contact with the lower surface of the fibrous matrix. The interior of the reaction well, therefore, is generally sized or contains positioning means to maintain the fluid communication between the absorbant material and the fibrous matrix. Preferably, a spike located at the bottom of the reaction well can be used to force the absorbant material into contact with the lower surface of the fibrous matrix. Additionally, it is preferable to vent to the atmosphere the gases displaced in the absorbant material by the liquids absorbed therein during the performance of an immunoassay.

According to the immunoassay methodologies described above, standard solutions of the analyte of known concentrations covering the clinical concentration range are typically prepared and assayed as is the test sample to be assayed. This blank assay provides a series of signal measurements corresponding to the known concentrations from which a standard curve is drawn. The optical signal corresponding to the unknown sample is correlated in a concentration value through interpretation from the blank or standard curve.

Automated analytical methodology for effecting analysis of a plurality of test samples according to the present invention is achieved by introducing reagent packs, test sample container and reaction vessels onto concentric carousels of a main carousel. The test sample container can be a test tube, cuvette, vacutainer tube, and the like, for holding a test sample. The reagent packs and test sample containers are identified and aligned respectively with a reaction vessel for transfer and kitting of the reaction vessel by transfer of test sample and specific reagents from the reagent pack for preparation of a predetermined test. The reaction vessel containing the test sample and one or more reagents is transferred to a process carousel wherein controlled environment conditions exist for incubation once the sample has been appropriately mixed with various reagents to form a reaction mixture. When all assay processing steps have been completed, the reaction mixture is identified and transferred to at least, for example, one of a fluorescent polarization immunoassay reader or a microparticle enzyme immunoassay cartridge positioned on a separate cartridge wheel or carousel for further preparation before reading. The processed test samples are read and the readings are calculated with the resulting data being recorded and/or printed.

The methodology of the automated immunoassay analytical system is achieved through the use of a self-contained, fully automated, continuous and random access instrument comprising a main carousel assembly consisting of the reagent pack carousel, a reaction vessel carousel and a test sample container carousel concentrically and independently rotatable. The main carousel assembly is provided with a transfer pipette operated by a boom arm for transferring and kitting test sample and reagents into the reaction vessel automatically following a predetermined test schedule. The main carousel assembly is provided with bar code readers for reagent packs and test sample containers and has the capability of aligning the reagent pack carousel and test sample container carousel and a reaction vessel for pipette transfer operations. Once the assay to be performed is scheduled, the reaction vessel carousel, the reagent pack carousel and the test sample container carousel are rotated until the reaction vessel, a reagent pack and a test sample container, respectively, are determined to be in the transfer pipette access position. The transfer pipette then transfers the test sample from the test sample container and, depending upon the assay to be performed, the reagents from the reagent pack are transferred to the reaction vessel. The reaction vessel carousel is then rotated to a transfer station position which contacts the reaction vessel with a transfer mechanism and pulls the reaction vessel into the transfer station. The reaction vessel is then loaded onto the process carousel by the transfer mechanism.

When performing a fluorescent polarization immunoassay (FPIA) with the automated analytical system of the present invention, various pipetting activities are performed by a second transfer pipette apparatus which is in service for the process carousel, and the process carousel is rotated so that the reaction vessel, when properly pipetted with, for example, FPIA reagents, is at the read station of the FPIA processing stations and the FPIA determination on reading, is made on the reaction vessel. The process carousel is then rotated so that the read reaction vessel is at the transfer station. The reaction vessel is again contacted and transferred by the transfer station. The transfer station is rotated and pushes the reaction vessel into a release container opening.

For a microparticle enzyme immunoassay (MEIA) performed with the automated analytical system of the present invention, after the various pipetting activities for the MEIA, which can be completed at the main carousel assembly, the reaction vessel is transferred to the process carousel as described in the FPIA process. Pipetting can also be accomplished in the process carousel or jointly between the two carousels. To complete the MEIA, the reaction mixture is transferred from the reaction vessel to a matrix of an MEIA cartridge on a cartridge carousel with the second transfer pipette. The matrix is washed with a buffer and a substrate, such as MUP (defined earlier), or other suitable substrate known in the art. The cartridge carousel is then rotated so that the MEIA cartridge is positioned at an MEIA processing assembly and the MEIA determination is made. The MEIA reaction vessel is ejected into the waste container as described for the FPIA reaction vessel. The MEIA cartridge is independently ejected from the cartridge wheel by an ejector at an appropriate ejector station into a waste container.

Preferably, two distinct analytical technologies as described above, FPIA and MEIA, are incorporated into the automated analytical system of the present invention; however, more than two distinct analytical technologies can be incorporated into the inventive system. These methods are complimentary and share a commonality of apparatus and procedural steps, with the FPIA generally being the method of choice for analytes of low molecular weight and MEIA for molecules such as protein hormones, antibodies or analytes of low molecular weight requiring higher sensitivity. The two technologies share system components including the operator control panel, pipetting boom assemblies, fluidic systems, air and liquid reagent heaters, printers, bar code reader and stepper motors. Such commonality of use of system components allows for a compact instrument despite the dual FPIA and MEIA capability.

The FPIA optic systems (such as described in U.S. Patent No. 4,269,511 and incorporated herein by reference) can utilize a polarizing filter which is an electrically switched liquid crystal, maintaining a compact size and avoiding complex and potentially unreliable moving parts. When performing FPIA assays utilizing the automated analytical system of the present invention, the FPIA reagent packs will typically include a tracer comprising the analyte or analog thereof, coupled to a detectable moiety, an antibody specific to that analyte, and a specimen pretreatment reagent. In a preferred FPIA format, the analyte being determined competes with the tracer for a limited number of binding sites on the antibodies specific to the portion or portions of the analyte and tracer. The detectable moiety component of the tracer is preferably a fluorescent moiety selected from the group consisting of fluoresceins, aminofluoresceins, carboxyfluoresceins, fluoresceinamines, and the like, more preferably carboxymethyl-aminomethyl-fluorescein, carboxyethylaminomethyl-carboxyfluorescein, 6-carboxyfluorescein, 5-carboxyfluorescein, succinylanimomethyl-fluorescein, thioureaaminofluorescein, methoxytrianolylaminofluorescein, aminofluorescein, and the like.

In another embodiment, the FPIA format utilizes a unique, round, plastic, reaction cuvette suitable for fluorescence polarization and absorbance assay technologies which require no orientation other than top-to-bottom. This plastic reaction cuvette has physical characteristics of low birefringence throughout the optical read region as well as stringent dimensional tolerances which allow reproducible absorbance readings. Bifringence is defined as the degree of retardation of the extraordinary ray as it passes through a material. The greater the degree of retardation, the greater will be the level of birefringence. Retardation of the extra-ordinary ray is dependent on the magnitude and direction of the induced stress. Therefore, passing a ray of linearly polarized light through a material with induced stress will result in depolarization of the ray. In order for a cuvette to be utilized for fluorescence polarization measurements, it is important that the cuvette be prepared under conditions which yield minimum stress. The geometry of the cuvette has been designed to utilize the inherent fluidics of automated medical diagnostic instrumentation to minimize the hydrophobic effect of plastic.

MEIA results can be determined by quantifying the rate of fluorescence developed when fluorogenic substrate is converted by the action of an enzyme labeled conjugate. For example, when performing either a competitive MEIA or sandwich MEIA, the specifically bound alkaline phosphatase on the microparticles is detected by addition of the fluorogenic substrate MUP to the matrix. The alkaline phosphatase catalyzes hydrolysis of the MUP to inorganic phosphate and fluorescent 4-methylumbelliferone (4-MU). The liberated 4-mu is detected by the MEIA optics assembly front surface fluorometer which is designed to detect fluorescence of low concentrations of 4-MU without interference by fluorescence of 4-MUP at a wavelength of 367. A system of lenses and optical filters focus filtered light (wavelength = 365) from a mercury arc lamp on to the surface of the matrix and focus emitted fluorescence from 4-MU (wavelength = 448) on to a photo multiplier tube. Like the FPIA optics assembly, the MEIA optics system is compact and has no moving parts. About five percent of the excitation light is detected by a photodiode, allowing normalization of the fluorescence data and generation of a control signal used by the lamp power supply to maintain the intensity of the excitation light within five percent over the useful life of the lamp. The MEIA post-processor uses linear regression analysis to convert the data from multiple successive determinations of 4-MU fluorescence to a rate which is proportional to the concentration of alkaline phosphatase conjugate specifically bound to the microparticles.

MEIA formats can be run with a multi-position MEIA auxiliary carousel and process carousel as well as a MEIA reagent pack containing microparticle reagent, an alkaline phosphatase conjugate and, in some cases, a dilute buffer specific for the assay being performed. Because the microparticles tend not to settle out of suspension during the course of the assay, they can readily be pipetted. The effective surface area of polystyrene latex microparticles is several fold greater than that of a large diameter polystyrene bead (e.g., one quarter inch beads) commonly used in commercial immunoassays. Because of this large surface area and the very small diffusion distance between analyte and the capture molecules on the surface of the microparticles, the capture phase employed in many of the MEIA methods being performed reaches equilibrium within several minutes, allowing for a full carousel of test samples to be completed in a very short time frame.

Unlike an FPIA, the heterogeneous immunoassays, such as a MEIA, require a separation step as described above. In particular, after incubation of the microparticles with a test sample, the microparticles are separated from the reaction mixture by transfer to the matrix contained in the MEIA cartridge as described above. The matrix provides a precisely located mechanical support for
the microparticles during the subsequent optical read phase-of the assay. This precisely located mechanical support, i.e. the cartridge, is fit into the auxiliary carousel at a predetermined spacing from the reader apparatus by camming means.

### Detailed Description of the Drawings

Preferred embodiments of the automated immunoassay analytical system according to the present invention are presented only with those components of primary interest with respect to the inventive system apparatus and processes of the present invention. The drawings do not illustrate all of the mechanical and electrical elements for driving and controlling the various components of the system, wherein an of such omitted elements may have various known forms which can be readily realized by one of ordinary skill in the art having knowledge of the information provided herein with regard to the mode of operation of the system and the various components and related processes utilized for treating samples and determining analytical results.

Referring to the drawings, FIGURES 1 and 2 present isometric views of the automatic immunoassay analytical system apparatus of the present invention. The system apparatus as it appears in FIGURE 1 presents the system apparatus as used by the technician, with FIGURE 2 illustrating an isometric view of the frame and cabinetry with component parts removed. The system apparatus of the present invention is identified generally by the reference numeral 2 in FIGURE 1. The system apparatus 2 has an exposed front end carousel 4 which is serviced by a first transfer pipette mechanism 6 for kitting scheduled tests along with samples into a reaction vessel. The system provides a computer screen 8 and computer keyboard 10 along with access panels 1 2 for accessing storage and waste compartments. The system system apparatus 2 is provided with rollers 14 for movement of the system apparatus within a laboratory complex as required. The freedom of movement of the system apparatus through rollers 14 is allowed since the system is fully self-contained but for power requirements.

In FIGURE 2, the system apparatus 2 cabinet frame 16 is illustrated with substantially all functioning components of the system apparatus removed. A controlled environment zone 18 is a closed unit during operation with light shielding and rigid control of airflow as well as temperature as opposed to the open front end carousel 4. The front end carousel 4 communicates with the controlled environment zone 18 through a transfer port 20. The front end carousel 4 is mounted to an aluminum base plate which rests on a support platform 22 and the first transfer pipette mechanism is mounted on means 24.

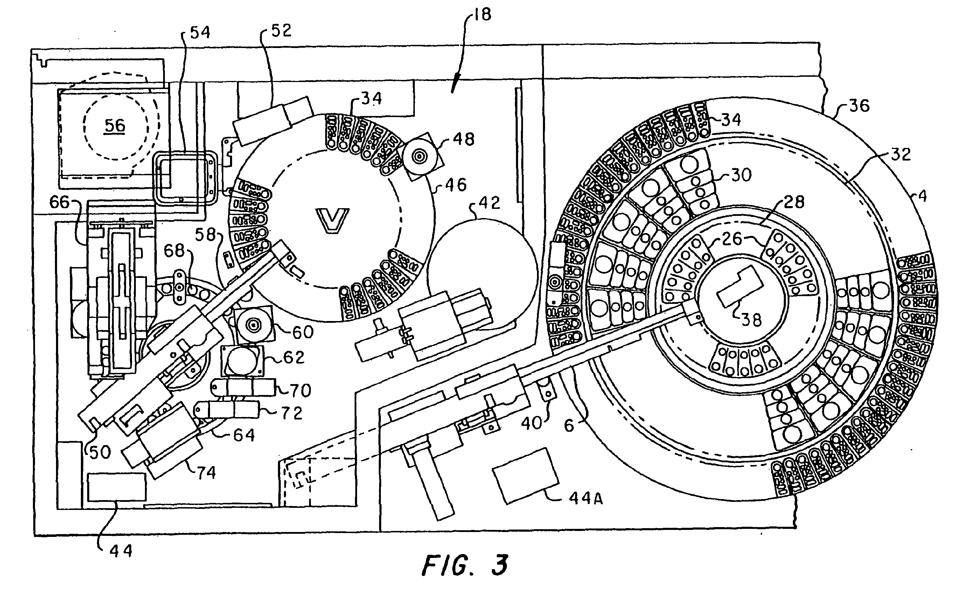

The top plan view in section of FIGURE 3 presents the functioning component system apparatus in some detail with relative positioning of the system apparatus to further illustrate the process flow of the system apparatus. For example, sample cups 26 are mounted on a sample cup carousel 28 which is concentrically fitted within the front end carousel 4 along with reagent pack carousel 32 and reaction vessel carousel 36. The reagent pack carousel 32 is concentrically fitted between the sample cup carousel 28 and the reaction vessel carousel 36. The reagent pack carousel carries reagent packs 30 and the reaction vessel carousel 36 carries reaction vessels 34. The front end carousel 4 has an operable bar code reader 38 for automatically identifying reagent pack carousel 32 and sample carousel 28. A wash cup 40 is provided for the first transfer pipette mechanism 6 for washing as required between transfer of various sample and reagents. The first transfer pipette mechanism 6 is utilized in kitting the various reagent pack liquid materials and sample into a reaction vessel 34. The reagents and the sample are properly kitted through means of the first transfer pipette mechanism 6 inclusive of pump means. The various carousels are rotated and aligned for kitting at the pipetting station. The kitted reaction vessel 34 is positioned by reaction vessel carousel 36 into the proper position for transfer to the transfer station 42. The reaction vessel 34 is transferred to the transfer station 42 through transfer means wherein the transfer station 42 is then rotated to move the reaction vessel onto process carousel 46. As shown, the process carousel is driven by a stepper motor 48 and is serviced by a second transfer pipette mechanism 50. Both the FPIA and MEIA procedures utilize the system apparatus commonly up through and including the process carousel 46. The process carousel 46 includes FPIA processing 52 and FPIA processing lamp 54 for direct reading of FPIA analysis of kitted, pipetted and properly reacted reagents sample from the reaction vessel 34. The controlled environmental zone 18, which includes the transfer station 42 and process carousel 46, provides FPIA processing with air circulation under temperature control by cabinet air circulation fan 56. A wash cup 58 for the second transfer pipette mechanism 50 is provided. The second transfer pipette 50 is utilized for adding reagents (pipetting) under conditions of incubation and timing to the sample in the FPIA test schedule reaction vessel 34 for FPIA processing. MEIA processing can also utilize the second transfer pipette 50 for adding reagents to the sample before the reaction mix is added to MEIA cartridges 68 which are mounted on the cartridge wheel carousel 64. The transfer of the MEIA reagent mixed sample to the MEIA cartridge 68 is by the function of the second transfer pipette 50. A motor 60 drives the cartridge wheel 64. The cartridge wheel 64 is provided with MEIA cartridges 68 through the operation of a cartridge hopper 66 which automatically feeds and positions the MEIA cartridges 68 onto the cartridge wheel 64. The process area includes the second transfer pipette mechanism 50 and heater/pump 44. The cartridge wheel carousel 64 is further serviced by a MEIA buffer heater and dispenser 70, MUP heater and dispenser probe 72, and MEIA reader 74. The MEIA cartridges 68 are removed from the cartridge wheel 64 by a cartridge ejector 62 after the MEIA read has been completed.

It is to be understood that the utilization of the first transfer pipette mechanism 6 and the second transfer pipette mechanism 50 as described herein provide a safety mechanism to ensure that test samples and reagents are pipetted to thereby prevent false negative results in the event there are incorrect amounts of the respective sample and reagents for a particular assay.

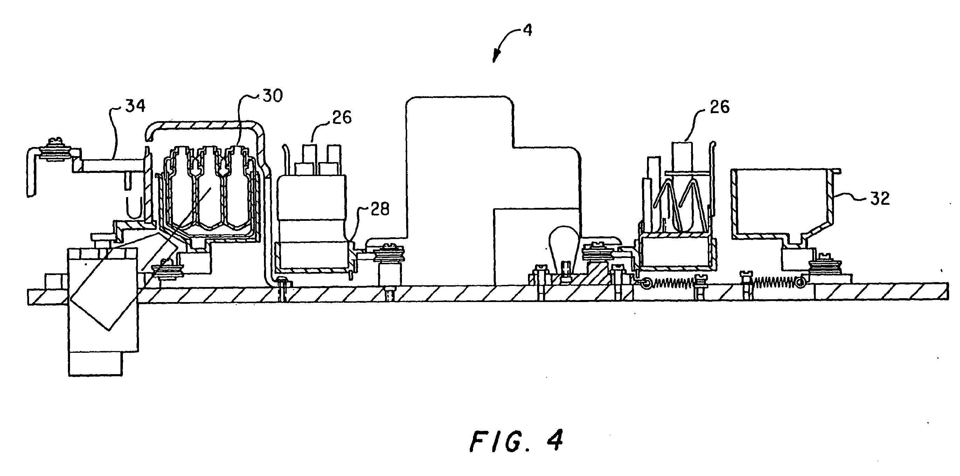

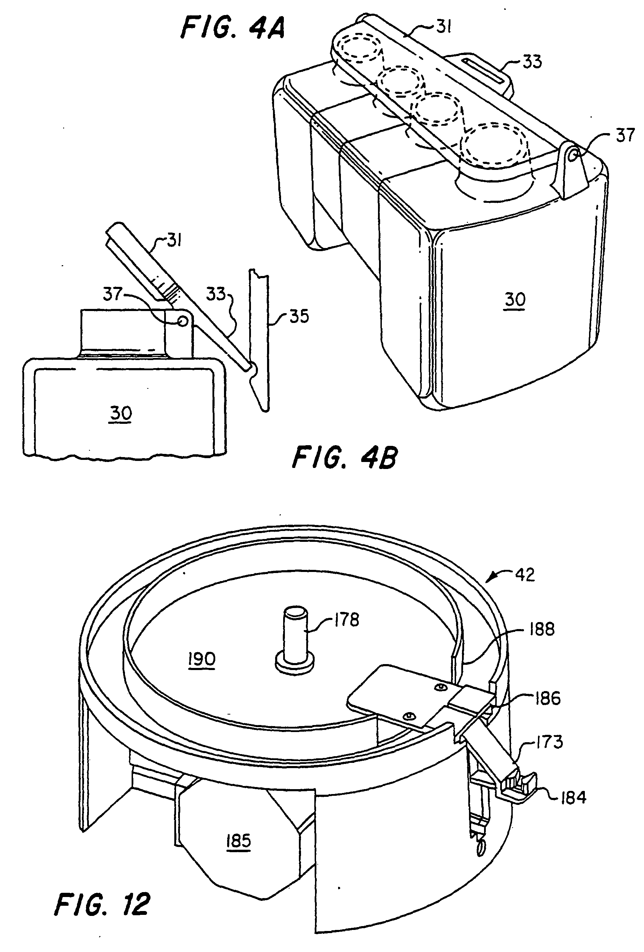

Approaching the operable elements of the system apparatus in greater detail, FIGURE 4 provides a front elevational view in isolation and partial section of elements of the front end carousel 4. FIGURES 4A and 4B illustrate a reagent pack with a cover means 31 which is opened and closed pivoting along axis 37. A return notched drive arm 35 is utilized to open and close the cover means 31 by contact with the cover contact surface 33.

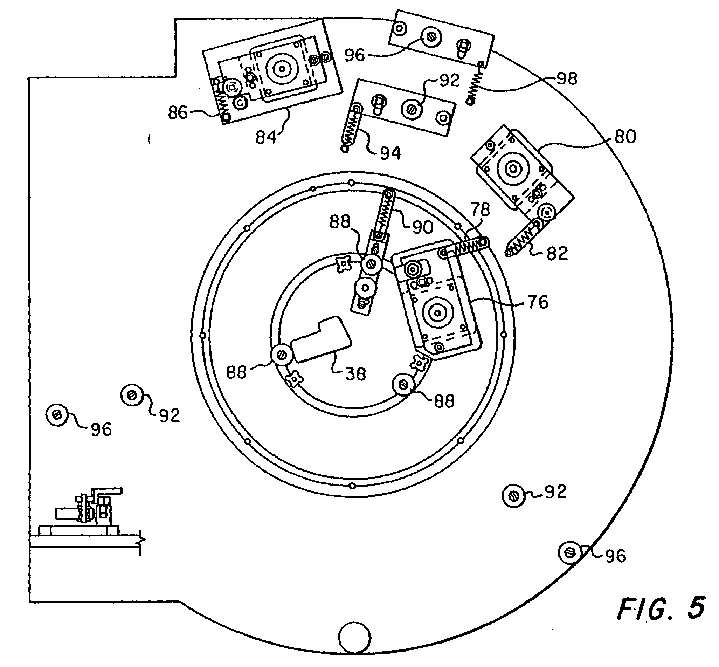

FIGURE 5 provides a top view in isolation and partial section of elements of the drive and guide systems of the main carousel 4 with the various carousels removed. In FIGURE 5 a sample cup carousel stepper motor 76 is shown mounted with mounting spring 78. The reagent pack carousel motor 80 is also shown with a mounting spring 82. The reaction vessel carousel motor 84 and mounting spring 86 are positioned to the exterior of the two inner carousels, i.e. the sample cups carousel 28 and the reagent pack carousel 32. Roller guides 88 are provided for the sample cup carousel 28 and a tensioning spring 90. The reagent pack carousel is provided with roller guides 92 and tensioning means 94. The reaction vessel roller guides 96 are also provided with spring elements 98, the purposes of the guide and these various spring elements being to maintain very finite tracking of the concentric carousels when motivated by the individual stepper motors.

A non-microprocessor based stepper motor controller with ramping and error detection capabilities are presented. The controlling is performed by programmable sequencer/controller integrated circuits. The devices are programmed by calculating a profile using a base velocity, final velocity, acceleration and number of steps. The profiles can be symmetrical or asymmetrical and various error detection schemes can be incorporated. All motor movement sequences and error detection schemes are affixed in hardware and can be implemented/initiated by simply changing the status of an input BIT.

Stepper motors and linear actuators are used in a variety of applications requiring precise rotation and/or linear movement as in automated, continuous and random access analytical systems. Stepper motors are two-phase permanent magnet motors which provide discreet angular movement every time the polarity of a winding is changed.

The control and drive circuitry for such a motor can be implemented digitally by using a PAL with high current driver buffering on the output. For example modern systems have presented need in error detection and correction techniques in managing and controlling stepper motors for whatever the broad applications may be.

Non-microprocessor based stepper motor controller with ramping and error detection is presented according to the invention wherein the controlling is performed by programmable/controller integrated circuits. The devices are programmed by calculating the profile by using the base velocity, final velocity acceleration and number of steps.

The functions provided by the method and apparatus are: step pulses; direction control; power control; done status; home status; and error status. The profiles can be symmetrical or asymmetrical with various error detection schemes which can be incorporated with the use of sensors to detect motor or mechanism position. All motor movement sequences and error detection schemes are affixed in hardware and can be implemented or initiated by simply changing the status of an input BIT. Such simple modifications by changing the status of an input BIT substantially reduces hardware requirements of the system.

For example, the following mechanisms can be driven by indexers, those mechanisms being transfer mechanism R-access ejector, trap door and shuttle. The velocity and acceleration profile will be affixed in hardware. Base velocity, final velocity, acceleration and total number of steps will be required for the profile. The profile will be a linear staircase and may be symmetrical or asymmetrical. The maximum number of staircase steps available over an entire profile will be forty. The indexer will require single input BIT to start an action. The action performed is defined as follows:
If not home, then move towards home at a predefined base rate until the home sensor is found.
If home, then ramp out a predefined number of steps, delay for a fixed period of time, then ramp back a predefined number of steps.

The indexer will have a single output BIT that will be asserted upon completion of the motor movements defined above. This BIT will remain asserted until a new motor movement command is requested. The indexer will provide a step BIT, direction BIT and power high/low BIT to the motor drive of choice. The home flag output will be routed to an indexer input for detection and determination of appropriate motor movement.

The front end carousel 4 inclusive of the three front end carousels, the sample cup carousel 28, reagent pack carousel 32 and reaction vessel carousel 36 can by example contain the following capacities. The sample cup carousel 28 can hold 60 blood collection tubes, such as Vacutainer blood collection tubes, or 90 sample cups which are injection molded as one piece and can be provided with standalone base mounts. Standalone base mounts are suitable for technician handling and pipetting of samples into the sample cups. The reagent pack carousel 32 provides for 20 different reagent packs 30. The reaction vessel carousel 36 provides 90 reaction vessels 34.

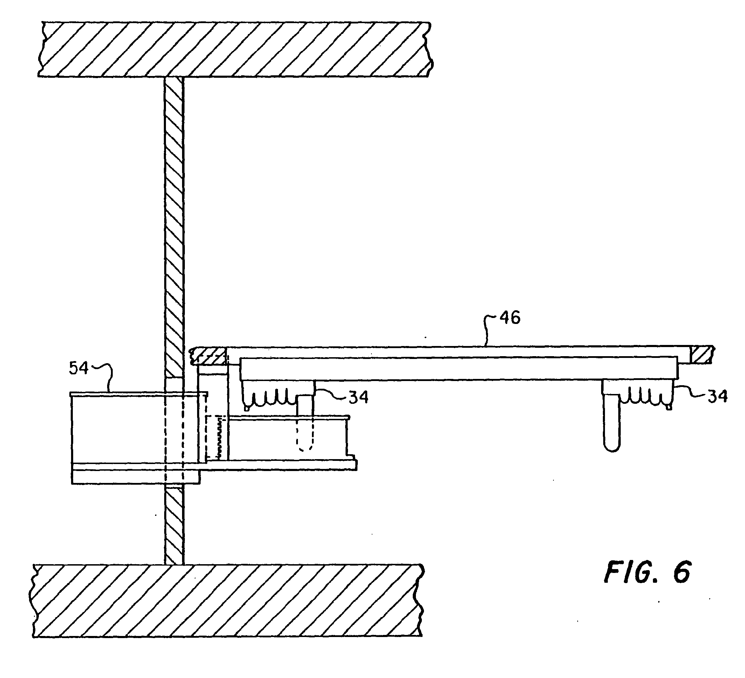

The process carousel 46 as shown in FIGURE 6 is an isolational cross-sectional side view. One reaction vessel 34 is at rest or nonoperative position and a second reaction vessel 34 is in position for FPIA read. The process carousel 46 is capable of bidirectional motion for timely movement of the various reaction vessels 34 to pipettor action, read, or transfer to and from the carousel. Up to about 36 or more reaction vessels 34 can be processed at one time on the process carousel 46 depending on diameter and sizing of the reaction vessels 34.

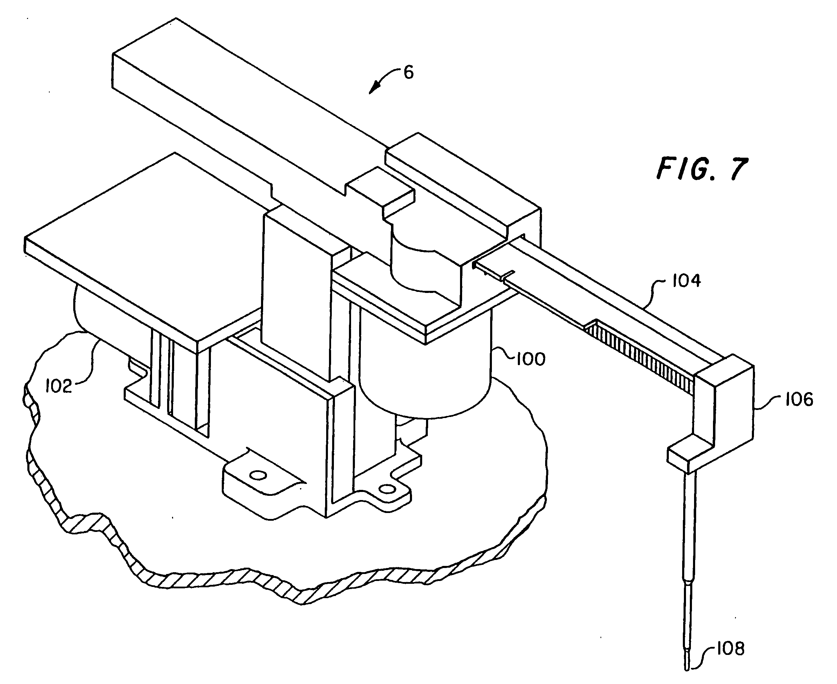

The first transfer pipette mechanism 6 of FIGURE 7 includes a transfer pipette Z axis motor 102 which moves the probe arm 104, probe 106 and probe tip 108 in a vertical direction while transfer pipette R axis motor 100 drives the probe arm 104, probe adjustment means 106 and probe tip 108 in a horizontal motion. The first transfer pipette mechanism 6, sometimes labeled "Sample Probe Arm Mechanism", moves the probe between the sample cup 26, the reagent pack 30, the reaction vessel 34 and the wash cup 40. The wash cup 40 is used to wash the interior and exterior surfaces of the first transfer pipettor mechanism 6 probe. The drive of the first transfer pipette mechanism is a rack-and-pinion drive means along the Z and R axis by two- stepper motor drivers. A brake is provided to hold the Z axis position when power is lost, thus avoiding damage to the system apparatus. For example, the first transfer pipette mechanism can be designed to have a Z axis travel of about 3 inches and an R axis travel of about 11 - 1/2 inches.

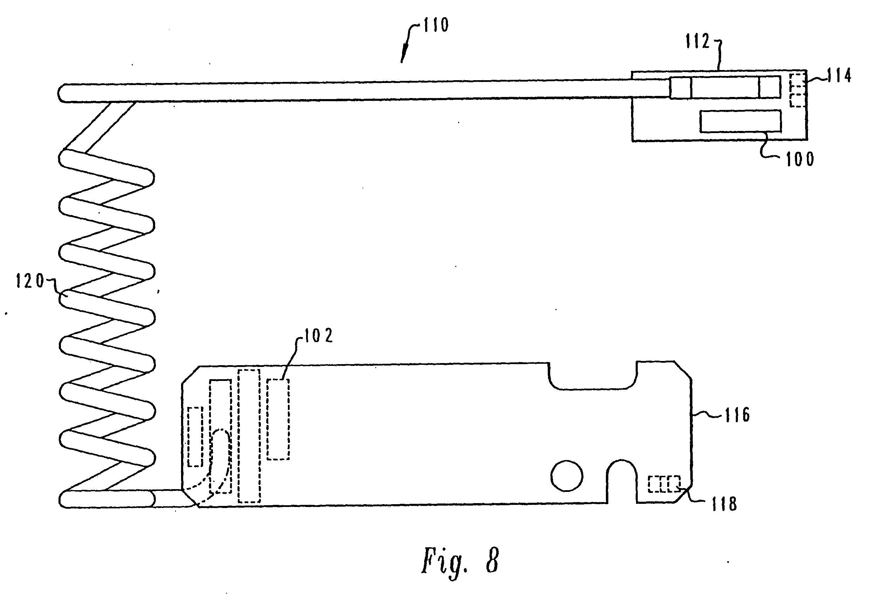

The first transfer pipette mechanism 6 and the second transfer pipette mechanism 50 are closely related in general system apparatus function and design, with variation on travel and size being the only substantial differences. Both units have a probe arm circuit 110 as illustrated by the schematic side view of FIGURE 8. The schematic illustrates the R axis motor 100 and the Z axis motor 102 in relationship to an upper PCB 112 and a R axis home sensor 114. A lower PCB 116 is illustrated in relationship to the Z axis home sensor 118 with a coil cable 120 connecting the various elements.

The bubble flushing aspirating and dispensing syringe according to the invention can be used in any automated analytical system or other applications wherein fluidics are utilized in processes which are dependent on the precision and accuracy with which a syringe can aspirate and dispense fluids. A syringe which has the capacity for automatically flushing bubbles completely out of the fluidics system can achieve and maintain such precision and accuracy. The syringe according to the invention is configured such that a piston reciprocates through a seal into a close-fitting bore, the bore having a closed end and the bore and piston defining an annulus which is in communication with fluid inlet and outlet means. Fluid is introduced into the annulus around the piston creating a cross-flow flushing bubbles from the annulus. While the cross-flow is occurring, the piston is reciprocated within the bore. The reciprocation causes high fluid velocities in the annulus between the piston and the bore. The high flow velocity dislodges any bubbles that may be adhering to the piston or bore wall. When the piston strokes to its full inward extension, it comes very close to the bore end, thus dislodging any bubbles stuck on the bore end which are swept upon withdrawal of the piston to the outward extension.

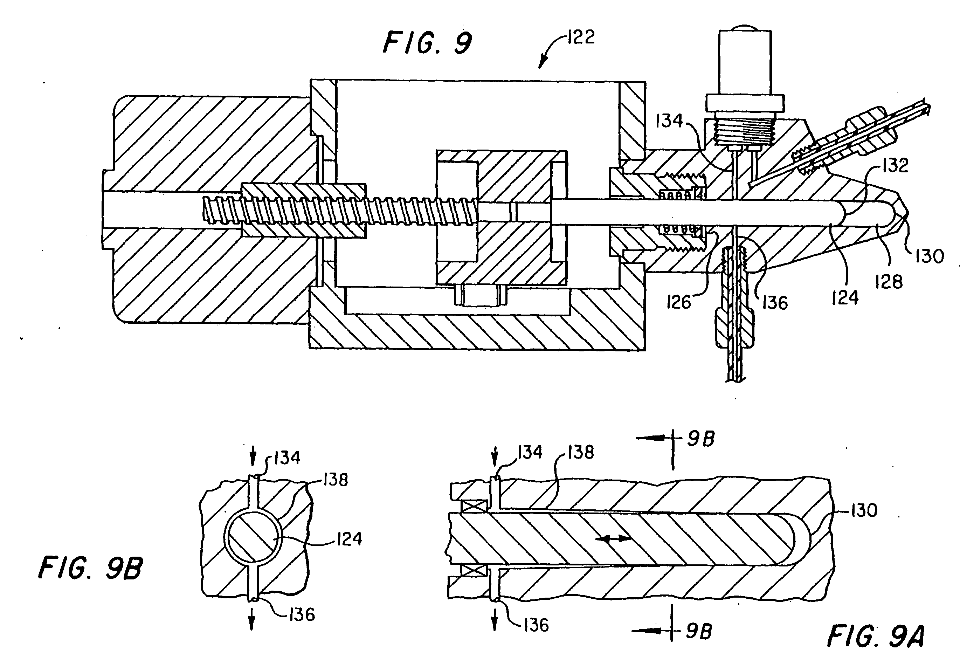

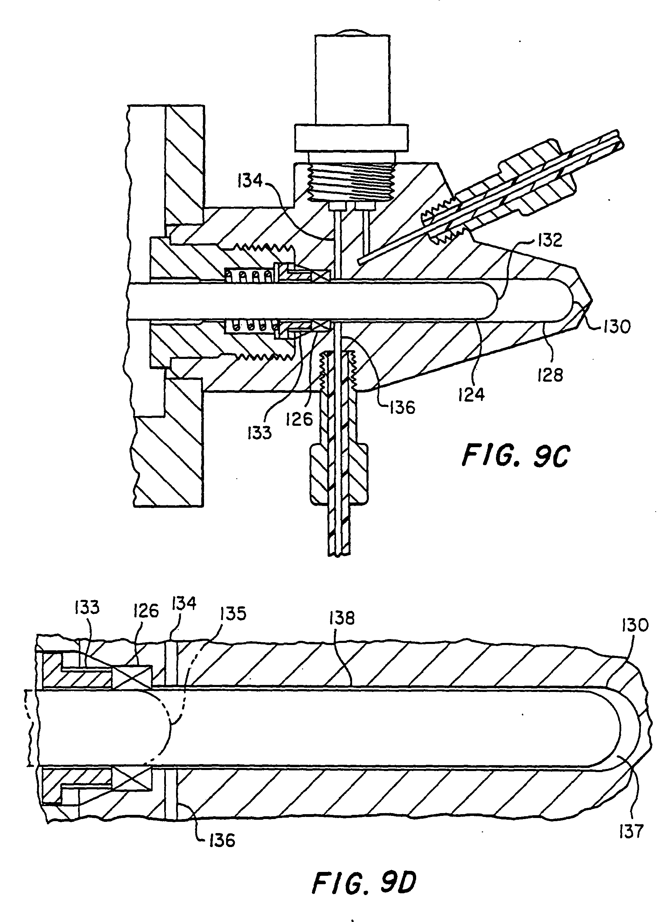

Various elements of syringe 122 which provides automatic bubble flushing and fluids to the various pipetting mechanisms is provided in various views in FIGURES 9, 9A and 9B. The ability of diagnostic instrumentation to accurately perform an assay is critically dependent on the precision and accuracy with which syringes, i.e. pipetting, can aspirate and dispense reagents and samples. The precision and accuracy of a syringe is severely degraded by the presence of small air bubbles inside a syringe. Bubbles, unfortunately, are all too common and are difficult to remove or avoid. Syringe 122 avoids these problems by automatically flushing bubbles completely out of the fluidics system. The syringe 122 is configured such that a piston 124 reciprocates through a seal 126 and into a close-fitting bore 128. The end of the bore 130 is closed. The piston 124 has a piston end 132 which approximates the geometry of the closed bore end 130. Two ports to the bore are 1800 apart and are located near the seal and are comprised of a fluid entry port 134 and a fluid exit port 136. An annulus 138 exists between the piston 124 and bore 128. Pressurized line diluent is introduced to the fluid entry port 134. The fluid flows out into the annulus 138 around both sides of the piston 124 and then into the fluid exit port 136. This crossflow flushes bubbles from the area near the seal. While the crossflow is occurring, the piston 124 is reciprocated inside the bore 128. This reciprocation causes high fluid flow velocities in the annulus 138 between the piston 124 and the bore 128. The high flow velocity dislodges any bubbles that may be adhering to the piston 124 or bore wall. The inward stroke of the piston 124 pushes these dislodged bubbles across the crossflow area where they are swept out of the syringe. The piston end 132 and the bore end 130 have similar spherical shapes. When the piston 124 strokes to its full inward extension, it comes very close to the bore end 130. Any bubble that may be stuck on the bore end 130 is disrupted and dislodged. Likewise, when the piston strokes to its full outward extension, its end is flush with the seal 126. The sequence of reciprocating the piston while crossflowing can be automatically executed any time by the system apparatus.

Once the fluid leaves the fluid exit port 136 of the syringe 122, it must travel through a tube fitting, through a length of tubing, through another tube fitting, into a probe 106 and out the probe tip 108. It is at the probe tip 108 that the aspirating and dispensing of reagents actually occurs. Any bubbles trapped between the syringe and the probe tip will also degrade performance, so there must be no place for the bubbles flushed out of the syringe to lodge. It is therefore necessary to use zero dead volume tubing fittings on the tubing between the syringe and the probe.

In operation of the bubble flushing aspirating and dispensing syringe of the present invention, initial withdrawal velocity of the piston from the at rest or home position is slower than the velocity of the piston as it approaches total withdrawal position. This type of manipulation of the piston action in relationship to the end of the bore avoids high vacuum and bubble creation within the bore. On the other hand, the piston can be withdrawn from the home position at full speed in order to expedite removal of preformed bubbles in the end of the bore. After such bubble flushing procedures, the valves are closed and aspiration can be perfected. If, however, the syringe is used for dispensing, the valve can be open for metered amounts of liquid for dispensing purposes.

In particular, the partial cross sectional side elevation view as illustrated in FIGURE 9C of the automatic bubble flushing syringe apparatus presents the piston 124 in a position of travel between fully withdrawn and at home position within the close-fitting bore 128. The piston 124 reciprocates through seals 126 which are springloaded and carried by a polyethylene wear ring 133, the seals 126 being comprised of an O-ring over the polyethylene wear ring. The cross sectional view in isolation of the syringe bore end portion of the automatic bubbles flushing syringe with the reciprocating piston near the end of travel toward the bore end portion or at home position according to FIGURE 9D also shows in phantom the piston 124 in a fully withdrawn position within the bore 130. When the piston 124 is fully withdrawn, the piston tip 132 is slightly beyond the cross sectional flow of inlet 131 and outlet 136. When the piston 124 is in a home position 137 the piston end 132 is very close to the bore end 130.

The syringe 122 configuration can be, but is not intended to be limited to, about 8.3" long, 3.5" wide and about 2.7" deep. A linear actuator 125 is mounted to the frame 123. An actuator motor spins a nut means 127 into which a mating lead screw 133 is screwed. The lead screw 133 is clamped to the coupler 129 which has a bearing 131 mounted on its bottom side. The bearing 131 runs in a groove in the frame 123. Since the coupler 129 is rotationally constrained by the bearing 131, the coupler 129 reciprocates when the linear actuator 125 motor spins the nut and the piston 124 which is clamped into the coupler 129, thus reciprocating the piston 124 through the seal 126. In addition, the bubble flushing aspirating and dispensing syringe of the present invention eliminates air bubbles which otherwise create inefficiencies in the precision and accuracy of dispensing and aspirating fluids by automatically and completely flushing bubbles from the fluidics system. To perform a bubble flush operation, the valve is opened and the piston 124 is reciprocated through its full stroke at least once, preferably from between about five and about ten strokes. The fluid flows around both sides of the piston 124 or across the bore and then reconverges at the fluid exit port 136. This cross flow pattern flushes bubbles in the area of the seal 126 out through the fluid exit port 136. The clearance between the piston and the bore is small and, according to a preferred embodiment, from between about 0.002" and about 0.008". When piston 124 reciprocates, very high flow rates are generated in the annulus 138 between the piston 124 and the bore 128. These high flow velocities flush bubbles in the bore 128 to the seal area where they are swept out of the syringe 122 by the cross flow. Zero (0) dead volume fittings are positioned between the syringe 122 and the tip release means to ensure that bubbles flushed out of the syringe 122 have no place to lodge as they are swept down the communicating tube and out the tip.

In one embodiment, deionized water under pressure is supplied to the fluid entry port 134 of the syringe 122. The water passes through a two-way solenoid valve and is ported to one side of the piston bore or across the bore when the piston tip is fully withdrawn. When the valve is open, such as, for example, during certain fluid flow requirements, the fluid flows from this entry port 134, around both sides of the piston, i.e., the annulus 138 or across the bore and out through the fluid exit port 136. The fluid then flows through conduit means to an open ended tip. When the valve is closed, reciprocating the piston causes fluid to be aspirated or dispensed at the tip.

In another embodiment, the fluid entry port 134 and fluid exit port 136 are about 180° apart and located near the seal between the seal 126 and bore end 130. Pressurized fluid is introduced to one of the ports. The fluid flows through such a port around both sides of the piston, i.e., the annulus defined by the piston and the bore into the opposite port or across the bore. This cross flow flushes bubbles from the area near the seal. While the cross flow is occurring, the piston reciprocates inside the bore, wherein the inward stroke of the piston pushes dislodged bubbles toward cross flow area where the bubbles are swept out of the syringe. In still another embodiment the piston 124 has an end configuration which is similar to that of the bore end 130, i.e., a similar spherical shape. When the piston 124 strokes to its full inward extension, the piston comes very close to the bore end 130. Any bubble that may be adhered to the bore end 130 is dislodged. When the piston 124 strokes to its full outward extension, the end of the piston is flush with the seal 126. The conical end of the piston is thus between the fluid entry port and the fluid exit port and any bubbles that may be adhered to the tip of the piston are swept off by the cross-flow. This sequence of reciprocating the piston 124 while cross flowing can be automatically executed at any time by the instrument.

It is to be understood that the automatic bubble flush syringe of the present invention can be used in any situation where the precise manipulation of fluids is desired, whether the syringe is operated manually or by an automated instrument, including, but not intended to be limited to, precision aspirating and dispensing of fluids such as found in many medical diagnostic instruments and devices, precision analytical pipetting, and the like situations where precision manipulation of various volumes of fluid is needed, particularly small volumes of fluid. In addition, the inclusion of a second valve downstream of the syringe converts the syringe into a precision positive dispiacement pump.

According to another embodiment of the present invention, a bubble flushing, aspirating and dispensing apparatus is provided which can be used in any automated analytical system or other applications where fluidics are utilized in processes which are dependent on the precision and accuracy with which fluids are aspirated and dispensed. In particular, the apparatus of the present invention is a syringe-like device which has the capacity for automatically flushing all, or substantially all, bubbles from the fluidics system so that it can perform precise and accurate manipulation of fluids. The syringe is configured such that a piston reciprocates through a seal into a close-fitting bore, the bore having a closed end and the bore and piston defining an annulus which is in communication with fluid inlet and outlet means. Fluid is introduced into the annulus around the piston near the seal creating a cross flow flushing bubbles from the annulus. While the cross-flow is occurring, the piston is reciprocating within the bore. The reciprocation causes high fluid velocities in the annulus between the piston and the bore. The high flow velocity dislodges any bubbles that may be adhering to the piston or bore wall. When the piston strokes to its full inward extension, it comes very close to the bore end, thus dislodging any bubbles adhered to the bore and which are swept upon withdrawal of the piston to the outward extension. Likewise, when the piston strokes to its full outward extension, the crass-flow sweeps bubbles from the spherical end of the piston.

The syringe of the present invention is particularly useful with an automated analytical system which is capable of simultaneously performing two or more assays on a plurality of test samples in a continuous and random access fashion, such as the system described in greater detail herein. In particular, the automated immunoassay analytical system apparatus of the invention can be viewed as a microprocessor based system of integrated subassemblies with different groups of assays being run through separate and changeable software modules. The microprocessor based system uses robotic arm pipetters with two degrees of freedom and bidirectional rotating carousels to process samples. Critical assay steps such as incubations, washes and specimen dilution are performed automatically by the instrument as scheduled.

According to the invention, automated, continuous and random access analytical system capable of simultaneously effecting multiple assays of a plurality of liquid samples is provided, and enables performing a method wherein various assays are scheduled for a plurality of liquid samples. Through kitting means the present system is capable of creating a unit dose disposable by separately transferring liquid sample and reagents to a reaction vessel without initiation of an assay reaction sequence. From the kitting means multiple, kitted unit dose disposables are transferred to a process area, wherein an aliquot is mixed for each independent sample with one or more liquid reagents at different times in a reaction vessel to form independent reaction mixtures. independent scheduling of such kitting and mixing is achieved during incubation of the multiple reaction mixtures. Independent scheduling of such kitting and mixing is achieved during incubation of the multiple reaction mixtures, simultaneously and independently.

Contemplation of automated, continuous and random access analytical systems for simultaneously performing at least two different types of assays requires reaction vessel apparatus having multiple uses and requiring various handling devices. The need to provide an automated analytical system having continuous and random access capabilities is accomplished through the utilization of the reaction vessel and various reaction vessel handling devices.

The reaction vessel 34 is discussed in detail relative to either the MEIA scheduling or the FPIA scheduling in FIGURES 10, 10A, 10B and 10C. FIGURES 10 and 10A present the FPIA kitting utilization wherein cuvette 140 is illustrated in both the top plan view, FIGURE 10, and the side view, FIGURE 10A. S reagent antiserum is deposited in well 142 while T reagent tracer is deposited in well 144 with P reagent popper being deposited in well 148. Wells 150 and 152 can serve for providing a variety of reagents, buffers and/or dilution liquids to the apparatus. The sample is deposited in well 148 and predilution liquid in well 154. The utilization of the transfer pipettor in depositing the required reagents into a reaction vessel along with the sample is called kitting. The depositing of the various required reagents and the like into a single reaction vessel along with a sample to be analyzed is called pipetting.

The MEIA reaction vessel as shown in top and side views of FIGURES 108 and 10C, respectively, contains prediluent in well 156; microparticle materials being deposited in well 158; conjugate directly in the reaction well 166; assay diluent in well 162; and the sample in well 164. The buffer well is 168 and predilution well is 170. Once kitting is complete, many of the subsequent FPIA and MEIA pipetting steps can be performed either in the main carousel or in the process carousel utilizing the pipetting mechanisms of both carousels. This is possible because the kitted reaction vessel, once kitted, is transferred immediately into the transfer station and thus into the process carousel which exists in a controlled temperature environment.

The isometric view of Figure 10D of the reaction vessel loading strip 175 in section having two reaction vessel 34 mounted thereon illustrating the continuous strip upper portion handling ledge segments 177 which are separated by ledge cut-outs 179. Each ledge segment 177 coincides with a reaction vessel mounting means 182 which are on a lower portion of the continuous strip wall 181. The reaction vessel mounting means 182 present for mounting of each reaction vessel flexible leg portions 183 having double fin sets 187 on each leg portion 183. The double fin sets 187 project perpendicularly from the plane of the strip continuous wall 181 and leg portions 183. The leg portions 183 are flexible and in combination with the double fin sets 187 allow for firm holding of the reaction vessels when mounted on the reaction vessel loading device strip and yet release the vessels when inserted into the reaction vessel carousel.

A top view of the reaction vessel loading device strip 175 having ten reaction vessels 34 mounted thereon is shown in Figure 10E. The reaction vessels 34 in the top view of Figure 10E presents the various chambers of the reaction vessel which have been identified only for purposes of simplicity for FPIA utilization as indicated in Figure 10. It is really apparent that the MEIA utilization as illustrated in Figure 108 could also be presented in relationship to Figure 10E. The reaction vessels 34 are mounted on the reaction vessel holding device strip 175 through assertion of the reaction vessel mounting means 182 into well 152 as identified for purposes of FPIA utilization. The same well is identified as 168 for MEIA utilization. The reaction vessel holding device strip is utilized for loading multiple reaction vessels at one time into the reaction vessel carousel by arcing the strip continuous wall 181 to correspond to the radius of curvature of the reaction vessel carousel. Ten reaction vessels are shown in Figure 10E attached to one continuous strip wall 181; however, the reaction vessel loading device strip 175 can be expanded in length to accommodate more than ten reaction vessels or less than ten reaction vessels can be mounted on the same length strip or reduced strip lengths.

The reaction vessel loading device is comprised of a semi-rigid plastic strip which holds up to ten or more reaction vessels at one time for loading the reaction vessels onto the reaction vessel carousel. An operator would bend the strip into an arc which matches the radius of the carousel. Then the ten or more reaction vessels mounted on the semi-rigid plastic strip are inserted into their respective slots on the carousel. The multiple reaction vessels are snapped into place on the carousel with the reagent vessel loading device strip then being removed for reuse or for discarding.

Unit dose disposable reaction vessels play an important role in automated, continuous and random access analytical systems which are capable of simultaneously performing at least two different forms of assays on a plurality of test samples in a continuous and random access fashion, with generally one reaction vessel required for each assay, wherein multiple reaction vessel are utilized by such systems. In particular, the automated immunoassay analytical system apparatus can be viewed as a microprocessor based system of integrated subassemblies with different groups of assays being run through separate and changeable software modules. The microprocessor based system uses robotic arm pipetters with two degrees of freedom and bidirectional rotating carousels to process samples. Critical assay steps such as incubations, washes and specimen dilution are performed automatically by the instrument as scheduled. Means for handling and loading such reaction vessels in multiple units into the reaction vessel carousel is particularly useful for the uniform and continuous operation of the system by the operator. According to the present invention, such handling and loading means is a reaction vessel loading device comprising a semi-rigid plastic planar cover having an upper planar surface with spaced apart reaction vessel opening shaped depressions which are insertable into the reaction vessel openings. The reaction vessel loading device is preshaped having substantially the same curved dimensions and spacing of the reaction vessel positioning as found on the reaction vessel carousel. The reaction vessel loading device is provided with additional depressions or protrusions from the reaction vessel opening depressions of the loader which snap into at least one of the reaction vessel wells and cuvette of the reaction vessel. The reaction vessel loading device provides loading capability of a plurality of reaction vessels at one time into a reaction vessel carousel as well as providing for dust cover means for the plurality of reaction vessels before, during and after loading into the reaction vessel carousel. In particular, the reaction vessel loading device provides a cover before loading and use of the reaction vessel by the automated system. The reaction vessel well protrusion, as well as the cuvette protrusion, provide a diminished sizing as the protrusion extends perpendicularly from the plane of the reaction vessel loading device for ease of insertion into the reaction vessels, as well as ease of removal from the reaction vessels once the reaction vessels are snapped into place on the reaction vessel carousel.

The isometric view of FIGURE 10D' of the reaction vessel loading device 450 in section having two reaction vessels 34 mounted thereon illustrates the reaction vessel loading device planar surface 452 which provides a continuous surface or dust cover means. The reaction vessel loading device 450 planar surface 452 has spaced apart therein reaction vessel loading device insertion depressions 454 which are suitable for fitting into the opening of reaction vessels open end portion. The reaction vessel insertion depressions also provide a further depressed protrusion means 456 which is spaced to fit into at least one well of the reaction vessel. In addition, the reaction vessel insertion depression 454 has an additional depression protrusion 458 for fitting into the reaction vessel cuvette 140. Planar surface 452 of reaction vessel loading device 450 has a continuous elevated rim 460 which defines the outer parameters of the planar surface 452 of reaction vessel loading device 450. The continuous elevated rim 460 terminates in a substantially flat surface 461 which is parallel to the reaction vessel loading device planar surface 452. At each end of the curved lengths of the reaction vessel loading device 450 are elevated handling fins 462 and 464. These elevated handling fins allow for withdrawal and handling of the reaction vessel loading device 450 which are more clearly illustrated in FIGURE 10E'.

The top view of the reaction vessel loading device 450, having ten reaction vessel counting means thereon, is shown in FIGURE 10E'. The reaction vessels 34 shown in hidden line presentation in the top view of FIGURE 10E' presents the various chambers of the reaction vessels 34 which have been identified only for purposes of simplicity for FPIA utilization as indicated in FIGURE 10. It is readily apparent that the MEIA utilization as illustrated by FIGURE 10B could also be presented in relationship to FIGURE 10E'. The reaction vessels 34 are mounted on the reaction vessel loading device 450 through insertion of the reaction vessel loading device planar surface 452 and corresponding reaction vessel well fitting protrusions 456 and reaction vessel cuvette fitting protrusions 458. The reaction vessel loading device is utilized for loading a plurality of reaction vessels at one time into the reaction vessel carousel by downward pressure on the reaction vessel loading device 450 which corresponds to the radius of curvature of the reaction vessel carousel. For example, and as shown in FIGURE 10E, ten reaction vessels can be attached to one continuous reaction vessel loading device. However, it is to be understood that the reaction vessel loading device 450 can be expanded in length to accommodate more than ten reaction vessels, or less than ten reaction vessels can be mounted on the same length of reaction vessel 450 or a reaction vessel loading device of reduced dimension.

The reaction vessel loading device comprises, for example, a semi-rigid plastic planar surface 452 preferabiy having ten or more reaction vessel insertion depressions 454 for loading ten or more reaction vessels at one time onto the reaction vessel carousel. An operator is not required to use extraordinary care in shaping the loader since the loader is preshaped to fit the dimensions of the reaction vessel carousel. In this regard, the reaction vessel loading device 450 a "drop in" type of insertion and loading of reaction vessels mounted on the loader into the reaction vessel carousel. Multiple reaction vessels are snapped into place on the reaction vessel carousel with the reaction vessel loading device then being removed for reuse, or can be discarded. In addition, the reaction vessel loading device can remain mounted on the reaction vessels which are loaded into the reaction vessel carousel for purposes of maintaining dust cover protection until used as described herein.

Since dust or other contaminants can, for example, affect assay performance, a prepackaged, reaction vessel mounted reaction vessel loading device provides from point of manufacture a dust or contaminant cover for the reaction vessels until the reaction vessels are actually loaded into the reaction vessel carousel which eliminates possible contamination. Once inserted into the reaction vessel carousel's respective slots, the reaction vessel loading device with the correspondingly mounted reaction vessels are pressed down by the pressure on the reaction vessel loading device upper planar surface until the reaction vessels snap into place on the reaction vessel carousel. Removal of the reaction vessel loading device from the reaction vessels and the carousel is easily accomplished by pulling upward, utilizing, for example, the elevated handling fins on the reaction vessel loading device which does not dislodge the snapped-into-place reaction vessels in the reaction vessel carousel. The force required to remove the reaction vessel loading device from the reaction vessels which are snapped into place in the reaction vessel carousel is less than the holding force of the snapped-in-place reaction vessels in the reaction vessel carousel. This reduced force requirement is due in part to the reaction vessel insertion depression construction and particularly to the reaction vessel well fitting protrusions and reaction vessel cuvette fitting protrusions which provide a diminished cross-section from the base of the protrusions to the end of the protrusions which not only allows for ease of removal, but also ease of insertion of the reaction vessel loading device into and from the reaction vessels.

The utilization of a reaction vessel loading device strip offers the operator significant time savings in loading multiple reaction vessels at one time as opposed to individually handling each reaction vessel for insertion into the reaction vessel carousel. In general, the reaction vessel carousel will hold ninety reaction vessels or more at one time.

In further assistance to the operator, the reaction vessels can be prepackaged mounted on the removable semi-rigid handling strip which allows efficiency of operator's time for removing the multiple reaction vessels from packaging and for quick loading into the reaction vessel carousel. Once inserted into the reaction vessel carousel respective slots, the reaction vessels are pressed down by the pressure on the strip upper portion until the reaction vessels snap into place on the reaction vessel carousel. Removal of the strip from the reaction vessels and the carousel is simply by pulling upward which does not dislodge the snapped into place reaction vessels in the reaction vessel carousel.

The transfer station 42 plays a key role in apparatus and process function. In FIGURE 11, a sectional side view of the transfer element of the transfer station 42 is shown engaging reaction vessel 34 by means of a reaction vessel transfer projection 172. The transfer arm 173 is projected out between reaction vessel elements of the reaction vessel carousel 36 and, by rotation of the transfer station 42, engages the reaction vessel transfer projection 172. By means of a transfer arm drive gear 174, the transfer arm 173 rack gear 176 moves the transfer arm 173 out and in relationship to the transfer station 42. The transfer station 42 has a rotation axis 178. In FIGURE 11A, a reaction vessel is shown in phantom as would be mounted on the front end carousel 4, reaction vessel carousel 36 engaged by the transfer arm 173 by means of reaction vessel transfer projection 172. The reaction vessel in phantom 180 has a transfer handling means, i.e. transfer projection 172 which allows the transfer arm 173 of the transfer carousel to position an engagement means or pick 184 for engaging the reaction vessel 180 transfer projection 172. The reaction vessel 34 in FIGURE 11 is illustrated onboard the transfer station by reaction transfer station 42 moves the reaction vessel 34 between the front end carousel 4 and the process carousel 46. The transfer station 42 moves the discarded reaction vessel 34 from the process carousel 46 to the waste ejection station (not shown). The transfer station 42 is driven by a stepper motor drive and is supported by precision linear ball bearings and axis of rotation ball bearings.

The process carousel 46 holds, for example, 36 reaction vessels 34 and has a carousel diameter of about 12.5 inches. The process carousel 46 moves the reaction vessel 34 between the transfer station 42, the second transfer pipettor mechanism 50, the point of pipetting, and the FPIA reader processing 52. The process carousel 46 is driven by a stepper motor and supported by three wheels for height control and control of any radial movement caused by irregularly shaped carousel elements.

The second transfer pipette mechanism 50 moves the pipette probe between the wells in the reaction vessel 34 on the process carousel 46 to the MEIA cartridge 68 on the auxiliary carousel 64 and to the wash cup 58. A rack-and-pinion drive through two axis stepper motor drives achieves precision drive on both the R and Z axis. Travel, for example, on the Z axis can be about 3 inches and on the R axis about 4.5 to 5.0 inches.

The auxiliary carousel 64 holds, for example, 32 MEIA cartridges 68 and has a diameter of about 9.5 inches. The auxiliary carouser 64 moves the MEIA cartridges 68 between various stations including the second transfer pipettor mechanism pipette point, the MUP dispense station 72, the MEIA washstation 70 and the MEIA reader 74 and the MEIA cartridge ejection point 62. The auxiliary carousel 64 is stepper motor driven and is carried by three wheels with one wheel located at the Z axis height control at the cartridge insertion point, the second wheel at the pipette point, and the third wheel at the MEIA reader in order to maintain the auxiliary carousel 64 within desired geometric relationships to these various functions.

MEIA cartridges 68 are loaded into a cartridge hopper 66 which feeds the MEIA cartridges 68 into the auxiliary carousel 64. The automatic feeding of the MEIA cartridges 68 is provided with a proper height adjustment of the cartridge 68 into the auxiliary carousel 64 as required by MEIA reading. The cartridge hopper 66 feeds individual cartridges 68 to the auxiliary carousel 64 and changes the axis of orientation of the cartridge 68 from horizontal to vertical by automatic means. Removal of the MEIA cartridges 68 is achieved through the use of an ejector 62 which operates through an ejection rod and forces the MEIA cartridge 68 from the auxiliary carousel 64 which is dropped into a solid waste container.

Buffer supply stations are presented in FIGURE 14 which is a top plan view in section of the apparatus showing the cabinet frame 16, front end carousel 4 in partial phantom and a power supply element 192 along with diluent system or buffer pressurization means 194. A supply bottle 196 is also mounted in the lower cabinet of frame 16 as well as solid waste 198 and liquid waste 200 containers for receiving processed liquids and solid waste.

A controlled environment zone is necessary for incubation and chemical reactions within automated continuous and random access analytical systems. Temperature control is maintained in the controlled environment zone for purposes of controlling the temperature of disposables, chemicals, tubing, mechanisms and the like within the incubation and reaction zone which is optimum to the appropriate chemical reactions. Temperature control is achieved utilizing air flow and air temperature as the thermal dynamic working fluid. Although air or gases do not transfer heat as rapidly as a liquid bath, air does not have the associated problems of leakage, evaporation or contamination.

The controlled environment zone contains carousels carrying chemistries of different reagents and volumes thus requiring an unusual temperature control approach by forcing the heated air to negotiate a pathway with a substantial pressure drop immediately upstream of the process carousel. The pressure drop of the pathway is higher than the pressure drop the air experiences as it passes under the carousel, whether the carousel is fully loaded or not. Thus, the heated air distributes itself evenly about the carousel rather than preferentially funneling itself into a gap which may exit at the empty position of the carousel. Air flow control within the controlled environment provides for minimal air flow above the top surfaces of the carousels. Slow-moving air above liquid surfaces exposed by the open containers on the top portions cause less evaporation than air moving rapidly. However, total air flow is relatively high within the controlled environment zone and air flow along the carousel under sides can be a combination of turbulent and laminar flow. A reasonably high turnover rate and air flow is necessary to minimize temperature variation.

A schematic view illustrating the environmental airflow and temperature control system is shown in FIGURE 15 wherein make up air 204 enters and hot air exits at exhaust 206. Airflow 202 is indicated by arrows and the controlled environmental airflow schematic 214 is provided with at least one heater element 208 and fan element 210. At least one temperature sensor 212 is provided for control of the air temperature and can be correlated with the airflow 202 control.

A schematic view illustrating the environmental airflow and temperature control system is shown in FIGURE 15 wherein make up air 204 enters and hot air exits at exhaust 206. Airflow 202 is indicated by arrows and the controlled environmental airflow schematic 214 is provided with at least one heater element 208 and fan element 210. At least one temperature sensor 212 is provided for control of the air temperature and can be correlated with the airflow 202 control.

Temperature control for reaction and incubation zones of a continuous analytical system is achieved utilizing heated air flow to control the environmental zone 18 which includes of principle importance, the process carousel 46. Both FPIA and MEIA procedures utilize the system apparatus commonly through and including the process carousel 46. A controlled environmental air flow schematic 214 is illustrated in Figure 15A wherein no air is recirculated for temperature controlled purposes. Air flow 202 is motivated by fan element 210 and enters through air inlet 205 including an appropriate air filter system, pass an air heater element 208. The air flow 202 is forced through conduit means embodied in the base plate of the instrument. As the air emerges from the ducting, the air is directed toward the underside of carousels which contain the samples to be analyzed, the necessary reagents and the disposable which are used in the process. Air, after leaving the vicinity of the carousels, is allowed to circulate within the controlled environment zone 18.

As the heated air is applied to the process carousel, its temperature is sampled by a sensor 212. The sensor 212 output is monitored by a controller and when the controller determines that the system requires additional amounts of heat, the controller energizes a solid state relay which applies electrical power to the heating element 208. One or more heating elements 208 are situated in the air flow 202 path for efficient transfer of heat to the air. An air exhaust conduit 206 allows air to flow through multiple openings in the conduit in a controlled manner. While fan element 210 forces the heated air throughout the system, ambient air is introduced through air inlet 207 downstream from the most critical areas of temperature control within the controlled environment zone 18 in order to provide cooling for fluidics of the system through provision of cooling fluid to the heater blocks which are utilized in the fluidics system. This introduction of ambient air through air inlet 207 is near the air exhaust 206 conduit and the various outlets which are in communication with the controlled environmental zone and the air exhaust conduit 206.

The controlled environment zone 18 is kept at a desired temperature by heating air to the correct temperature and applying air in large amounts to the most critical areas of the zone. Heat is transferred by convection to the critical areas by using air which is generally experiencing turbulent flow, thus in this manner, critical areas may be brought to temperature as rapidly as possible. The less critical areas are downstream and are heated under less forceful conditions, i.e. slow moving air flow. In addition to having turbulent flow in the critical areas, the total air flow is relatively high within the controlled environment zone 18 with the air being completely expelled with no recycle of any portion of the air as opposed to partial recycling of the system as illustrated in Figure 15.

Potential problems which could occur in dealing with fully loaded carousels versus partially loaded carousels is solved by forcing the heated air to negotiate a pathway with a large pressure drop immediately upstream of the carousel. The pressure drop of the pathway is higher than the pressure drop the air experiences as it passes under the carousel, whether the carousel is fully loaded or not. Thus, the air distributes itself evenly about the carousel rather than preferentially funneling into a gap which may exist at the empty positions on the carousel.

The MEIA cartridge 68 is shown in a side elevational view in FIGURE 16. The MEIA cartridge 68 has a funnel throat 216 and a cartridge opening 218. The MEIA cartridge 68 contains support matrix material 222.

A MEIA cartridge 68 and cartridge hopper 66 are shown in a side elevational view in FIGURE 17. The MEIA cartridges are positioned horizontally in the cartridge hopper 66 and are manipulated from the bottom of the V-shaped cartridge hopper 66 one-by-one through a cartridge shuttle 222. The cartridge feeder has a cartridge cam block 224 and a cartridge orientation shoot 226 which functions through cartridge orientation pin 228 and cartridge orientation pin 230 for providing the MEIA cartridge 68 in vertical alignment for insertion into the auxiliary carousel 64. The orientation pins 228 and 230 are illustrated in FIGURE 18 which is a side sectional view in isolation of the MEIA cartridge feeder cartridge orientation mechanism. The MEIA cartridge 68 is shown in an enlarged view in FIGURE 18 as being engaged and disengaged by cartridge orientation pin 228 and cartridge orientation pin 230. The cartridge orientation pin 230 is shown in engagement position at position 232 against the base 236 of the MEIA cartridge 68 while cartridge orientation pin 228 is shown in engagement position 234 of the cartridge funnel throat portion 218. Upon withdrawal of these pins from the engaging positions, the MEIA cartridge 88 is released from the bottom portion first, i.e. the withdrawal of cartridge orientation pin 230, thus allowing the bottom of a cartridge 68 to drop by gravity before the top of the cartridge is released which is engaged by cartridge orientation pin 228 in the cartridge funnel throat 216. The rounded or semicircular holding surfaces of the orientation pin allow the release of the bottom of the MEIA cartridge and the rolloff of the funnel throat portion 216 from the cartridge orientation pin 228. The vertically aligned MEIA cartridge 68 is then inserted into the auxiliary carousel 64 to a controlled height by the action of an insertion cam means 227 as shown in FIGURE 17.

The cartridge feeder apparatus for automated, continuous and random access analytical systems provides a mechanism which will feed cartridges used for example in MEIA assays, singly, upright and on demand to a feeder means which inserts the cartridges into a carousel. The hopper loader and feeder orientates each cartridge using cartridge contact means inclusive of axially aligned contact surface having rounded edges and recesses in the contact means between the contact surface and extension arms or rings which are positioned approximal to the outer cartridge walls, the contact means contacting the cartridge from both ends and engaging both ends simultaneously. The cartridge is then dropped to a feeder means. As the cartridge drops, it hangs momentarily on the contact means which protrudes into an indention of the cartridge at the open end while the bottom end of the cartridge falls free, toward the lower carousel feeding means. A combination of packaging having specific opening means for feeding cartridges from cartons into a cartridge hopper feed means coupled with the orientation apparatus provides full service needs of an automated, continuous and random access analytical system.

The side cross-sectional side view in isolation of a MEIA cartridge feeder cartridge orientation mechanism 215 is shown in Figure 18A'. A MEIA cartridge 68 is shown in an engaged mode by orientation contact means 237 and orientation means 239. The cartridge orientation contact means 237 is shown in an engaged position 231 and the orientation means 239 in shown in an engaged position 233. On withdrawal of these orientation contact means from engagement with the MEIA cartridge 68, the cartridge 68 is released from the bottom first by engagement orientation means 239, thus allowing the bottom of the cartridge 68 to drop by gravity before the top of the cartridge is released from engagement position 233 from engagement orientation means 239. The orientation means 239 is positioned in the cartridge funnel throat 216 thus requiring disengagement by the orientation contact 237 before the cartridge rolls or slides away from the orientation means 239 which is comprised of a semi-conical engaging head projecting in axial alignment with the cartridge access head being spaced from arm members or ring members which project approximal to the outside container wall of the cartridge thus creating a semi-form fit between the orientation means 239 and the configuration of the cartridge funnel throat 216. Such approximate mating of the configurations of the orientation means 239 and cartridge funnel throat 216 upon release of the bottom of the cartridge by orientation contact means 237 allows the cartridge 68 to be momentarily delayed from release of orientation means 239 thus creating a bottom first drop of the cartridge 68. The rounded or semi-circular holding surfaces of the orientation means 239 allows the release of the bottom of the MEIA cartridge and a slide-off of the funnel throat portion 216 from the orientation means 239. The vertically aligned MEIA cartridge 68 is then inserted into the auxiliary carousel 64 to a control height by the action of an insertion cam means 227 as shown in Figure 17.

A side view of a MEIA cartridge ejector 62 is illustrated in FIGURE 19. The cartridge ejector 62 functions through an ejector rod 240 and can be driven by manual or automatic drive means 242. The ejected MEIA cartridge is ejected through an ejection passage to the solid waste 198 container.

The side cross sectional view of a cartridge hopper 66 of Figure 29" is shown with a cartridge carton 480 positioned for unloading cartridges 68 into cartridge hopper 66. The cartridge carton 480 resting on unloading roller pins 484. The cartridge carton is also shown in phantom at a maximum open, unloading mode, again resting and being guided by roller pins 484. A slight downward force on the carton against the roller pins 484 will achieve the maximum open position as shown in the phantom 481. The cartridge cartons 480 have various break open or tab opening 482 for ease of opening and unloading in combination with the roller pins 484. The cartridge hopper 66 of Figure 29" has one substantial wall configuration in the vertical and angled upper portion in a lower portion to the hopper cartridge release opening while the opposite wall is configured with a partial upper vertical configuration with a slanted inwardly wall portion down to the horizontal level where the opposing wall goes into an angled direction to the hopper cartridge release opening 486. The lower portion of both walls forms a feed type configuration approximately of the same angulation to the hopper cartridge release opening 486. The cartridge carton 480 can be designed to contain any number of cartridges, however a carton capacity of about 100 is suitable for operation within the environments of the hopper and roller pins 480 locations.

A second embodiment of the cartridge hopper 66 is shown in Figure 30" in a side cross-sectional view in isolation with cartridges loaded into the hopper and a cartridge carton positioned after unload on the roller pins 484. The cartridge carton break open or opening zone being open for unloading. The cartridge hopper has symmetrical wall positioning and angulation to a hopper cartridge release opening 486. A cross-sectional side view as shown in Figure 31" is taken along the line B-B of Figure 30". The side cross-sectional view shows a slightly widen cartridge feed zone at the top of the hopper, roller pins 484, cartridges 68 in place and the hopper cartridge release opening 486.

Still another embodiment of the cartridge hopper is shown in Figure 32" in an isometric view of a stand alone hopper 488 which is detachable from the remainder of the feed means, the stand alone hopper 488 being easily detached for loading purposes. The hopper presents cartridge availability indication 494 through a transparent wall portion for operator inspection. The stand alone hopper has an attached stand alone base or platform 492 for supporting the hopper during loading of multiple cartridges from a carton 480 as shown in Figures 29" and 30", utilizing the roller pins 484.

The roller pins 484 can be considered as breaking roller pins in combination with the cartridge carton 480 which uses a free standing carton break open 482. Other carton opening schemes can be utilized such as a rip tab or partial opening or for full opening while still using roller pins 484 for positioning the opened or partially opened cartridge carton 480 in the hopper. The roller pins 484 obviously operate with minimal friction action on movement of the carton positioned for opening thus allowing the carton to achieve full opening without the necessity of manual force. The cartons contain numerous cartridges in random orientation. Thus the cartons deposit the numerous cartridges into the hopper in the same random orientation. However, such random orientation does not pose a problem in view of the cartridge orientation mechanism 215 having identical orientation means 237 and 239 which can function on either end of the cartridge for orientation purposes.

The present invention provides a data acquisition system which implements ratiometric measurements with improved noise performance to enable simplified communication and operation of an automated analytical instrument, such as the automated continuous and random access analytical system described herein. In particular, the data acquisition system comprises an electronic firm wear utilizing digital signal processing and single chip analog to digital converter to improve noise performance. The system is combined with digital micro-controller means to simplify communication and operation.

FPIA optics analog signals are provided to a DSP A/D chip which sends serial bus signals to an optic signal processor 8-bit digital micro-controller which is in communication with a computer. The digital micro-controller is in communication through serial bus to FPIA optics through high voltage power PMT supply and FPIA tungsten lamp power supply, and in electronic communication with FPIA optics. External MEIA optics analog signals are provided to a second DSP A/D chip which also sends serial bus signal to the optic signal processor 8-bit digital micro-controller, wherein the digital micro-controller presents SIGNAL TO MEIA optics which is in communication through serial bus to a high voltage PMT power supply mercury lamp power supply. The high voltage PMT power supply MEIA mercury lamp power supply is in electronic communication with said MEIA optics.

In addition, the system functions within an FPIA subsystem to acquire and convert to digital format, wherein PM high voltage sample fluorescence intensity is under excitation by vertical and horizontal polarized light and provides liquid crystal control, as well as with an MEIA reader subsystem to acquire and convert to digital format, mercury lamp intensity level and sample fluorescence intensity level, and PMT high voltage.

A box diagram of the optics signal processor of the apparatus is provided in FIGURE 20 wherein the signal from the FPIA optics 248 is fed to a DSP A/D 250 which also sends serial bus signal 252 from an optic signal processor 8-bit microcontroller 254. The controller 254 is connected to computer elements through 256. Signal from the MEIA optics 258 are fed into a DSP A/D element 260 which also sends serial bus 262 from the controller 254. Signal is fed to the FPIA optics through 264 from high voltage power supply 266 and serial bus 268 which is in communication between the microcontroller 254 and the optics power supply board 270A. The FPIA tungsten lamp power supply FPIA 270 is in electronic communication with the FPIA optics 272. Signal is sent to the MEIA optics through 274 from high voltage power supply 276 which is in communication through serial bus 268 to the microcontroller 254 and mercury lamp power supply MEIA 280. The MEIA mercury lamp power supply 280 is also in electronic communication with MEIA optics through 282.

A schematic view of the FPIA optical system 284 is shown in FIGURE 21. The FPIA optical system 284 has a tungsten halogen source lamp 286 which focuses light through a heat reflector 288, an aperture 290 and heat absorber 292 to a lens 293 for introduction into an excitation filter 294. The light energy is then contacted with a beam splitter 296 which presents part of the beam to a polarizer 298 and liquid crystal 300. The light continues into another lens 301 before being focused on the cuvette 140 containing the FPIA reaction mixture. Light is emitted from the cuvette through lens means 303 before entering an emission filter 302. The reflected light from the emission filter 302 passes through a polarizer 304 before going to a focusing lens 306 and being focused for feed into photo multiplier tube 308. The beam splitter 296 splits out part of the light from the original source through lens 310 into a reference detector 312 which, in turn, controls the tungsten halogen source lamp.

A schematic view of the FPIA read sequence 314 is presented in FIGURE 22. The FPIA read sequence 314 has a preread time 316 divided into carousel move time 318 and carousel settle time 320. Sub-read interval 340 is divided into a horizontal sub-read 342, A/D converter settle time 344, and a liquid crystal activation time 346. A vertical sub-read interval is identified by 348 which is inclusive of A/D converter settle time 350. Liquid crystal relaxation time is indicated by 352. The liquid crystal relaxation time 352 is illustrated in a preread time sequence. High voltage settle time 324 is further illustrated by lamp settle time 326 that shows the lamps in a sinner 328 and full burn 330 activation. Activities of the FPIA read sequence 314 provide for activities where scheduling windows 332 as exemplified by read prep 334, read parameter 336 during which the lamps are at full burn, and collection results 338 during the lamp settlement time and liquid crystal relaxation time 352.

FIGURE 24 is a schematic view of the MEIA system optical assembly 364. An MEIA light source is provided by mercury source lamp 364 which passes light through an excitation filter 362 to a filter reflector 360 before being fed through lens 358 into MEIA cartridge 68. Reflected fluorescent light is fed back through the filter 360 to a photomultiplier tube 374 after passing through a wide band-pass emission filter 370 and narrow band-pass emission filter 372. Part of the light energy from the mercury source lamp 364 passes directly through filter 360 to a bandpass filter 368 before influencing the photo diode 366.

An apparatus and method for increasing fluorescent lamp life within automated, continuous and random access analytical systems requiring quick full burn responses is presented. The quick start up full burn response is necessitated due to extended periods of standby-shut off of the light source means. The standby-shut off of the light source means prolongs life and usefulness. Within the multiple analytical systems environment, full burn start up or turn on of the light source means is required within one second or less in order to eliminate delays of various programmed operations of the multi-faceted automation systems. Fluorescent lamp life is increased due to the ability of the equipment to shut off the lamp during periods of nonuse rather than leaving the lamp continuously in an on mode.

Figure 24A is a schematic view of a MEIA optical assembly 364 with the various elements as presented in Figure 24. In addition, Figure 24A presents a heater block 363 for maintaining the mercury source lamp 364 at a constant minimum temperature of about 70°C, especially during periods of lamp shut off. By maintaining the mercury source lamp 364 at an elevated temperature, the mercury within the lamp is maintained in a vapor state thereby providing start up or warm up periods of less than about one second. The mercury source lamp 364 is activated automatically upon need by the automated continuous and random access analytical systems. The mercury source lamp 364 being activated and deactivated electronically by systems programming and computer capacities. The mercury source lamp 364 life cycle is increased due to the ability of the apparatus as a whole to shut off the mercury source lamp 364 during periods of nonuse since mercury source lamp 364 can be reactivated to full burn within less than one second due to maintaining the mercury source lamp 364 at a temperature sufficient to maintain the mercury in a vapor state.

Apparatus and method for improving tungsten filament lamp life in continuous and random access analytical systems requiring multiple standby periods is achieved by configuration of apparatus components and electronic circuits which improves lamp life using computer control of lamp current or brightness in an optical measurement device. The tungsten lamp must provide as long a life as possible while still being capable of achieving full brightness on short notice. Using simmer burn cycles of the tungsten filament lamp provides improved life of the lamp as compared to constant full burn methods and also provides a short warm up time or short time to full burn of about one second or less which is appropriate for FPIA procedures within the automated, continuous and random access analytical systems as well as other clinical or special chemistry analyzers requiring multiple full burn and standby modes.

The FPIA optical system 284 of Figure 21 and the FPIA read sequence 314 of Figure 22 clearly emphasize the requirement of a long life, reliable full burn tungsten halogen source lamp 286 within continuous and random access analytical systems having at least two analytical systems incorporated within the same instrument. The FPIA assay system being only one of two or more systems by definition will be in a standby mode for various periods and yet due to the requirements of high through put of modern instrumentation, the FPIA optical system 284 must have a tungsten halogen source lamp 286 which is capable of achieving full burn within about a second or less. Threshold of the FPIA read can be based on the full burn tungsten halogen lamp source 286 or on a predesigned lapse time. If the tungsten halogen lamp source 286 is left at full burn, present sources of such tungsten halogen source lamps have a life expectancy of only fifty hours. Not only is the life expectancy short but as the tungsten halogen source lamp ages, there is a tendency for the source lamp to fall in intensity or brightness. Such reduction or fall in intensity is of significance in a FPIA reader system because the tungsten halogen source lamp 286 must bum bright or hot at an upper limit in order to generate the appropriate wavelengths required by the FPIA optical system. Using simmer/burn cycles for the tungsten halogen source lamp 286 maintains the lamp at a warm or elevated temperature during standby periods, thus allowing the lamp to be driven to full burn within a short warm up, one second or less.

The tungsten halogen source lamp 286 is generally operated within the continuous and random access analytical systems for substantial periods of time at a low intensity or simmer condition. This simmer low intensity is selected to keep the lamp warm without being too detrimental to the lamp's longevity. A FPIA read request will be issued by the scheduler of the analytical system a few seconds preceding the read commencement. Since the lamp is thermally near its operating point, there is adequate time to change the intensity to full burn in preparation for the read. After the read is completed, the lamp is returned to simmer until a new read request is issued; thus improving the tungsten halogen source lamp element life as opposed to constant full bum even during standby.

This cycling between full burn and simmer burn standby mode methodology differs significantly from previous systems. A main departure is that the lamp intensity may be compromised slightly during the read interval because of the cycling of the full burn and simmer burn modes. A higher dependency on a compensation method is recognized which requires both a linear reference detector over a range of operation and a ratiometric data acquisition subsystem. In order to provide good compensation by the reference detector/data acquisition system combination, the settling time defaults to the amount of radiant power needed to achieve good noise performance. The FPIA read will be allowed to commence as soon as the minimum radiant power is confirmed. This minimal power, though somewhat tenuous, should be at least about 90% or greater based on the results of the FPIA optics noise protocol. In scenarios where the compensation technique proves to be inadequate, and an unstable light brightness cannot be tolerated, the read will not be allowed to proceed until the lamp is completely stable, long after full burn is achieved.

An MEIA read sequence schematic is presented in FIGURE 25 wherein the MEIA read sequence 376 has a preread time 378 inclusive of carousel move time 380 and carousel settle time 382. High voltage settle time is indicated by graph 384 which is coincident with the lamp settlement time 386 showing lamp simmer 388 and lamp full burn 390. MEIA read sequence 376 has activities with scheduling windows 392 inclusive of read prep 394, read parameter 396 and collection results 398. The actual MEIA read sequence 376 is inclusive of sub-read interval 400 having a sub-read 402 and a dwell time 404. Another segment of the MEIA read sequence 376 is indicated by sub-read interval 406 inclusive of sub-read number to 408 and dwell time 410 with additional sub-reads 412 as indicated by number 3 through (N-1) and partial sub-read interval 414 inclusive of sub-read number N-416. The next possible preread time is indicated by 418.

The apparatus and method for controlling evaporation of reagents used in diagnostic testing and assays are provided by an apparatus that opens multiple containers from an evaporatively sealed condition to fully open for system's access. Conversely, the device closes multiple containers to an evaporatively seal condition. The apparatus and method also control the opening acceleration of the container's closure systems to reduce the contamination potential of randomly sprayed droplets of contaminated liquids into the containers. The reagent or liquid containing containers are open and resealed by the apparatus to minimize the time the container stands in an open condition, thus minimizing evaporation while maximizing accessibility.

The apparatus and method for controlling evaporation of reagents used in an automatic, continuous and random access analytical system provides for opening multiple containers from an evaporatively sealed condition to fully open for system access. The apparatus also closes the multiple containers to an evaporatively sealed condition through opening and closing means. The opening acceleration of the container's closure system, for example flip top caps, are controlled in order to reduce the contamination potential of randomly sprayed droplets of liquid in the containers.

Presently reagent containers have septum designs to help control evaporation, after the shipping seal is left open. This system has a major disadvantage of contributing to cross-contamination. In addition, manual operating of the container closures, use of septum caps, is unsatisfactory in both contamination and evaporation rates of costly reagents. Within automatic, continuous random access analytical systems, a computer-controlled robotic opening and closing station is required which replaces the need for manual intervention, and which will minimize evaporation. The apparatus inclusive of the opening and closing station as well as the cover and cap means on the reagent vessel openings is less likely to spread contamination between probe and containers like septum systems have been found to do while minimizing evaporation. The use of systems with similar closures which stay open of their own design with no automatic reclosure features incorporated in the cap makes such a remaining open apparatus attractive. However, operational certainty requirements of the automated continuous and random access analytical systems can also afford to utilize systems which open the caps and hold the caps open and forceably reclose the caps to one or two levels of closure, a soft closure for routine day-to-day usage of opening and closing or a hard closure during periods of stand-down or for example, hard closure of the caps for handling purposes wherein the containers contain reagents.

The apparatus according to the invention opens liquid containers and reseals the containers to minimize the time the container stands in an open condition, thus minimizing evaporation while maximizing accessibility. The apparatus minimizes the potential for cross-contamination due to flying droplets typical of the present manual methods when opening the sealed containers. The system methodology accomodates variations in container evaporation differences and will open and close containers properly while maintaining an evaporative seal. The apparatus performs in one motion, the same action as two fingers of a human hand, giving the device dexterity to open one or more sealed containers and reclose same.

Diagnostic systems utilizing reagents to analyze fluids depend heavily on the correct concentration of these fluids or reagents. It is therefore important to minimize evaporation of these fluids from their storage containers. Prior to removing of fluid, the container is moved below the opening and closing station and then the station opens the container's closure. The fluids are quickly withdrawn and the opening and closing station reseals the containers until the next time the fluids are needed in the process.

In the top view of Figure 29', a reagent pack 30 containing reagent containers 450 present a view of reagent containers 450 having reagent container openings 452 closed by cover and cap means 454. The reagent containers 450 are maintained within reagent pack walls 456 as well as an open bulk liquid container 460. The reagent pack walls 456 give the reagent pack a configuration which is suitable for insertion into the reagent carousel of the front carousel. The reagent containers 450 and the open bulk liquid container 460 are maintained within the reagent pack 30 by reagent pack container mounting and stabilization surfaces 458. The side sectional view of Figure 30' is a section taken along section A-A of Figure 29' and shows multiple positions of the cover and cap means 454 inclusive of open and lock back, opened but not locked back and closed capping the reagent container opening 452. The isometric view of Figure 31' presents a reagent container 450, cover means 31, contact surface 33 and reagent container opening 452. The cover and cap means 454 are shown in an open position exposing a cap member 462 which fits inside the reagent container opening 452 and together with a spaced-apart ring member 464 provides an evaporatively sealed reagent container 450 when the cover and cap means 454 are in a closed position.

An opening and closing station is shown in a perspective side elevational view in Figure 32' and a different perspective side elevational view in Figure 33. The opening and closing station 464 has a housing 466 and a drive motor 468 mounted thereon. The opening and closing station 464 housing 466 has a mounting means 470 for mounting and fixing the opening and closing station 464 to a position above a reagent carousel which moves the reagent containers 450 to the opening and closing station 464 for either opening the cover and cap means 454 or closing the cover and cap means 454.

In Figure 32' the cover and cap means 454 has been open by opening pins 472 which make contact with the cover and cap means 454 pivoting said means about pivot 476 to open the cover and cap means 454, to a vertical position. The reagent pack closure activator 474 comprising of three valve-shaped heads are in an inactive position while the opening pins 472 are pushed down and against a portion of the cover and cap means 454 for opening the reagent containers 450. The carousel moves the containers 450 in Figure 32' away from the opening pin 472 and under reagent pack closure activator numbers 474 which in one embodiment drag against the open cover and cap means 454 to push said means past the vertical away from the closure position wherein the cover and cap means are locked by internal spring means.

The reagent containers 450 shown in a closed position in Figure 33 are also positioned under the reagent pack closure actuator members 474. The opened reagent containers for closure purposes are moved again into contact with either partially lowered opening pins 472 or partially lowered reagent pack closure actuators 474 which drag against the opened and spring locked cover and cap means 454, overcoming the spring load and returning the cover and cap means 454 partially closed which is then pressed to a soft closed position on the reagent containers 450. Optionally the reagent pack closure actuator members (valve members) can then be brought into a more firm contact with the soft closed cover and cap means to force the cover and cap means into a hard closed position if desired.

Optionally the cover and cap means 454 can be individual for each reagent container 450 as shown in Figure 31' or can be a gang-type of cover and cap means as shown in Figures 32' and 33. The opening and closing station 464 having three opening pins 472 and three reagent pack closure actuator valve-type members 474 which can function independently and operate for opening either individual reagent containers or, more likely, operate in unison to open all of the reagent containers 450, cover and cap means 454 whether the cover and cap means are individual for each reagent container 450 or joined together in presenting an expanded cover and cap means covering multiple reagent containers 450.

Multiple automated assay analytical systems are feasible through use of the apparatus, software, hardware and process technology of the present invention and include, but are not intended to be limited to, the following menus: ferritin, creatinine kinase MIB (CK-MB), digoxin, phenytoin, phenobarbitol, carbamazepine, vancomycin, valproic acid, quinidine, leutinizing hormone (LH), follicle stimulating hormone (FSH), estradiol, progesterone, IgE, vitamin B2 micro- globulin, glycated hemoglobin (Gly. Hb), cortisol, digitoxin, N-acetylprocainamide (NAPA), procainamide, rubella-lgG, rubella-IgM, toxoplasmosis IgG (Toxo-IgG), toxoplasmosis IgM (Toxo-IgM), testosterone, salicylates, acetaminophen, hepatitis B surface antigen (HBsAg), anti-hepatitis B core antigen IgG IgM (Anti-HBC), human immune deficiency virus 1 and 2 (HtV 1 and 2), human T-cell leukemia virus 1 and 2 (HTLV), hepatitis B envelope antigen (HBeAg), anti-hepatitis B envelope antigen (Anti-HBe), thyroid stimulating hormone (TSH), thyroxine (T4), total triiodothyronine (Total T3), free triiodothyronine (Free T3), carcinoembryoic antigen (CEA), and alpha feta protein (AFP).

In order to insure consistent, rapid resuspension and continued mixing of reagents with minimal operator involvement. the reagents are mixed automatically each time a new reagent pack is added to the reagent carousel, and periodically during instrument operation. This automated mixing can be accomplished by a back and forth motion of the reagent carousel with asymmetric pauses and is complete within approximately 1-2 minutes. The carousel acceleration, velocity, distance moved, and pause-asymmetry are optimized to yield the most rapid reagent resuspension without foaming or bubble formation for the range of fill volumes used on the instrument.

Automated reagent mixing provides the following benefits. The operator need not manually mix (e.g. by inversion or shaking) reagents which have been stored prior to their placement on the instrument. This allows the reagents to be loaded onto the instrument in less time and with less involvement of the operator. There is less tendency for reagents to foam or form bubbles with automatic mixing than with manual mixing such as inversion. Foam and bubble formations are detrimental to instrument function and can negatively impact assay performance. Automated mixing insures that reagents are always mixed sufficiently and that they are mixed consistently. Occasional automatic mixing during instrument operation keeps reagents in a consistent suspension, and makes it unnecessary for the operator to periodically remove reagent packs in order to mix the reagents. In some circumstances, automated mixing can dissipate bubbles present at the start of mixing. A detailed description of kitting and process activities according to the invention are presented in the following for FPIA procedures; system description of process activities for a phenobarbital assay; and MEIA procedures for a CEA assay.

It is to be appreciated that the following description comprises an outline of the various functions and steps involved in preferred methods of the automated analytical system of the invention, which functions and methods as also will be appreciated by those skilled in the art, are conducted under computer control using various types of mathematical algorithms and associated computer software, depending on the particular menu of assays being performed on the instrument.

### DESCRIPTION OF KITTING AND PROCESS AREA ACTlVlTIES FOR FPIA

### SYSTEM DESCRIPTION OF KITTING AREA FOR PHENOBARBITAL ASSAY

### A. ASSUMPTIONS

1. Analyzer is in Standby/Ready mode when sample is loaded. System has been previously initialized (All motors are homed, syringe and pumps are purged, all electronics and sensors are checked.)
2. Waste has been emptied, Diluent, MEIA buffer, MUP, and Quat bulk liquid consumables have been checked for sufficient volume.
3. All Consumable inventory files have been updated.

### B. PREPARATION STEPS

1. User loads empty Reaction Vessel (RV) into RV carousel.
2. To load a reagent pack(s), the user must first pause the front end carousels. The system will complete kitting of the current test and transfer the test to the process area.
3. User opens the reagent carousel cover, loads reagent pack(s) into reagent carousel, closes the reagent carousel cover, then resumes the front-end.
4. instrument automatically scans all reagent packs onboard to verify reagent status.
   (a) Each reagent pack is positioned in front of the reagent pack barcode reader by rotation of the reagent carousel.
   (b) Reagent pack barcode reader reads barcode to identify assay type and carousel location.
   (c) If the barcode is unreadable, the system will request a barcode override.
   (d) If the barcode is good or override complete, the system will check the system inventory. The user will be notified if the pack is found to be empty, invalid or outdated. Once the reagent pack is found to be good, it is ready to use.

### C. REQUESTING A TEST

1. User has two options for requesting a test or group of tests on one or more patient samples.
   (a) User may download the test request loadlist from a host computer to create an order list.
   (b) User enters test request or creates an order list on the System directly.
2. If sample cups (no barcode) are used, the following scenario occurs:
   (a) User refers to order list for segment ID and position number to place sample.
   (b) User loads a sample cup into referenced position in segment.
   (c) User transfers patient sample from blood collection tube into sample cup.
   (d) Segment is placed into sample carousel.
   (e) Indication is made to instrument that samples have been loaded.
   (f) Instrument checks consumable inventories, waste status, cal status, etc.
   (g) Sample carousel rotates segment to segment identification reader.
   (h) Instrument reads segment identification.
3. If primary tubes (with barcode) are used, the following scenario occurs (two types of carriers are used for primary tubes: one for tubes with heights of 75 mm and a second for tubes with heights of 100 mm.):
   (a) User loads primary tube into next available segment location on sample carousel. '
   (b) Indication is made to instrument that samples are available to be run.
   (c) Instrument checks consumable inventories, waste status, cal status, etc.

### D. SCHEDULING A TEST

1. When the sample is presented to the pipettor, the System attempts to schedule the tests ordered on that sample for processing. Each test ordered for the sample will be scheduled separately.
   (b) The System checks for adequate inventory (reagent packs, cartridges, buffer, MUP), system resources, sample time to complete the test.
   (c) The System checks for valid calibration or orders for them on the order list.
   (d) If all test requirements are met, the test is scheduled for processing.
   (e) If all test requirements are not met, the test request is moved to the exception list. Once the test requirements have been met, the test request is moved back to the order list by the user.
2. When a test has been scheduled, the System moves it to the processing list and attempts to schedule other tests ordered for that sample.
3. When all tests for the current sample have been kitted, the System advances to the next sample on the sample carousel.

### E. KITTING A TEST

1. Once a test is scheduled, it is immediately kitted. (No tests are kitted until the scheduler ensures that the test can be transferred onto the process carousel immediately and processed within the timing requirements of the assay.)
2. RV carousel is rotated clockwise until an RV is detected in pipette axis position.
3. Reagent pack carousel is rotated until reagent pack for test ordered is at the actuator position. The actuator opens the reagent cartridge caps and the reagent pack carousel is then rotated until a reagent pack for test ordered is in the pipette axis position. After all pipetting steps have been completed, the reagent pack carousel is rotated back to the actuator position where the reagent cartridge caps are closed.
4. Sample carousel is rotated until sample cup (or primary tube) is in pipette axis position.
5. Pipette is always at "HOME" position (Pipette R-axis is parked over wash station and Pipette Z-axis is at the Z-clear position) when not in use.
6. Sample kitting.
   (a) Sample aspirate.
      (i) Syringe aspirates "X" uL of air at a rate of "X" ul/sec.
      (ii) Pipette R-axis is moved over sample cup.
      (iii) Pipette Z-axis is moved down to the Z-above position.
      (iv) LLS is enabled to ensure that no liquid is currently detected.
      (v) Pipette Z-axis is moved down at constant speed until fluid is detected or until Z-Asp limit has been reached (It will be assumed that fluid is detected)
      (vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (If insufficient volume is present, the test is aborted and the test request moved to the exception list. The exception list provides notice to an operator of tests which cannot be completed).
      (vii) The following occur simultaneously until the total volume of sample required is aspirated:
         (1) Pipette Z-axis motor is moved down at a rate of "X" steps/sec.
         (2) Syringe motor aspirates "X" uL at a rate of "X" ul/sec.
         (3) LLS is checked to ensure probe still in liquid Liquid Level Sense (LLS) is disabled. Pipette Z-axis is moved up to Z-clear position.
         (4) Pipette R-axis is moved over the RV sample well.
         (5) Pipette Z-axis is moved down to the dispense position within the RV sample well.
         (6) Syringe dispenses "X" uL of sample at a rate of "X" ul/sec.
         (7) Pipette Z-axis is moved up to Z-clear position.
   (b) Probe Post-Wash
      The probe is washed to ensure that it is free from contamination. It is to be understood that all pipette activities (in both kitting and process areas) are followed with a probe post-wash to minimize carryover from one fluid aspirate to another. In some cases, pipette activities may be preceded with a probe prewash if necessary to guarantee the validity of the next fluid aspirate. For this assay description, it will be assumed that only a post-wash is used.
      (i) The inside of the probe is cleaned first.
         (1) Pipette R-axis is moved over waste area.
         (2) Pipette Z-axis is moved down to appropriate position within the waste area.
         (3) The wash valve is opened for the amount of time specified in the assay protocol.
         (4) Wash valve is closed.
         (5) Pipette Z-axis is moved up to the Z-clear position.
      (ii) The outside of the probe is cleaned next.
         (1) Pipette R-axis is moved over wash cup.
         (2) Pipette Z-axis is moved down to wash position within the wash cup.
         (3) The wash valve is opened for the amount of time specified in the assay protocol.
         (4) Wash valve is closed.
      (iii) Pipette is returned to "HOME" position.
7. Popper kitting ("Popper" is defined as a substance which eliminates in general interfering substances in assays such as, for example, those discussed and claimed in U.S. Patent 4,492,762 issued January 8, 1985 and hereby incorporated by reference.)
   (a) Popper aspirate.
      (i) Syringe aspirates "X" uL of air at a rate of "X" ul/sec.
      (ii) Pipette R-Axis is moved over the popper reagent bottle in the Reagent Pack.
      (iii) Pipette Z-axis is moved down to the Z-above position.
      (iv) LLS is enabled to ensure no liquid currently detected.
      (v) Pipette Z-axis is moved down at constant speed until fluid is detected or until the Z-aspiration-lower (Z-Asp) limit is reached (it will be assumed that fluid is detected).
      (vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
      (vii) The following occur simultaneously until the total volume of popper required is aspirated:
         (1) Pipette Z-axis motor is moved down at a rate of "X" steps/sec.
         (2) Syringe aspirates "X" uL at a rate of "X" ul/sec.
         (3) LLS is checked to ensure probe still in liquid.
         (4) LLS is disabled.
         (5) Pipette Z-axis is moved up to Z-clear position.
         (6) Pipette R-axis is moved over the RV reagent 1 well.
         (7) Pipette Z-axis is moved down to the dispense position within the RV reagent 1 well.
         (8) Syringe dispenses "X" uL of popper at a rate of "X" ul/sec.
         (9) , Pipette Z-axis is moved up to Z-clear position.
   (b) Probe post-wash.
      The probe is again washed to ensure that it is free from contamination as described in section 6 (Sample Kitting).
8. Antiserum kitting
   (a) Antiserum aspirate
      (i) Syringe aspirates "X" uL of air at a rate of "X" ul/sec.
      (ii) Pipette R-Axis is moved over the antiserum reagent bottle in the Reagent Pack.
      (iii) Pipette Z-axis is moved down to the Z-above position.
      (iv) LLS is enabled to ensure no liquid currently detected.
      (v) Pipette Z-axis is moved down at constant speed until fluid is detected or until the Z-Asp limit is reached (it will be assumed that fluid is detected).
      (vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
      (vii) The following occur simultaneously until the total volume of antiserum required is aspirated:
         (1) Pipette Z-axis motor is moved down at a rate of "X" steps/sec.
         (2) Syringe aspirates "X" micro liter (uL) at a rate of "X" ul/sec. LLS is checked to ensure probe still in liquid.
         (3) LLS is disabled.
         (4) Pipette Z-axis is moved up to Z-clear position.
         (5) Pipette R-axis is moved over the RV reagent 2 well.
         (6) Pipette Z-axis is moved down to the dispense position within the RV reagent 2 well.
         (7) Syringe dispenses "X" uL of antiserum at a rate of "X" ul/sec.
         (8) Pipette Z-axis is moved up to Z-clear position.
   (b) Probe post-wash.
      The probe is again washed to ensure that it is free from contamination as described in section 6 (Sample Kitting).
9. Tracer kitting.
   (a) Tracer aspirate.
      (i) Syringe aspirates "X" uL of air at a rate of "X" ul/sec.
      (ii) Pipette R-Axis is moved over the tracer reagent bottle in the Reagent Pack.
      (iii) Pipette Z-axis is moved down to the Z-above position.
      (iv) LLS is enabled to ensure no liquid currently detected.
      (v) Pipette Z-axis is moved down at constant speed until fluid is detected or until the Z-Asp limit is reached (it will be assumed that fluid is detected).
      (vi) Based on the Z-height position at which fluid is detected and the Z-height/volume table, the System calculates the volume of fluid in the well and compares it to the volume specified in the pipetting description. If sufficient volume is present in the well, the aspiration sequence is initiated.(if sufficient volume not is present, the test is aborted and the test request moved to the exception list).
      (vii) The following occur simultaneously until the total volume of tracer required is aspirated:
         (1) Pipette Z-axis motor is moved down at a rate of "X" steps/sec.
         (2) Syringe aspirates "X" uL at a rate of "X" ul/sec.
         (3) LLS is checked to ensure probe still in liquid.
         (4) LLS is disabled.
         (5) Pipette Z-axis is moved up to Z-clear position.
         (6) Pipette R-axis is moved over the RV reagent 3 well.
         (7) Pipette Z-axis is moved down to the dispense position within the RV reagent 2 well.
         (8) Syringe dispenses "X" uL of tracer at a rate of "X" ul/sec.
         (9) Pipette Z-axis is moved up to Z-clear position.
   (b) Probe post-wash.
      The probe is again washed to ensure that it is free from contamination as described in section 6 (Sample Kitting).

### F. TRANSFER OF REACTION VESSEL (RV) INTO PROCESS AREA

1. RV carousel is rotated to transfer station.
2. Process carousel is rotated so that the empty position is aligned with the transfer station.
3. Transfer mechanism O-axis is rotated to sample entry area.
4. Transfer mechanism R-axis grabs the RV and pulls it into the transfer mechanism.
5. Transfer mechanism 0-axis is rotated so that RV is aligned with the empty position on the process carousel.
6. RV is loaded onto process carousel.

### SYSTEM DESCRIPTION OF FPIA PROCESS AREA FOR PHENOBARBITAL

### A. Wait for temperature equilibration time and evaporation window to expire.

### B. FIRST PIPETTE ACTIVITY (preparation of sample blank comprising diluted sample and popper).

1. incubation timer is set according to assay file specifications.
2. Precision diluent aspirate. The following activities are performed simultaneously:
   (a) Syringe aspirates "X" uL at a rate of "X" ul/sec.
   (b) Wash valve is opened.
   (c) Wait "n" seconds.
   (d) Wash valve is closed.
3. Sample aspirate.
   (a) Pipette R-axis is moved over the RV sample well.
   (b) LLS is enabled to ensure no liquid currently detected.
   (c) Pipette Z-axis is moved down at constant speed until fluid is detected OR until the Z-Asp limit is reached (it will be assumed that fluid is detected).
   (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
   (e) The following occur simultaneously until the total volume of sample required is aspirated:
      (i) Pipettor Z-axis motor is moved down at a rate of "X" steps/sec.
      (ii) Syringe aspirates "x" uL of sample at a rate of "X" ul/sec.
      (iii) LLS is checked to ensure probe still in liquid.
      (iv) LLS is disabled.
      (v) Pipette Z-axis is moved up to Z-above position.
4. Diluent/sample dispensed to the RV predilute well.
   (a) Pipette R-axis is moved over the RV predilute well.
   (b) Pipette Z-axis is moved down to the dispense position within the RV predilute well.
   (c) Syringe dispenses "X" uL of diluent/sample at a rate of "X" ul/sec.
   (d) Pipette Z-axis is moved up to Z-clear position.
5. Probe post-wash.
   The probe is again washed to ensure that it is free from contamination as described in section 6 (Sample kitting).
6. Precision diluent aspirate. The following activities are performed simultaneously:
   (a) Syringe aspirates "X" uL at a rate of "X" ul/sec.
   (b) Wash valve is opened.
   (c) Wait "n" seconds.
   (d) Wash valve is closed.
7. Popper aspirate.
   (a) Pipette R-axis is moved over the RV Reagent (popper) well.
   (b) LLS is enabled to ensure no liquid currently detected.
   (c) Pipette Z-axis is moved down at constant speed until fluid is detected or until the Z-Asp limit is reached (it will be assumed that fluid is detected).
   (d) Based on the Z-height position at which fluid is detected and the Z-height/volume table; the System calculates the volume of fluid in the well and compares it to the volume specified in the pipetting description. If sufficient volume is present, the aspiration sequence is initiated (if sufficient volume is not present, the test is aborted and the test request moved to the exception list).
   (e) The following occur simultaneously until the total volume of popper required is aspirated:
      (i) Pipette Z-axis motor is moved down at a rate of "X" steps/sec.
      (ii) Syringe aspirates "X" uL at a rate of "x" ul/sec.
      (iii)LLS is checked to ensure probe still in liquid.
      (iv) LLS is disabled.
      (v) Pipette Z-axis is moved up to the Z-above position.
8. Diluted sample aspirate.
   (a) Pipette R-axis is moved over the RV predilute well.
   (b) LLS is enabled to ensure no liquid currently detected.
   (c) Pipette Z-axis is moved down at constant speed until fluid is detected or until the Z-Asp limit is reached (it will be assumed that fluid is detected).
   (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
   (e) The following occur simultaneously until the total volume of diluted sample required is aspirated:
      (i) Pipette Z-axis motor is moved down at a rate of "X" steps/sec.
      (ii) Syringe aspirates "X" uL at a rate of "x" ul/sec.
      (iii) LLS is checked to ensure probe still in liquid.
      (iv) LLS is disabled.
      (v) Pipette Z-axis is moved up to the Z-above position.
11. Diluted sample/popper diluent dispensed to RV cuvette.
   (a) Pipette R-axis is moved over to the RV cuvette position.
   (b) Pipette Z-axis is moved down to the dispense position in the RV cuvette.
   (c) Syringe dispenses "X" uL of diluted sample/popper/diluent at a rate of "X" uUsec.
   (d) Pipette Z-axis is moved up to the Z-above position.
12. Probe post-wash.
   The probe is again washed to ensure that it is free from contamination as described in section 6 (sample kitting) to complete first pipette activity.

### C. BLANK READ PREPARATION

When incubation timer expired, the following activities are started:
1. The FPIA reader is prepared to take a read; lamp intensity is brought from simmer state to burn state.
2. Photomultiplier tube (PMT) gain is set.

### D. BLANK READ (BACKGROUND)

1. Incubation timer is set according to assay file specifications.
2. Process carousel is rotated so that the RV is at the read station.
3. Horizontal intensity is read for "X.XX" seconds.
4. The crystal is flipped for the vertical read.
5. Wait "n" seconds until the crystal settles.
6. Vertical intensity is read for "X.XX" seconds.
7. The raw reads are converted to normalized reads (light intensity hitting detector/lamp intensity) by the optics microprocessor.
8. Background reads are stored.
9. System calculates BLANK I to complete blank read.
10. Next activity started when incubation timer expires.

### E. SECOND PIPETTE ACTIVITY (for reaction between diluted sample, popper, tracer and antiserum).

1. Incubation timer is set according to assay file specifications.
2. Precision diluent aspirate.
   (a) The following activities are performed simultaneously:
      (i) Syringe aspirates "X" uL at a rate of "X" ul/sec.
      (ii) Wash valve is opened.
      (iii) Wait "n" seconds.
      (iv) Wash valve is closed.
3. Antiserum aspirate.
   (i) Pipette R-axis is moved over the RV Reagent 2 (antiserum) well.
   (ii) LS is enabled to ensure no liquid currently detected.
   (iii) Pipette Z-axis is moved down at constant speed until fluid is detected OR until the Z-Asp limit is reached (it will be assumed that fluid is detected).
   (iv) Based on the Z-height position at which fluid is detected and the Z-height/volume table, the System calculates the volume of fluid in the well and compares it to the volume specified in the pipetting description. If sufficient volume is present, the aspiration sequence is initiated. (If sufficient volume is not present, the test is aborted and the test request moved to the exception list.)
   (v) The following occur simultaneously until the total volume of antiserum required is aspirated:
      (1) Pipette Z-axis motor is moved down at a rate of "X" steps/sec.
      (2) Syringe aspirates "X" uL at a rate of "X" ul/sec.
      (3) LLS is checked to ensure probe still in liquid.
      (4) LLS is disabled.
      (5) Pipette Z-axis is moved up to the Z-above position.
4. Tracer aspirate.
   (a) Syringe aspirates "X" uL of air at a rate of "X" ul/sec.
   (b) Pipette R-axis is moved over the RV Reagent 3 (tracer) well.
   (c) LLS is enabled to ensure no liquid currently detected.
   (d) Pipette Z-axis is moved down at constant speed until fluid is detected OR until the Z-Asp limit is reached (it will be assumed that fluid is detected).
   (e) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
   (f) The following occur simultaneously until the total volume of tracer required is aspirated:
      (i) Pipette Z-axis motor is moved down at a rate of "X" steps/sec.
      (ii) Syringe aspirates "X" uL at a rate of "X" ul/sec.
      (iii) LLS is checked to ensure probe still in liquid.
      (v) LLS is disabled.
      (vi) Pipette Z-axis is moved up to the Z-above position.
5. Diluted sample aspirate.
   (a) Pipette R-axis is moved over the RV predilute well.
   (b) LLS is enabled to ensure no liquid currently detected.
   (c) Pipette Z-axis is moved down at constant speed until fluid is detected OR until the Z-Asp limit is reached (it will be assumed that fluid is detected).
   (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list.)
   (e) The following occur simultaneously until the total volume of diluted sample required is aspirated:
      (1) Pipette Z-axis motor is moved down at a rate of "X" steps/sec.
      (2) Syringe aspirates "X" uL at a rate of "X" ul/sec.
      (3) LLS is checked to ensure probe still in liquid.
      (4) LLS is disabled.
      (5) Pipette Z-axis is moved up to the Z-above position.
6. Diluted sample/tracer/aspirate/antiserum/diluent dispensed to RV cuvette.
   (a) Pipette R-axis is moved over to the RV cuvette position.
   (b) Pipette Z-axis is moved down to the dispense position in the RV cuvette.
   (c) Syringe dispenses "X" uL of diluted sample/tracer/air/antiserum/diluent at a rate of "X" ul/sec.
   (d) Pipette Z-axis is moved up to the Z-above position.
7. Probe post-wash.
   The probe is again washed to ensure that it is free from contamination as described in section 6 (Sample kitting) to complete the second pipette activity.
8. Next activity started when incubation timer expires.

### E. FINAL READ PREPARATION

1. The FPIA reader is prepared to take a read; lamp intensity is brought from simmer state to burn state.
2. PMT gain is set.

### F. FINAL READ

1. Process carousel is rotated so that the RV is at the read station.
2. Horizontal intensity is read for "X.XX" seconds.
3. The crystal is flipped for the vertical read.
4. The System delays "n" seconds until the crystal settles.
5. Vertical intensity is read for "X.XX" seconds.
6. The raw reads are converted to normalized reads (light intensity hitting detector/lamp intensity) by the optics microprocessor.
7. Reads are stored.
8. System calculates NET intensity (I) and milipolarization (mP).
9. mP value is fitted to calibration curve to yield a concentration result.

### G. RV UNLOAD (this activity occurs when resources are not in use.

The following are performed simultaneously:
1. Process carousel is rotated so that the empty position is at the transfer station. Transfer mechanism O-axis is moved to process carousel.
2. RV is grabbed with the transfer mechanism R-axis and pulled into the transfer mechanism.
3. Transfer mechanism 0-axis is rotated so that RV is aligned with the waste container.
4. RV is pushed into the waste container.

### DESCRIPTION OF KITTING AND PROCESS AREA ACTIVITIES FOR MEIA

### SYSTEM DESCRIPTION OF KITTING AREA FOR CEA ASSAY

### A. ASSUMPTIONS

1. Analyzer is in Standby/Ready mode when sample is loaded. System has been previously initialized (All motors are homed, syringe and pumps are purged, all electronics and sensors are checked).
2. Waste has been emptied, dilution, MEIA buffer, MUP, and Quat bulk liquid consumables have been checked for sufficient volume.
3. Cartridges have been placed into hopper and are available for loading onto auxiliary carousel when needed (for MEIA assays only).
4. All Consumable inventory files have been updated.

### B. PREPARATION STEPS

1. User loads empty RVs into RV carousel.
2 To load a reagent pack(s), the user must first pause the front end carousels. The system will complete kitting of the current test and transfer the test to the process area.
3. User opens the reagent carousel, loads reagent pack(s) into reagent carousel, closes the reagent carousel cover, then resumes the front-end.
4. Instrument automatically scans all reagent packs onboard to verify reagent status.
5. Each reagent pack is positioned in front of the reagent pack barcode reader by rotation of the reagent carousel.
6. Reagent pack barcode reader reads barcode to identify assay type and carousel location. If the barcode is unreadable, the system will request a barcode override.
7. If the barcode is good or override complete, the system will check the system inventory. The user will be notified if the pack is found to be empty, invalid or outdated. Once the reagent pack is found to be good, it is ready to use.

### C. REQUESTING A TEST

1. User has two options for requesting a test or group of tests on one or more patient samples.
   (a) User may download the test request loadlist from a host computer to create an order list.
   (b) User enters test request or creates an order list on the System directly.
2. If sample cups (no barcode) are used, the following scenario occurs:
   (a) User refers to order list for segment ID and position number to place sample.
   (b) User loads a sample cup into referenced position in segment.
   (c) User transfers patient sample from blood collection tube into sample cup.
   (d) Segment is placed into sample carousel.
   (e) Indication is made to instrument that samples have been loaded.
   (f) Instrument checks consumable inventories, waste status, assay calibration, etc.
   (g) Sample carousel rotates segment to segment identification reader.
   (h) Instrument reads segment identification.
3. If primary tubes (with barcode) are used, the following scenario occurs:
   (a) User loads primary tube into next available segment location on sample carousel (two types of carriers are used for primary tubes: one for tubes with heights of 75 mm and a second for tubes with heights of 100 mm.).
   (b) indication is made to instrument that samples are available to be run.
   (c) Sample carousel rotates segment to segment identification reader.

### D. SCHEDULING A TEST

1. When the sample is presented to the pipettor, the System attempts to schedule the tests ordered on that sample for processing. Each test ordered for the sample will be scheduled separately.
   (a) The System checks for adequate inventory (reagent packs, cartridges, buffer, MUP), system resources, sample time to complete the test.
   (b) The System checks for valid calibration or orders for them on the order list.
   (c) If all test requirements are met, the test is scheduled for processing.
   (d) If all test requirements are not met, the test request is moved to the exception list. Once the test requirements have been met, the test request is moved back to the order list by the user.
2. When a test has been scheduled, the system moves it to the processing list and attempts to schedule other tests ordered for that sample.
3. When all tests for the current sample have been kitted, the System advances to the next sample on the sample carousel.

### E. KITTING A TEST

1. Once a test is scheduled, it is immediately kitted. (no tests are kitted until the scheduler ensures that the test can be transferred onto the process carousel immediately and processed within the timing requirements of the assay).
2. RV carousel is rotated clockwise until an RV is detected in pipette axis position.
3. Reagent pack carousel is rotated until reagent pack for test ordered is at the actuator position. The actuator opens the reagent cartridge caps and the reagent pack carousel is then rotated until reagent pack for test ordered is in the pipette axis position. After all pipetting steps have been completed, the reagent pack carousel is rotated back to the actuator position where the reagent cartridge caps are closed.
4. Sample carousel is rotated until sample cup (or primary tube) is in pipette axis position.
5. Pipette is always at HOME position (Pipette R-axis is parked over wash station and Pipette Z-axis is at the Z-clear position) when not in use.
6. Sample kitting.
   (a) Sample aspirate.
      (i) Syringe aspirates "X" uL of air at a rate of "X" ul/sec.
      (ii) Pipette R-axis is moved over sample cup.
      (iii) Pipette Z-axis is moved down to the Z-above position.
      (iv) Pipette Z-axis is moved down to the Z-LLS position.
      (v) LLS is enabled to ensure that no liquid is currently detected.
      (vi) Pipette Z-axis is moved down at constant speed until fluid is detected or until Z-Asp limit has been reached (it will be assumed that fluid is detected).
      (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
      (viii) The following occur simultaneously until the total volume of sample required is aspirated:
         (1) Pipette Z-axis motor is moved down at a rate of "X" steps/sec.
         (2) Syringe aspirates "X" uL at a rate of 'X" ul/sec.
         (3) LLS is checked to ensure probe still in liquid.
         (4) LLS is disabled.
         (5) Pipette Z-axis is moved up to Z-clear position.
         (6) Pipette R-axis is moved over the RV sample well.
         (7) Pipette Z-axis is moved down to the dispense position within the RV sample well.
         (8) Syringe dispenses "X" uL of sample at a rate of "X" ul/sec.
         (9) Pipette Z-axis is moved up to Z-clear position.
   (b) Probe post-wash.
      The probe is washed to ensure that it is free from contamination. It is to be understood that pipette activities in both kitting and process areas are generally followed with a probe post-wash to minimize carryover from one fluid aspirate to another. In some cases, pipette activities may be preceded with a probe prewash if necessary to guarantee the validity of the next fluid aspirate. For this assay description, it will be assumed that only a post-wash is used.
      (i) The inside of the probe is cleaned first.
         (1) Pipette R-axis is moved over waste area.
         (2) Pipette Z-axis is moved down to appropriate position within the waste area.
         (3) The wash valve is opened for the amount of time specified in the assay protocol.
         (4) Wash valve is closed.
      (ii) Pipette Z-axis is moved up to the Z-clear position.
      (iii) The outside of the probe is cleaned next.
         (1) Pipette R-axis is moved over wash cup.
         (2) Pipette Z-axis is moved down to wash position within the wash cup.
         (3) The wash valve is opened for the amount of time specified in the assay protocol.
         (4) Wash valve is closed.
         (5) Pipette is returned to "HOME" position.
7. Microparticle kitting.
   (a) Microparticle aspirate (microparticles are pipetted directly into the RV incubation well to save on volume, as this is the most costly MEIA reagent).
      (i) Syringe aspirates "X" uL of air at a rate of "X" ul/sec.
      (ii) Pipette R-Axis is moved over the microparticle reagent bottle in the Reagent Pack.
      (iii) Pipette Z-axis is moved down to the Z-above position.
      (iv) Pipette Z-axis is moved down to the Z-LLS position.
      (v) LLS is enabled to ensure no-liquid currently detected.
      (vi) Pipette Z-axis is moved down at constant speed until fluid is detected or until the Z-Asp limit is reached (it will be assumed that fluid is detected)
      (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
      (viii) The following occur simultaneously until the total volume of microparticles required is aspirated:
         (1) Pipette Z-axis motor is moved down at a rate of "X" steps/sec.
         (2) Syringe aspirates "X" uL at a rate of "X" ul/sec.
         (3) LLS is checked to ensure probe still in liquid.
      (ix) LLS is disabled.
      (x) Pipette Z-axis is moved up to Z-clear position.
      (xi) Pipette R-axis is moved over the RV incubation well.
      (xii) Pipette Z-axis is moved down to the dispense position within the RV incubation well.
      (xiii) Syringe dispenses "X" uL of microparticles at a rate of "X" ul/sec. Pipette Z-axis is moved up to Z-clear position.
   (b) Probe post-wash.
      The probe is again washed to ensure that it is free from contamination as described in section 6 (Sample kitting).
8. Conjugate kitting.
   (a) Conjugate aspirate (conjugate, special wash fluid, and/or specimen diluent are pipetted into either RV reagent wells or RV predilution well, depending on volume requirements).
      (i) Syringe aspirates 'X": uL of air at a rate of "X" ul/sec.
      (ii) Pipette R-Axis is moved over the conjugate reagent bottle in the Reagent Pack.
      (iii) Pipette Z-axis is moved down to the Z-above position.
      (iv) Pipette Z-axis is moved down to the Z-LLS position.
      (v) LLS is enabled to ensure no liquid currently detected.
      (vi) Pipette Z-axis is moved down at constant speed until fluid is detected or until the Z-Asp limit is reached (it will be assumed that fluid is detected.
      (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
      (viii) The following occur simultaneously until the total volume of conjugate required is aspirated:
         (1) Pipette Z-axis motor is moved down at a rate of "x" steps/sec.
         (2 ) Syringe aspirates "X" uL at a rate of "X" ul/sec.
         (3) LLS is checked to ensure probe still in liquid.
      (ix) LLS is disabled.
      (x) Pipette Z-axis is moved up to Z-clear position.
      (xi) Pipette R-axis is moved over the RV reagent well.
      (xii) Pipette Z-axis is moved down to the dispense position within the RV r reagent well.
      (xiii) Syringe dispenses "X" uL of conjugate at a rate of "X" ul/sec.
      (xiv) Pipette Z-axis is moved up to Z-clear position.
   (b) Probe post-wash.
      The probe is again washed to ensure that it is free from contamination as described in section 6 (Sample kitting).
9. MEIA Buffer Kitting.
   (a) RV Carousel is rotated until RV buffer well is under the MEIA buffer dispenser at buffer kitting station.
   (b) "X" uL of MEIA buffer is dispensed into the buffer well at a rate of "X" ul/sec

### F. TRANSFERRING RV INTO PROCESS AREA

1. RV carousel is rotated to transfer station.
2. Process carousel is rotated so that the empty position is aligned with the transfer station.
3. Transfer mechanism 0-axis is rotated to sample entry area.
4. Transfer mechanism R-axis grabs the RV and pulls it into the transfer mechanism.
5. Transfer mechanism 0-axis is rotated so that RV is aligned with the empty position on the process carousel.
6. RV is loaded onto process carousel.

### SYSTEM DESCRIPTION OF MEIA PROCESS AREA FOR CEA

### A. System waits for temperature equilibration time and evaporation window to expire.

### B. FIRST PIPETTE ACTIVITY (microparticle/sample reaction)

1. Incubation timer is set according to assay file specifications.
2. MEIA buffer aspirate.
   (a) The process carousel is moved so that the RV is at the pipetting station.
   (b) Syringe aspirates "X" uL of air at a rate of "X" ul/sec.
   (c) Pipette R-axis is moved over the RV buffer well.
   (d) Pipette Z-axis is moved down to the Z-above position over the RV buffer well.
   (e) Pipette Z-axis is moved down to the Z-LLS position.
   (f) LLS is enabled to ensure no liquid currently detected.
   (g) Pipette Z-axis is moved down at constant speed until fluid is detected or until the Z-Asp limit is reached (it will be assumed that fluid is detected).
   (h) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
   (i) The following occur simultaneously until the total volume of MEIA buffer required is aspirated:
      (1) Pipette Z-axis motor is moved down at a rate of "X" steps/sec.
      (2) Syringe aspirates "X" uL at a rate of "X" ul/sec.
   (j) LLS is checked to ensure probe still in liquid.
   (k) LLS is disabled.
   (I) Pipette Z-axis is moved up to Z-above position.
3. Sample aspirate
   (a) Pipette R-axis is moved over the RV sample well.
   (b) Pipette Z-axis is moved down to the Z-LLS position.
   (c) LLS is enabled to ensure no liquid currently detected.
   (d) Pipette Z-axis is moved down at constant speed until fluid is detected or until the Z-Asp limit is reached (it will be assumed that fluid is detected).
   (e) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
   (f) The following occur simultaneously until the total volume of sample required is aspirated:
      (1) Pipettor Z-axis motor is moved down at a rate of "X" steps/sec.
      (2) Syringe aspirates "X" uL at a rate of "X" ul/sec.
   (g) LLS is checked to ensure probe still in liquid.
   (h) LLS is disabled.
   (i) Pipette Z-axis is moved up to Z-above position.
4. MEIA buffer and sample are added to microparticles in incubation well.
   (a) Pipette Z-axis is moved down to the dispense position within the RV incubation well.
   (b) Syringe dispenses "X" uL of MEIA buffer and sample at a rate of "X" ul/sec.
   (c) Pipette Z-axis is moved up to Z-clear position.
5. Probe post-wash.
   The probe is again washed to ensure that it is free from contamination as described in section 6 (Sample kitting).

### C. CARTRIDGE LOAD (This activity occurs when resources are not in use)

1. Move the auxiliary carousel so that reserved position is under feeder.
2. Cycle trap-door mechanism to load flashlight into carousel.
3. Cycle shuttle mechanism to place another MEIA cartridge on trap door (for next tab load).
4. Check incubation timer. When expires start next pipetting.

### D. SECOND PIPETTE ACTIVITY (transfer of reaction mixture to matrix)

1. Incubation timer is set according to assay file specifications,
2. Buffer aspirate.
   (a) The process carousel is moved so that the RV is at the pipetting station.
   (b) Syringe aspirates "X" uL of air at a rate of "X" ul/sec.
   (c) Pipette R-axis is moved over the RV buffer well.
   (d) Pipette Z-axis is moved down to the Z-above position.
   (e) Pipette Z-axis is moved down to the Z-LLS position.
   (f) LLS is enabled to ensure no liquid currently detected.
   (g) Pipette Z-axis is moved down at constant speed until fluid is detected or until the Z-Asp limit is reached (it will be assumed that fluid is detected).
   (h) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
   (i) The following occur simultaneously until the total volume of buffer required is aspirated:
      (1) Pipette Z-axis motor is moved down at a rate of "X" steps/sec.
      (2) Syringe aspirates "X" uL at a rate of "X" ul/sec.
   (j) LLS is checked to ensure probe still in liquid.
   (k) LLS is disabled.
   (l) Pipette Z-axis is moved up to the Z-above position.
3. Reaction mixture aspirate.
   (a) Pipette R-axis is moved over the RV incubation well.
   (b) Pipette Z-axis is moved down to the Z-LLS position.
   (c) LLS is enabled to ensure no liquid currently detected.
   (d) Pipette Z-axis is moved down at constant speed until fluid is detected or until the Z-Asp limit is reached (it will be assumed that fluid is detected).
   (e) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
   (f) The following occur simultaneously until the total volume of reaction mixture required is aspirated:
      (1) Pipette Z-axis motor is moved down at a rate of "X" steps/sec.
      (2) Syringe aspirates "X" uL at a rate of "X" ul/sec.
   (g) LLS is checked to ensure probe still in liquid.
   (h) LLS is disabled.
   (i) Pipette Z-axis is moved up to the Z-clear position.
4. Reaction mixture dispense onto matrix.
   (a) The following are performed simultaneously and concurrently with the reaction mixture aspirate (above):
      (i) The auxiliary carousel is moved so that the cartridge is at the pipetting station.
      (ii) Pipette R-axis is moved over the MEIA cartridge (matrix) surface.
      (iii) Pipette Z-axis is moved down to the matrix dispense position.
      (iv) Syringe dispenses "X" uL of reaction mixture at a rate of "X" ul/sec.
      (v) System delays "X" seconds until reaction mixture has been absorbed by matrix.
5. Buffer wash of matrix.
   (a) Syringe dispenses "X" uL of buffer at a rate of "X" ul/sec.
   (b) Pipette Z-axis is moved up to the Z-clear position.
6. Probe post-wash.
   The probe is again washed to ensure that it is free from contamination as described in section 6 (Sample kitting).
7. When incubation timer expires, next pipette activity begins.

### E. THIRD PIPETTE ACTIVITY (conjugate addition)

1. Incubation timer is set according to assay file specifications.
2. Conjugate aspirate.
   (a) The process carousel is moved so that the RV is at the pipetting station.
   (b) Syringe aspirates "X" uL of air at a rate of "X" ul/sec.
   (c) Pipette R-axis is moved over the RV reagent 1 (conjugate) well.
   (d) Pipette Z-axis is moved down to the Z-above position.
   (e) LLS is enabled to ensure no liquid currently detected.
   (f) Pipette Z-axis is moved down at constant speed until fluid is detected or until the Z-Asp limit is reached (it will be assumed that fluid is detected).
   (g) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
   (h) The following occur simultaneously until the total volume of conjugate required is aspirated:
      (i) Pipette Z-axis motor is moved down at a rate of "X" steps/sec.
      (ii) Syringe aspirates "X" uL at a rate of "X" ul/sec.
   (i) LLS is checked to ensure probe still in liquid.
   (j) LLS is disabled.
   (k) Pipette Z-axis is moved up to the Z-clear position.
3. Conjugate dispense (performed simultaneously).
   (a) The auxiliary carousel is moved so that the cartridge is at the pipetting station.
   (b) Pipette R-axis is moved over the cartridge (matrix) surface.
   (c) Pipette Z-axis is moved down to the matrix dispense position.
   (d) Syringe dispenses "X" uL of conjugate at a rate of "X" ul/sec.
   (e) Pipette Z-axis is moved up to the Z-clear position.
   (f) Wait "X" seconds until reaction mixture has been absorbed by matrix.
4. Probe post-wash.
   The probe is again washed to ensure that it is free from contamination as described in section 6 (Sample kitting).

### F. RV UNLOAD (This activity occurs when resources are not in use)

1. The following are performed simultaneously:
   (a) Process carousel, is rotated so that the empty position is at the transfer station.
   (b) Transfer mechanism 0-axis is moved to process carousel.
2. RV is grabbed with the transfer mechanism R-axis and pulled into the transfer mechanism.
3. Transfer mechanism 0-axis is rotated so that RV is aligned with the waste container.
4. RV is pushed into the waste container.
5. Check incubation timer. When expires start next activity.

### G. MEIA READ PREPARATION

1. Lamp intensity is brought from simmer state to burn state.
2. PMT gain is set.

### H. MATRIX WASH

1. Auxiliary carousel is rotated so that the cartridge is at the matrix wash station.
2. The following steps are repeated until all the buffer specified in the assay file for cartridge wash has been dispensed.
   (a) "X" uL of heated MEIA buffer are dispensed in 50uL cycles at a rate of "X" ul/sec onto the matrix.
   (b) Wait "n" seconds.

### 1. MUP DISPENSE

1. Auxiliary carousel is rotated so that the cartridge is at the MUP station.
2. 5OuL of heated MUP are dispensed at a rate of "X" uL/sec onto the matrix.
3. Wait "n" seconds.

### J. MEIA READ

1. Auxiliary carousel is rotated so that the cartridge is at the read station.
2. The following steps are repeated until the number of micro-reads specified in the assay file have been taken (usually 8)
   (a) Read for "X.XX" seconds.
   (b) Wait "X.XX" seconds.
3. The reader is returned to its idle state.
   (a) Lamp intensity is turned to simmer state.
   (b) PMT gain is set.
4. The raw reads are converted to normalized reads (light intensity hitting detector/lamp intensity) by the optics microprocessor.
5. A rate is calculated by the System from the normalized reads vs time.
6. For quantitative assays, the rate is fitted to a calibration curve to yield a concentration result.
7. For qualitative assays, the sample rate is compared to an index or cutoff rate to determine if the sample is positive or negative (or reactive or nonreactive).

### K. CARTRIDGE UNLOAD (This activity occurs when resources are not in use)

1. Auxiliary carousel is rotated so that cartridge is at the ejector station.
2. Ejector is cycled to place cartridge into waste container.

Schematic reaction sequences are presented in FIGURES-26, 27 and 28 which are typical of assays that can be handled by the automated immunoassay analytical system of the invention. In FIGURE 26, a T4 assay, FPIA sequence 420, is presented wherein Step 1, T4 bound by thyroxine binding protein (TBP) 424, is reacted with T4 displacing agent 426 to yield TBP 428 plus unbound T4 (430). In step 2, the T4 (430) is added to T4 antibody 432 which yields a reaction product 434 (T4 antibody-T4 complex). In Step 3, the T4 antibody-T4 complex 434 is treated with T4 tracer (fluorescent) 436 which yields a fluorescent polarization measurable reaction product 438.

In FIGURE 27, a schematic reaction sequence 440 for a 1-step sandwich MEIA determination (ferritin) is presented. In Steps 1 and 2 an anti-ferritin alkaline phosphatase conjugate is mixed with ferritin sample 444 and anti-ferritin microparticles 446 to yield a ferritin antibody-antigen-antibody complex 448. In step 3, the antibody-antigen-antibody complex 448 is reacted with 4-methylumbelliferyl phosphate (MUP) 450 which yields methylumbelliferone (MU) which is fluorescent. The rate of MU production is measured.

In FIGURE 28, the schematic reaction sequence 456 for a 2-step sandwich MEIA is provided for HTSH assay. Anti-hTSH specific microparticles 458 are added to the HTSH sample 460 which provides a reaction product HTSH antibody-antigen complex 462. In Steps 2 through 4, the complex 462 is combined with an anti-hTSH alkaline phosphatase 464 yielding hTSH antibody-antigen-antibody complex 466. In step 5, the complex 466 is reacted with MUP 450 to yield MU which is fluorescent. The rate of MU production is measured.

The present invention may be more fully appreciated by reference to many embodiments which are described hereinbelow.

According to a first embodiment of the invention, there is provided a method of operating an automated, continuous and random access analytical system capable of simultaneously effecting multiple assays of a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) transferring one or more of said unit dose disposables to a processing workstation, (d) mixing an aliquot of said first liquid sample with one or more of said reagents at different times in said reaction vessel to form a first reaction mixture (e) mixing aliquots of the same or different one or more samples with one or more of said reagents at different times in different reaction vessels to form multiple, independently scheduled reaction mixtures, (f) incubating said multiple reaction mixtures simultaneously and independently, (g) performing more than one scheduled assay on said reaction mixtures in any order in which more than scheduled assays are presented, and (h) analyzing said incubated reaction mixtures independently and individually by at least two assay procedures.

In a preferred embodiment of such method, at least two different assays are scheduled to be performed on the system for a plurality of liquid samples, and said method provides scheduling of said assays in advance of performance thereof, each assay test definition containing several timing parameters with each activity of the assay test containing time values which scheduling uses to determine which resources of the system and activity required by each of said assays and the time period that said resources need. In the latter embodiment, preferably the system is capable of allowing special priority handling through stat procedure scheduling of specific samples, and said stat procedure scheduling interrupts prior scheduling, allowing the system to finish preparing assays on a current sample and then prepare to perform an assay on the sample through a modification of the scheduling, particularly when calibration procedures scheduling is scheduled as a stat procedure. Alternatively in said latter embodiment, preferably scheduling for performing assays maximizes the number of assays the system is capable of processing per unit time by allowing sufficient time gaps between assay protocol steps to enable other assay protocol steps to be performed within such time gaps.

In said first embodiment of the method, the scheduling process preferably includes scheduling of each activity to be performed in an assay before the assay is kitted, and scheduling performance of each assay activity prior to its originally scheduled execution time, thus minimizing resource idle time. In this preferred embodiment, assay throughput may be increased in the system. Also in this preferred embodiment, operating the automated continuous and random access analytical system includes kitting a unit dose disposable by separately transferring assay samples and reagents to a reaction vessel without initiation of an assay reaction sequence.

In said first embodiment, the assay performed on said reaction mixture in said reaction vessel may be a homogeneous assay or a heterogeneous assay. Preferably, at least two assays are immunoassays, for example immunoassays comprised of MEIA and FPIA assays. The analyzing step may include optically monitoring said reaction mixtures. The reaction mixtures may be monitored by turbidimetric, colorimetric, fluorometric or luminescent means. Partial initiation of an assay reaction sequence may be achieved simultaneously with the creating of the unit dose disposable. The system may achieve simultaneous creation of unit dose disposables, transferring of a unit dose disposable reaction vessel and mixing of a reaction mixture while incubating multiple reaction mixtures and performing at least one scheduled assay and analysis simultaneously.

According to a second embodiment of the invention, there is provided a method of operating an automated, continuous and random access analytical system capable of simultaneously effecting multiple assays of a plurality liquid samples, comprising (a) introducing sample cups, reagent packs, and reaction vessels for performing said assays onto concentric carousels of a front end carousel, the reaction vessels being introduced to an outer carousel, (b) identifying the reagent packs and sample cups, (c) scheduling the assays, (d) aligning the sample cups and reagent packs with a reaction vessel at a kitting station by rotating the respective carousels, (e) kitting a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent pack, (f) transferring the kitted reaction vessel to a process carousel which is maintained under controlled environment conditions, (g) pipetting the sample and various reagents into a reaction well of the reaction vessel, the amounts of reagent, sequencing of transfer and time spacing there between being predetermined by assay scheduling, (h) incubating the pipetted sample and reagent mix, (i) identifying and transferring the incubated mixture in the reaction well to one of at least two assay analytical stations, (j) performing an analysis by reading the prepared reaction mixture and calibrating the reading, and (k) recording the resulting assay reading analysis. Preferably, the front-end carousel and the concentric carousels of the front end carousel as well as the process carousel are rotably disposed for bidirectional rotational motion about a vertical axis, particularly when the front end carousel being capable of bidirectional motion provides a bidirectional shaking motion for stirring or agitating reagent pack reagents after a period of inactivity of the front end carousel. In said second embodiment, preferably both kitting and partial initiation of an assay reaction sequence is achieved simultaneously creating a unit dose disposable within the reaction vessel. The assay being performed on said reaction mixture in said reaction vessel may be a heterogeneous assay or a homogenous assay. Preferably, at least two assays are immunoassays, for example immunoassays comprised of a fluorescent polarization immunoassay and a microparticle immunoassay. In the fluorescence polarization immunoassay, a FPIA reading sequence may include lamp simmer and full burn modes. In the microparticle immunoassay, settling of the microparticles may be substantially eliminated by providing a sufficient sucrose concentration to microparticle diluent ratio to achieve neutral density. Furthermore, in the microparticle immunoassay, the kitted sample and reagents may be pipetted directly from the reaction vessel on the process carousel to a microparticle immunoassay matrix for optically monitoring said reaction mixtures. In said second embodiment of the invention, the reagent packs may be provided with closure elements for avoiding reagent evaporation, particularly when covering is provided to the reagent packs when not in use to avoid evaporation of the reagents. Pipetting functions on the front end carousel and pipetting functions on the process carousel are achieved by aspirating-dispensing, driven by an airless syringe pump.

In a further embodiment of the invention, there is provided a method of operating an automated, continuous and random access analytical system capable of simultaneously effecting multiple assays to determine the presence or amount of a plurality of analyte of interest in a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) transferring one or more of said unit dose disposables to a processing workstation, (d) mixing an aliquot of said first sample with one or more of said reagents at different times in said reaction vessel to form a first reaction mixture, (e) mixing aliquots of the same or different ones of said samples with one or more of said reagents at different times in different reaction vessels to form multiple, independently scheduled reaction mixtures, (f) incubating said multiple reaction mixtures simultaneously and independently, (g) performing more than one scheduled assay on said reaction mixtures in any order in which said scheduled assays are presented, and (h) analyzing said incubated reaction mixtures independently and individually by at least two assay procedures to determine the presence or amount of one or more analyte of interests in said samples.

The present invention also provides an automated, continuous and random access analytical system apparatus capable of simultaneously effecting multiple assays of a plurality of liquid samples, comprising (a) a front end carousel assembly inclusive of a sample cup carousel, a reagent pack carousel and a reaction vessel carousel mounted concentrically and serviced by a transfer pipetting means suitable for kitting a reaction vessel, (b) a transfer station providing means for transferring a kitted reaction vessel to a process carousel, the process carousel maintained within a controlled environment, (c) a process carousel transfer pipetting means suitable for mixing reagents with the sample in a reaction well of the reaction vessel, (d) means for transferring the resulting reaction mixture to one of at least two assay reader means, (e) means for transferring a reaction vessels from the assay reader to a transfer station, and (f) means associated with said transfer station for removing the disposable reaction vessel from the system. One assay reader means may be comprised of a cartridge wheel carousel containing multiple disposable cartridges, the apparatus providing means for supplying said cartridges to the cartridge wheel carousel and disposing of the cartridges from the cartridge wheel carousel. The assay reader means may include means for optically monitoring said assay reaction. Preferably, the assay reader means provides calibration and reader means as well as recording means for the resulting assay data. The transfer station transfer means may be comprised of a carousel rotatable about an axis, an arm with a pick for mating with a reaction vessel transfer projection means and means for pulling the reaction vessel from the front end carousel, rotating and placing the reaction vessel onto the process carousel through rotation and rack and pinion movement of the pick arm. The sample handling means and reagent handling means may include means for identifying said liquid samples and liquid reagents from coded information associated with the sample cups and the reagent packs. The apparatus may further include means to store the output readings of the assay reader. The apparatus may further include means to calculate the concentration of an analyte from the output readings of said assay reader. Advantageously, the reaction vessel may contain a reaction cuvette having a physical characteristic of low birefringence through an optical read region.

A still further embodiment of the invention is an automated, continuous and random access analytical system capable of simultaneously effecting multiple assays of a plurality of liquid samples, comprising (a) a front end carousel assembly inclusive of sample cup carousel, reagent pack carousel and reaction vessel carousel, the reaction vessel carousel being concentrically mounted exterior of the reagent pack carousel and the reagent pack carousel being concentrically mounted exterior of the sample cup carousel, (b) means for rotating the respective carousels to align with a kitting pippettor means for kitting reaction vessels, (c) means for transferring kitted reaction vessels from the reaction vessel carousel to a transfer station, the transfer station providing means for transferring the reaction vessel to a process carousel, the process carousel having environmental means for maintaining temperature control and timing of reaction incubation, (d) a transfer pippettor means for servicing the process carousel and a cartridge wheel carousel offset from the process carousel, the cartridge wheel carousel having means for receiving pipetted reaction mixtures from the process carousel and; means for supplying cartridges to the process carousel, (e) the process carousel having integrated therewith a microparticle enzyme immunoassay reader and processing station, (f) a fluorescent polarization immunoassay reader and processing station integrated with the process carousel, (g) means for removing the reaction vessel from the process carousel by operation of the transfer station and means for removing the cartridges from the cartridge wheel carousel, and (h) means for analyzing the reaction mixture either by fluorescent polarization immunoassay or microparticle immunoassay. The assay-reader means may include means for optically monitoring said assay reaction. The assay reader means may provide calibration and reader means as well as recording means for the resulting assay data. The transfer station transfer means may be comprised of a carousel rotatable about an axis, an arm with a pick for mating with a reaction vessel transfer projection means and means for pulling the reaction vessel from the front end carousel, rotating and placing the reaction vessel onto the process carousel through rotation and rack and pinion movement of the pick arm. The sample handling means and reagent handling means may include means for identifying said liquid samples and liquid reagents from coded information associated with the sample cups and the reagent packs. The system may include means to store the output readings of the assay reader. The system may include a means to calculate the concentration of an analyte from the output readings of said assay reader. Advantageously, the sample cup may be free standing on its base when separated from the sample cup carousel.

The present invention also provides a bubble flushing aspirating and dispensing syringe having precision and volumetric accuracy, comprising (a) reciprocating piston within a bore, the bore having seal means at a first end and a closed end at a second end, (b) an annulus defined between the piston, the bore wall and seal means, (c) fluid inlet and fluid outlet means in communication with the annulus and bore, the fluid inlet and fluid outlet means being located between the seal means and the bore closed end, (d) a fluid source in communication with the inlet means, (e) a fluid conduit in communication with the outlet means and an open ended release tip, and (f) a drive means for reciprocating the piston within the bore. Preferably, the fluid inlet and fluid outlet means are positioned approximal to the seal means and the fluid inlet and fluid outlet means are positioned about 180° apart. Preferably, the reciprocating piston has a piston head of a similar geometry as the inside configuration of the bore closed end, said piston head being preferably dome-shaped. In the bubble flushing aspirating and dispensing syringe, the reciprocating piston in its full inward extension position is preferably positioned sufficiently close to touching the inside bore end to disrupt any bubbles in the bore end. Preferably, the reciprocating piston in its full outward extension position is positioned with the piston head being flush with the seal and positioned between the fluid entry port and the fluid exit port. The fluid entry port of the syringe may be in communication with a pressurized source of fluid.

The present invention furthermore provides a bubble flushing aspirating and dispensing syringe having volumetric accuracy, comprising (a) a syringe frame, (b) a linear actuator mounted to the frame, (c) an actuator motor for spinning a nut means in communication with a lead screw, the lead screw being affixed to the coupler which has a bearing on one side, (d) the bearing mounted in a groove in the frame, (e) the coupler is rotationally constrained by the bearing, the coupler reciprocates when the linear actuator motor spins the nut, and the piston which is affixed to the coupler provides reciprocating motion of the piston through a seal means and a bore, (f) the reciprocating piston mounted within the bore having a closed end spaced from the seal means, (g) an annulus defined by the piston and bore, (h) fluid inlet and fluid outlet means in communication with the annulus and bore, the fluid inlet and fluid outlet means located between the seal means and the bore closed end, (i) a fluid source in communication with the inlet means, and (j) a fluid conduit in communication with the fluid outlet means and an open ended release tip. Preferably, the fluid inlet and fluid outlet means are positioned approximal to the seal means and the fluid inlet and fluid outlet means are positioned at about 180° apart. Preferably, the reciprocating piston has a piston head of a similar geometry as the inside configuration of the bore closed end, said piston head being preferably domed-shaped. The reciprocating piston, having a piston head of a similar geometry as the inside configuration of the bore closed end, in its full outward extension position from the bore end may be positioned with the piston head being flush with the seal and positioned between the fluid inlet and fluid outlet means. Preferably, the reciprocating piston in its full inward extension position is positioned sufficiently close to touching the inside bore end to disrupt any bubbles in the bore end. The fluid inlet means may be in communication with a pressurized fluid source.

In addition, the present invention provides a method for flushing bubbles from an aspirating and dispensing syringe and a fluidic system utilizing such syringe, which method comprises the steps of (a) introducing fluid into an annulus defined by a piston and bore wall, the annulus closed at a first end of the bore by seal means between the bore wall and the piston and at a second end of the bore by a closed bore end, (b) flowing the fluid through the annulus around the sides of the piston and out through a fluid outlet, (c) creating a cross flow pattern approximate to the seal area across the end of the piston in one position and around the piston when the piston is moving in or out of the bore, (d) reciprocating the piston; and (e) flushing bubbles from the fluidic system through reciprocating of the piston at least through one complete reciprocation cycle. Preferably, the method comprises creating the cross flow pattern approximate to the seal area across the end of the piston when the reciprocating piston is in a full outward extension position with the piston head being flush with the seal and around the piston when the piston is moving in or out of the bore, flowing the fluid around the sides of the piston and recapturing the fluid at a fluid outlet which is located approximately 180° from the fluid inlet. Preferably, bubbles near or on the closed bore end are disrupted by the reciprocating piston in its full inward extension position by positioning the end of the piston sufficiently close to touching the inside bore end. The method for flushing bubbles from an aspirating and dispensing syringe may comprise flushing of the bubbles from the fluidics system through reciprocating of the piston for multiple reciprocation cycles between each aspirating and dispensing function or automatically controlled at periodic multiples of the aspirating and dispensing function.

According to a further embodiment of the invention, there is provided a bubble flushing precision positive displacement pump syringe having precision and volumetric accuracy, comprising (a) a reciprocating piston within a bore, the bore having seal means at a first end and a closed end at a second end, (b) an annulus defined between the piston and the bore wall, (c) valved fluid inlet and valved fluid outlet means in communication with the annulus and bore, the valved fluid inlet and valved fluid outlet means being located between the seal means and the bore closed end, (d) a fluid source in communication with the inlet means through a valve means, (e) a fluid conduit in communication with the outlet means and an outlet valve means, and (f) a drive means for reciprocating the pump within the bore.

The invention furthermore provides a method of operating an automated, continuous and random access analytical system capable of simultaneously effecting multiple assays of a plurality liquid samples, comprising (a) introducing sample cups, reagent packs, and reaction vessels for performing said assays onto concentric carousels of a front end carousel, the reaction vessels being introduced to an outer carousel, (b) identifying the reagent packs and sample cups, (c) scheduling the assays, (d) aligning the sample cups and reagent packs with a reaction vessel at a kitting station by rotating the respective carousels, (e) kitting a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent pack, (f) transferring the kitted reaction vessel to a process carousel which is maintained under controlled environment conditions, (g) pipetting the sample and various reagents into a reaction well of the reaction vessel, the amounts of reagent, sequencing of transfer and time spacing there between being predetermined by assay scheduling, (h) pipetting functions on the front end carousel and pipetting functions on the process carousel are achieved by a bubble flushing aspirating and dispensing syringe, (i) incubating the pipetted sample and reagent mix, (j) identifying and transferring the incubated mixture in the reaction well to one of at least two assay analytical stations, (k) performing an analysis by reading the prepared reaction mixture and calibrating the reading, and (1) recording the resulting assay reading analysis.

The invention also provides an automated, continuous and random access analytical system apparatus capable of simultaneously effecting multiple assays of a plurality of liquid samples, comprising (a) a front end carousel assembly inclusive of a sample cup carousel, a reagent pack carousel and a reaction vessel carousel mounted concentrically and serviced by a transfer pipetting means in combination with a bubble flushing aspirating and dispensing syringe suitable for kitting a reaction vessel, (b) a transfer station providing means for transferring a kitted reaction vessel to a process carousel, the process carousel maintained within a controlled environment, (c) a process carousel transfer pipetting means in combination with a bubble flushing aspirating and dispensing syringe suitable for mixing reagents with the sample in a reaction well of the reaction vessel, (d) means for transferring the resulting reaction mixture to one of at least two assay reader means, (e) means for transferring a reaction vessels from the assay reader to a transfer station, and (f) means associated with said transfer station for removing the disposable reaction vessel from the system.

According to another embodiment of the invention, there is provided a temperature control apparatus for reaction and incubation zone of a continuous analytical system, comprising (a) ducting and flow chamber air inlet, (b) a heating means for the air in the ducting approximal to the air inlet, (c) an air flow driving means positioned downstream from the heating means, (d) a system of ducts in communication with the inlet, heating means and air flow driving means mounted in a base plate of the analytical system, (e) ducting means for directing heated air flow to the reaction and incubation zone of the analytical system carousels to an underside of the carousels, (f) temperature sensor in communication with a controller means for regulating the heating means, (g) said ducting means in communication with the flow chamber above the carousels, (h) said ducting means and flow chamber combined to provide reduction air flow above the carousels, and (i) multiple air flow exit means from the flow chamber to an exit ducting. The air flow driving means may be comprised of a fan which is positioned between the air inlet and the heating means, particularly when the fan is a variable speed fan responsive to the temperature sensor and controller means for regulating the heating means and fan speed. The apparatus through the ducting and flow chamber preferably provides the reaction and incubation zone of the continuous analytical system with single pass air flow. The ducting and flow chamber may provide the reaction and incubation zone of the continuous analytical system with recirculation of the heated air up to about 50% of the total air circulation requirement. The heating means may be comprised of at least one electrical resistant heating element positioned in the air flow path ducting means. The ducting and flow chamber air inlet may be provided with filter means. The flow chamber may have positioned prior to the multiple air flow exit means an ambient air entry with an air flow driving means for introducing ambient air directly on to fluidics heater blocks for cooling purposes.

In another aspect, the invention provides a method for controlling temperature of reaction and incubation zones of a continuous analytical system, comprising (a) introducing air into ducting and the reaction and incubation zones of the system, (b) heating the introduced air in the ducting flow path before introduction of the air to the reaction and incubation zones, (c) driving the introduced and heated air into the reaction and incubation zones to an underside portion of carousels of the system, the driving providing turbulent flow, high pressure drop in the zone below the carousels, (d) temperature sensing and control means for regulating the heating of the introduced air, (e) reducing the air flow by expansion into a flow chamber above the carousels to a minimum air flow sufficient only to maintain temperature within the chamber above the carousels, (f) avoiding evaporation of reagents and sample fluids contained in the carousels and exposed to the flow chamber atmosphere, and (g) exiting the air flow from the chamber through multiple exit ports. Preferably, the sensing means controls the heating means as well as the driving means volume capacity. Preferably, the heated air flowing through the ducting is first introduced under turbulent flow conditions to carousel undersides in the reaction and incubation zone, adjusting the temperatures of these zones as quickly as possible, particularly when temperature variations are minimized within the reaction and incubation zone by air flow volume and temperature control in order to accommodate the constant changes occurring within the reaction and incubation zone of the continuous and random access system.

The invention also provides a method of operating an automated, continuous and random access analytical system capable of simultaneously effecting multiple assays of a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) transferring one or more of said unit dose disposables to a processing workstation, (d) mixing an aliquot of said first liquid sample with one or more of said reagents at different times in said reaction vessel to form a first reaction mixture, (e) mixing aliquots of the same or different one or more samples with one or more of said reagents at different times in different reaction vessels to form multiple, independently scheduled reaction mixtures, (f) incubating said multiple reaction mixtures simultaneously and independently, (g) introducing air into the ducting flow path before introduction of the air to the reaction and incubation zones, (h) heating the introduced air in the ducting flow path before introduction of the air to the reaction and incubation zones, (i) driving the introduced and heated air into the reaction and incubation zones to an underside portion of carousels of the system, the driving providing turbulent flow, high pressure drop in the zone below the carousels, (j) temperature sensing and control means for regulating the heating of the introduced air, (k) reducing the air flow by expansion into a flow chamber above the carousels to a minimum air flow sufficient only to maintain temperature within the chamber above the carousels, (1) avoiding evaporation of reagents and sample fluids contained in the carousels and exposed to the flow chamber atmosphere, (m) exiting the air flow from the chamber through multiple exit ports, (n) performing more than one scheduled assay on said reaction mixtures in any order in which more than scheduled assays are presented, and (o) analyzing said incubated reaction mixtures independently and individually by at least two assay procedures.

In another embodiment of the invention, an apparatus is provided for improving fluorescent lamp life within continuous and random access analytical systems requiring quick start up response of the lamp after periods of shut down modes, such start up, burn and standby cycles being required by multiple process capabilities of the system, the apparatus comprising (a) an optical assembly, (b) a light source means, (c) an excitation filter means, (d) a filter reflector between the light source means and a lens, the lens being upstream from the object which is capable of reflecting fluorescent light, (e) reflected fluorescent light feedback filter means, (f) photomultiplier means, and (g) a heater means for maintaining the light source means at a predetermined temperature during light source means shut off. Preferably, the light source means is comprised of a mercury source lamp, particularly when the mercury source lamp is heated to a temperature by the heating element sufficient to maintain mercury in the mercury source lamp in a vapor state. Preferably, the optical assembly contains a sensing means for detecting light from the light source means to determine operational burn of the light source means, particularly when the sensing means for detecting light from the light source means is comprised of a band pass filter means between the light source means and a photodiode.

In another aspect of the invention, there is provided a method for improving fluorescent lamp life within an automated, continuous and random access analytical system requiring quick start up lamp burn and multiple periods of shut down of the fluorescent lamp, comprising maintaining the fluorescent lamp source at an elevated temperature during shut down and promoting quick start up burn of the lamp source within one second or less. In such method, preferably the lamp source is a mercury lamp source which is heated to about 70°C or sufficient temperature to maintain mercury in the mercury source lamp in a vapor phase. Also in such method, preferably the optical assembly is suitable for a MEIA diagnostic system within automated, continuous and random access analytical systems having at least two or more independent systems inclusive of the MEIA assay system.

According to another embodiment of the invention, there is provided a method of operating an automated, continuous and random access analytical system capable of simultaneously effecting multiple assays to determine the presence or amount of a plurality of analyte of interest in a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) transferring one or more of said unit dose disposables to a processing workstation, (d) mixing an aliquot of said first sample with one or more of said reagents at different times in said reaction vessel to form a first reaction mixture, (e) mixing aliquots of the same or different ones of said samples with one or more of said reagents at different times in different reaction vessels to form multiple, independently scheduled reaction mixtures, (f) incubating said multiple reaction mixtures simultaneously and independently, (g) performing more than one scheduled assay on said reaction mixtures in any order in which said scheduled assays are presented, and (h) analyzing said incubated reaction mixtures independently and individually by at least two assay procedures to determine the presence or amount of one or more analyte of interests in said samples; the improvement comprising a method for improving fluorescent lamp life within an automated, continuous and random access analytical system requiring quick start up lamp burn and multiple periods of shut down of the fluorescent lamp, comprising: (i) maintaining the fluorescent lamp source at an elevated temperature during shut down, and (ii) promoting quick start up burn of the lamp source within one second or less.

The invention also provides a method for improving tungsten filament lamp life within an optical measurement device in continuous and random access analytical systems requiring short full burn response times from a standby, low power, simmer mode, comprising (a) reducing the burn intensity of the tungsten filament upon completion of a read by electrical circuit and computer control, (b) maintaining the tungsten filament lamp at a simmer burn or intensity level which maintains a sufficient temperature of the filament to allow shift from simmer burn to full burn within about one second or less upon command for read, and (c) avoiding constant full burn of the tungsten filament lamp during standby nonuse of the reader within automated continuous and random access analytical systems which allow the reader to remain on standby a significant portion of instrumentation usage. The reader system may be a FPIA assay system.

According to one embodiment of the present invention, there is provided a method of operating an automated, continuous and random access analytical system capable of simultaneously affecting multiple assays to determine the presence or the amount of a plurality of analyte of interest in a plurality of liquid samples, comprising (a) scheduling various assays of a plurality of liquid sample, (b) creating one or more unit dose disposables by separately transferring a first of said liquid samples and reagents to a reaction vessel without initiation of an assay reaction sequence, (c) transferring one or more of said unit dose disposables to a processing work station, (d) mixing an aliquot of said first sample with one or more of said reagents at different times and said reaction vessel to form a first reaction mixture, (e) mixing aliquots of the same or different ones of said samples with one or more of said reagents at different times in different reaction vessels to perform multiple, independently scheduled reaction mixtures, (f) incubating said multiple reaction mixtures simultaneously and independently, (g) performing more than one scheduled assay on said reaction mixtures in which said scheduled assays are presented, (h) analyzing said incubated reaction mixtures independently and individually by at least two assay procedures to determine the presence or the amount of one or more analyte of interest in said samples, (i) the method further comprised of improving tungsten filament lamp life within an optical measuring device in the automated, continuous and random access analytical system requiring short, full burn response times from a standby, low power, simmer mode necessitated by reducing the bum intensity of the tungsten filament upon completion of an assay read by electrical circuit and computer control, (j) maintaining the tungsten filament lamp at a simmer burn or intensity level which maintains a sufficient temperature of the filament to allow shift from simmer burn to full burn within about one second or less upon command from the assay read, and (k) avoiding constant full burn of the tungsten filament lamp during standby nonuse of the assay reader within automated, continuous and random access analytical systems which allow the assay reader to remain on standby, a significant portion of instrumentation usage.

The invention also provides an apparatus for improving tungsten filament lamp life within continuous and random access analytical systems requiring quick start up response of the lamp after periods of standby simmer burn modes, such start up, high burn and standby cycles being required by multiple process capabilities of the system, the apparatus comprising (a) an optical assembly, (b) a tungsten filament lamp light source means, (c) an excitation filter means, (d) a beam splitter means splitting light energy to a polarizer means and liquid crystal means and a lens upstream from an object to be read, the object being capable of permitting light through lens means and an emission filter means, (e) a light emission polarizer means and focusing lens means upstream from a photomultiplier tube means, and (f) means for maintaining the tungsten filament lamp with a simmer burn mode during standby and means for increasing energy to the tungsten filament lamp to bring the lamp to full burn in short response time. In one embodiment, the beam splitter means directs a portion of the original light from the tungsten filament lamp through a lens means to a reference detector means for control purposes of controlling standby and bright burn modes of the tungsten halogen source lamp.

The invention furthermore provides a reaction vessel suitable for multiple assay utilization within an automated, continuous and random access analytical system which allows for kidding of sample and reagents and physical transfer of the kidded reaction vessel to a process carousel, comprising (a) multiple wells of various volume capacities, the multiple wells having openings on the same plane and depths extending from said plane the reaction vessel having at least one curvette, the curvette extending substantially below the multiple wells, the curvette having an opening on the same plane as the multiple wells, and (b) at a transfer projection on a well bottom portion of the well on a first end portion of the reaction vessel, the curvette projecting downward from a second end portion of the reaction vessel. The multiple wells may have various volume capacities and the same or different well cross section and depth. Preferably, the multiple wells and curvette have openings on the same plane and are joined on that plane by a platform having a thickness providing rigid strength to the multiple wells and the curvette, said platform, wells and curvette preferably being comprised of a single molded article wherein various well walls and curvette walls extending downwardly in a parallel direction from the platform define additional reinforcing material of construction for rigidity purposes. Preferably, the curvette is constructed with an optical reader region characterized by low birefringence.

Additionally, the invention provides an automated, continuous and random access analytical system apparatus capable of multiple assays of a plurality of liquid samples, comprising (a) a front end carousel assembly inclusive of sample cup carousel, a reagent pack carousel and a reaction vessel carousel mounted concentrically and serviced by a transfer pipetting means suitable for kidding a reaction vessel, (b) the reaction vessel having multiple wells of various capacities and at least one curvette, the curvette extending substantially below the multiple wells, (c) a well on a first end portion of the reaction vessel having a transfer projection on a bottom portion, the curvette projecting downward from a second end portion of the reaction vessel substantially beyond the projection depths of the multiple wells, the multiple wells and curvette extending from openings on the same plane, the projections being perpendicular to that plane, (d) a transfer station providing means for transferring a kidded reaction vessel through utilization of the well transfer projection to a process carousel, the process carousel maintained within a controlled environment, (e) a process carousel transfer pipetting means suitable for mixing reagents with the sample in a reaction well of the reaction vessel, (f) means for transferring the resulting reaction mixture to one of at least two assay reader means, (g) means for transferring a reaction vessel through use of the well transfer projection from the assay reader to a transfer station, and (h) means associated with said transfer station for removing the disposable reaction vessel from the system.

A further embodiment of the invention is a method of operating an automated, continuous and random access analytical system capable of simultaneously effecting multiple assays of a plurality liquid samples, comprising (a) introducing sample cups, reagent packs and reaction vessels for performing said assays on concentric carousels of a front end carousel, the reaction vessels being introduced to an outer carousel, (b) identifying the reagent packs and sample cups, (c) scheduling the assays, (d) aligning sample cups and reagent packs with a reaction vessel indicating the station by rotating the respective carousels, (e) kidding a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent packs, (f) transferring the kidded reaction vessel to a process carousel which is maintained under controlled environment conditions by means of a transfer station and use of a transfer projection on a bottom portion of a reaction vessel well which is exposed by the reaction vessel carousel at the circumference of the carousel, (g) pipetting the sample and various reagents into a reaction well of the reaction vessel, the amounts of reagent, sequencing of transfer and time spacing therebetween being predetermined by assay scheduling, (h) incubating the pipetted sample and reagent mix, (i) identifying and transferring the incubated mixture in the reaction well to one of at least two assay analytical stations, (j) performing an analysis by reading the compared reaction mixture and calibrating the readings, and (k) recording the resulting assay reading analysis. Both pipetting and partial initiation of an assay reaction sequence may be achieved simultaneously be creating a unit dose disposable within the reaction vessel followed by transfer of the reaction vessel to the process carousel. In a preferred embodiment, the scheduled assay is FPIA and the FPIA read is through the reaction vessel curvette. In another preferred embodiment, the reaction vessel may be transferred by means of a transfer station which pulls the reaction vessel from the front end carousel reaction vessel carousel by a pick means which mates with the transfer projection means, the transfer station rotating and placing the reaction vessel onto the process carousel through rotation movement of the pick arm. In the latter embodiment, the transfer station transfer means may remove the reaction vessel from the process carousel by pulling the reaction vessel from the process carousel to the transfer station by means of mating a pick with the reaction vessel transfer projection and pulling the reaction vessel from the process carousel and rotating the transfer station to a position allowing disposal of the used reaction vessel.

The present invention additionally provides a reaction vessel packaging and loading device, comprising a semi-rigid plastic strip having an upper portion handling ledge, the ledge having cut-outs between reaction vessel mounting zones, the strip defining a continuous wall with the strip having a lower portion mounted with reaction vessel mounting means, the mounting means capable of mounting multiple reaction vessels on one continuous strip through utilization of flexible leg portions extending from the strip lower portion, the leg portions having generally flat surfaces with each leg having mounted thereon a fin set, the fins projecting perpendicular from each side of the leg portion. In one embodiment of such reaction vessel handling device, the flexible substantially flat leg portions are separated but utilized in pairs along with the fins mounted perpendicularly to the leg portion surfaces for insertion into a reaction vessel well which has a cross section accommodating the leg portions and fins in a sliding contact mode. In another embodiment of such reaction vessel handling device, the reaction vessel handling device is utilized for loading multiple reaction vessels onto the reaction vessel carousel, the semi-rigid strip continuous wall is bendable to form an arc which matches the radius of the carousel. In the latter embodiment, preferably the arc through continuous wall with attached reaction vessels is positioned and snapped into the reaction vessel carousel reception openings and the reaction vessel handling device is removed from the reaction vessels by an upward motion sliding the device mounting means from contact in the open well, the reaction vessel being held in a snapped position on the reaction vessel carousel.

The invention furthermore provides an automated, continuous and random access analytical system apparatus capable of multiple assays of a plurality of liquid samples, comprising (a) a front end carousel assembly inclusive of a sample cup carousel, a reagent pack carousel and a reaction vessel carousel mounted concentrically and serviced by a transfer pipetting means suitable for kidding a reaction vessel, (b) a reaction vessel loading device allowing loading of multiple reaction vessels into the reaction vessel carousel, the device having a continuous strip wall which is flexible for arcing the device and the attached reaction vessels to match the curve radius of the reaction vessel carousel for contact and snapping the reaction vessels into the reaction vessel carousel and withdrawing of the reaction vessel loading device and device mounting means which are slidably inserted into a reaction vessel well having a mateable opening with the mounting means which provides fins and two separate legs which when inserted into the reaction vessel well opening or slightly compressed creating any spring load attachment, (c) a transfer station providing means for transferring a kidded reaction vessel to a process carousel, the process carousel maintained within a control environment, (d) process carousel transfer pipetting means suitable for mixing reagents with the sample in a reaction well of the reaction vessel, (e) means for transferring the resulting reaction mixture to one of at least two assay reader means, (f) means for transferring a reaction vessel from the assay reader to a transfer station, and (g) means associated with said transfer station for removing the disposable reaction vessel from the system.

In another embodiment of the invention, there is provided a method for loading multiple reaction vessels into a reaction carousel of an automated, continuous and random access analytical system capable of simultaneously affecting multiple assays of a plurality of liquid samples, comprising (a) having multiple reaction vessels removably mounted on mounting means of a reaction vessel loading device strip, the strip having a continuous wall and being inflexible along said wall, (b) positioning the reaction vessel loading device strip and removably attached reaction vessels thereon over a reaction vessel carousel, (c) arcing the loading device strip along the strip continuous wall to substantially perform with the radius of curvature of the reaction vessel carousel, (d) inserting the multiple reaction vessels into respective slots on the reaction vessel carousel, (e) snapping the reaction vessels into position into a holding means on the reaction vessel carousel and removing the slidably mounted reaction vessels from the reaction loading device strip by withdrawing the strip from the reaction vessels.

In still another embodiment of the invention, there is provided a method which provides ramping and error detection of stepper motor movements initiated under BIT or chip control, comprising (a) training in advance, sequencing stepper motor controller with ramping and error detection capabilities, (b) commanding the controller by prior training through utilization of built-in chips based on specific imprints, for example when errors are detected, and (c) instructing means for sequencing before movement of the stepper motors. In such embodiment, preferably velocity and acceleration profiles are prefixed in hardware, said profiles being sequences of base velocity, final velocity, acceleration and total number of stepper motor steps, the profile presenting a linear staircase which may be symmetrical or asymmetrical, the maximum number of staircase steps available over an entire profile is defined. In such preferred embodiment, the controller may have a single input BIT to start an action, a single output BIT that will be asserted upon completion of the motor movements following start up BIT functions. Also in such preferred embodiment of the stepper motor controller method with ramping and error detection, the method may further provide a step pulse, direction BIT and power high/low BIT to the motor driver controller means and a home flag output being routed to a controller input for detection and determination of appropriate motor movement.

A further apparatus according to the invention is an automated, continuous and random access analytical system apparatus capable of simultaneously affecting multiple assays of a plurality of liquid samples, comprising (a) a front end carousel assembly inclusive of a sample cup carousel, a reagent pack carousel and a reaction vessel carousel mounted concentrically and serviced by a transfer pipetting means suitable for kidding a reaction vessel, (b) a transfer station providing means for transferring a kidded reaction vessel to a process carousel, the process carousel maintained within a controlled environment, (c) a process carousel, transfer, pipetting means suitable for mixing reagents with a sample in a reaction well of the reaction vessel, (d) means for transferring the resulting reaction mixture to one of at least two assay reader means, (e) means for transferring a reaction vessel from the assay reader to a transfer station, (f) means associated with said transfer station by removing the disposable reaction vessel from the system, and (g) stepper motor means and control means for servicing carousels, pipetters and transfer means by a controller with ramping and error detection means for controlling stepper motor, said controller allowing ramping and error detection with stepper motor movement being initiated under BIT control.

A further method according to the present invention is a method for controlling evaporation and contamination of reagents used in automated diagnostic systems, comprising (a) moving reagent packs containing reagents in closed reagent containers mounted in a reagent pack carousel to an opening and closing station, (b) forcing open the evaporatively closed reagent containers contained in the reagent pack by the opening and closing station, (c) locking in an open position the reagent containers cover and cap which is affixed to the reagent containers, (d) moving the locked open reagent containers from the opening and closing station on the reagent pack carousel to a position wherein the system has access to the reagents, (e) withdrawing reagents from the reagent containers for system use, (f) returning the reagent pack and reagent containers on the reagent pack carousel to the opening and closing station, (g) unlocking the lock open cover and cap of the reagent containers, (h) force closing the cover and cap of the reagent containers of the reagent pack, and (i) removing the reagent pack from the opening and closing station. In one embodiment of such method, the reagent containers having an affixed cover and cap are providing a forced closing of the cover and cap to a soft closing which creates an evaporatively sealed condition, said soft closure of cover and cap provides simpler reopening and repeated closing modes as required by the diagnostic systems. In another embodiment, the forced closing creates an evaporatively sealed condition of a hard closure suitable for handling and shipping. In still another embodiment, the reagent pack contains at least two reagent containers and the reagent containers share a cover and cap unit wherein individual caps are positioned for closure onto the individual containers. In the method for controlling evaporation and contamination of reagents used in automated diagnostic systems, the reagent containers contained in a reagent pack are serviced by individual cover and cap mounted on the individual reagent container. In another embodiment of the method, the reagent container cover and cap provides a cap and seal which provide both soft and hard evaporatively sealed conditions of the reagent container by forming a ring seal around the exposed neck of the reagent container opening. According to a further embodiment of the method, (a) the opening and closing station provides a forced opening of the evaporatively sealed reagent containers by action of opening pins which contact the cover portion of the cover and cap, the contact being made beyond a pivot point on the cover which also serves as a mounting connection to an edge of the reagent container, said contacting by the opening pins with the cover portion beyond a cover and cap pivot provides for opening of the cover and cap, (b) locking the cover cap into an open position by contacting reagent pack closure actuator elements with the raised and open cover and cap edge forcing the cover and cap to a position beyond the vertical and spring locking the cover and cap into an open position, (c) upon return of the open reagent container to the opening and closing station overcoming the spring lock open condition of the cover and cap by forcing the cover and cap back to a near closed position, and (d) moving reagent pack closure actuator elements into contact with partially closed cover and cap to form either a soft or hard closure, both of which are evaporatively sealed conditions.

In addition, the invention provides a method of operating an automated, continuous and random access analytical system capable of simultaneously affecting multiple assays of a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) providing a method for opening and closing evaporatively sealed reagent container by opening a cover including a cap pivotally mounted on the reagent container and locking the cover and cap in an open position and moving the open reagent container to a position for withdrawing reagent from the reagent container, returning the open reagent container to the open and closing station for closing of the reagent container cover and cap forming an evaporatively sealed closure, (d) transferring one or more of said unit dose disposables to a processing work station, (e) mixing an aliquot of said first liquid sample with one or more of said reagents at different times in said reaction vessel to form a first reaction mixture, (f) mixing aliquots of the same or different one or more samples with one or more of said reagents at different times of different reaction vessels to form multiple independently scheduled reaction mixtures, (g) incubating said multiple reaction mixtures simultaneously and independently, (h) performing more than one scheduled assay on said reaction mixtures in any order in which more than scheduled assays are presented, and (i) analyzing said incubated reaction mixtures independently and individually by at least two assay procedures.

A further apparatus according to the invention is an apparatus for opening and closing reagent containers contained within a reagent pack having cover and cap means for control of evaporation and contamination of reagents used in automated diagnostic systems, comprising (a) reagent containers having mounted thereon a pivotal closure and cap means, the pivot being the mounting point on an edge of the reagent container, (b) an opening and closing station which provides reagent pack opening pins for contacting the reagent pack cover means portion which extends beyond the pivot and the reagent pack, (c) spring means within the cover and cap means for locking a cover and cap in an open position, (d) reagent pack means containing at least two reagent containers mounted within a reagent pack carousel for movement to and from the opening and closing station, and (e) reagent pack closure actuator means for unlocking the locked open cover and cap means and for force closing of the cover cap means onto the reagent packs renewing the evaporatively sealed closure. In one embodiment of such apparatus, the cover means has a cap portion on one side of a pivot of the cover means, the cover means being mounted to an edge portion of the reagent container top edge and the cover means extending substantially beyond the pivot point; the cap means comprised of a cap member which extends from the cover means for insertion into the reagent container opening neck and a spaced apart projecting ring member which closes around the reagent container opening neck. In the apparatus, the reagent pack closure actuator means may be comprised of engageable flat, circular surfaces for closing the reagent packs by contacting partially closed cover and cap means positioned for closure. In the apparatus, the reagent pack opening pins may be engageable for contacting the cover and cap means at a position beyond the reagent pack cover and cap means mounting pivot from the cap means, the pins forcing the cover and cap means to a substantially vertical position by extension of the pins. In a preferred embodiment of the apparatus, the opening and closing station has a housing and a drive means thereon, the housing has a mounting means for fixing the opening and closing station to a position above a reagent carousel which moves the reagent containers to the opening and closing station for either opening the cover and cap means or closing the cover and cap means, the reagent pack closure actuator means and the reagent pack opening pins are engageable to contact the cover and cap means at various levels for opening the cover and cap means, locking the cover and cap means in an open position and unlocking the open cover and cap means to a partially closed position and force closing of the cover and cap means; reagent container cover and cap means providing a unit cover means supporting multiple cap means, the reagent pack opening pins and closure actuator means are operated in unison when activated for opening and closing of reagent container cover and cap means.

The invention also provides a cartridge feeder apparatus for automated diagnostic systems, comprising (a) a cartridge hopper means for receiving, storing and feeding cartridges to a cartridge orientation mechanism, (b) the cartridge orientation mechanism comprised of two opposed engageable orientation means for engaging and disengaging a cartridge at each end of the cartridge, and (c) the cartridge having a funnel opening at a first end and a generally flat bottom at a second end. In a first embodiment of such apparatus, the engaging orientation means have the same engaging surfaces including a rounded or blunt protrusion aligned on the axis of the cartridge making a flat surface contact with the cartridge bottom or being matable with the funnel opening of the cartridge, the engaging means having arms or ring members spaced from the engaging means protrusion which accommodate the exterior walls of the cartridge in approximate position overlap on the engaging member which engages the funnel opening end of the cartridge. In such first embodiment, preferably the orientation engaging members are capable of orientating cartridges received in either horizontal orientation because of the same configuration of the opposed engaging members. In a second embodiment of the cartridge feeder apparatus, the cartridge hopper means has parallel walls for defining a somewhat flat hopper means to accommodate cartridges end to end, the hopper means having edge walls which slope inwardly at a lower portion toward a hopper opening capable of releasing one cartridge at a time to the orientation mechanism. In such second embodiment, preferably the hopper means for receiving, storing and feeding cartridges to a cartridge orientation mechanism and a cartridge wheel has an enlarged upper portion which is open for receiving a cartridge filled carton for unloading numerous cartridges into the hopper enlarged open upper end; the hopper having a sloped lower portion focusing on a cartridge release opening. In a third embodiment of the cartridge feeder apparatus, the hopper means has two spaced apart roller pins mounted in an upper portion of the hopper means for accommodating the cartridge loaded cartons with a central break apart element which allows the cartons once the break apart occurs, to ride along the spaced apart roller pins for discharging the numerous cartridges contained in the carton. In such third embodiment of the cartridge feeder apparatus, preferably the roller pins promote carton break open along a predefined break open line generally in the center portion of the carton for self releasing of cartridges from the carton as the carton rides on the roller pins, and still more preferably the carton has a tear strip opening means along a center portion of the carton leaving the carton intact along one edge, opened or partially opened carton opening fully on the roller pins for unloading cartridges. In a fourth embodiment of the cartridge feeder apparatus, preferably the hopper means is detachable and capable of stand alone during cartridge loading, the stand alone hopper having a raised central region for receiving cartons containing cartridges which break open and ride on the roller pins for unloading the cartridges, the hopper further comprised of a transparent mid-region section with indication markers thereon to indicate cartridge numbers remaining in the hopper based on cartridge level within the hopper.

The present invention also provides a method for feeding multiple cartridges to a cartridge feeder apparatus useful in automated diagnostic systems, comprising feeding multiple cartridges from a self-opening carton into a cartridge hopper having capacity for receiving, storing and feeding cartridges one by one to a cartridge orientation mechanism, orientating the individual cartridges to a bottom down position before feeding the orientated cartridge to a cartridge wheel for processing; the cartridge orientation mechanism functioning to orientate the individual cartridges in a bottom down orientation independently of the horizontal orientating cartridges as received from the hopper.

Furthermore, the present invention provides a data acquisition system comprising electronic firm wear utilizing digital signal processing and single chip analog to digital converter to improve noise performance, wherein said system is combined with digital micro-controller means to simplify communication and operation thereof. In a first embodiment of such data acquisition system, external FPIA optics analog signals are provided to a DSP A/D chip which sends serial bus signals to an optic signal processor 8-bit digital microcontroller which is in communication with a computer; wherein said digital micro-controller is in communication through serial bus to said FPIA optics through high voltage power PMT supply, FPIA tungsten lamp power supply, and in electronic communication with FPIA optics; wherein external MEIA optics analog signals are provided to a second DSP A/D chip which also sends serial bus signal to said optic signal processor 8-bit digital microcontroller; and wherein said digital micro-controller presents signal to MEIA optics which is in communication through serial bus to a high voltage PMT power supply mercury lamp power supply, said high voltage PMT power supply MEIA mercury lamp power supply is in electronic communication with said MEIA optics. According to a second embodiment, said system functions within an FPIA subsystem to acquire and convert to digital format, wherein PMT high voltage sample fluorescence intensity is under excitation by vertical and horizontal polarized light and provides liquid crystal control. According to a third embodiment, said system functions within an MEIA reader subsystem to acquire and convert to digital format, mercury lamp intensity level and sample fluorescence intensity level, and PMT high voltage.

In addition, the present invention provides a method of operating an automated, continuous and random access analytical system capable of simultaneously effecting multiple assays of a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) transferring one or more of said unit dose disposables to a processing workstation, (d) mixing an aliquot of said first liquid sample with one or more of said reagents at different times in said reaction vessel to form a first reaction mixture, (e) mixing aliquots of the same or different one or more samples with one or more of said reagents at different times in different reaction vessels to form multiple, independently scheduled reaction mixtures, (f) incubating said multiple reaction mixtures simultaneously and independently, (g) performing more than one scheduled assay on said reaction mixtures in any order in which more than scheduled assays are presented, and (h) analyzing said incubated reaction mixtures independently and individually by at least two assay procedures; wherein said analytical system comprises a data acquisition system comprising electronic firm wear utilizing digital signal processing and single chip analog to digital converter to improve noise performance, wherein said system is combined with digital micro-controller means to simplify communication and operation thereof. According to a first embodiment of such method, external FPIA optics analog signals are provided to a DSP A/D chip which sends serial bus signals to an optic signal processor 8-bit digital microcontroller which is in communication with a computer; wherein said digital micro-controller is in communication through serial bus to said FPIA optics through high voltage power PMT supply, FPIA tungsten lamp power supply, and in electronic communication with FPIA optics; wherein external MEIA optics analog signals are provided to a second DSP A/D chip which also sends serial bus signal to said optic signal processor 8-bit digital microcontroller; and wherein said digital micro-controller presents signal to MEIA optics which is in communication through serial bus to a high voltage PMT power supply mercury lamp power supply, said high voltage PMT power supply MEIA mercury lamp power supply is in electronic communication with said MEIA optics. In said first embodiment of the method, preferably the system is capable of allowing special priority handling through stat procedure scheduling of specific samples, and wherein said stat procedure scheduling interrupts prior scheduling, allowing the system to finish preparing assays on a current sample and then prepare to perform an assay on the sample through a modification of the scheduling. According to a second embodiment of the method, said system functions within an FPIA subsystem to acquire and convert to digital format, wherein PMT high voltage sample fluorescence intensity is under excitation by vertical and horizontal polarized light and provides liquid crystal control. According to such second embodiment, assay throughout may be increased in the system. Also in the second embodiment of the method, operating the automated continuous and random access analytical system may include kitting a unit dose disposable by separately transferring assay samples and reagents to a reaction vessel without initiation of an assay reaction sequence. Also, according to a preferred method of the second embodiment, scheduling for performing assays maximizes the number of assays the system is capable of processing per unit time by allowing sufficient time gaps between assay protocol steps to enable other assay protocol steps to be performed within such time gaps. According to a third embodiment of the method, said system functions within an MEIA reader subsystem to acquire and convert to digital format, mercury lamp intensity level and sample fluorescence intensity level, and PMT high voltage. According to a fourth embodiment of the method, at least two different assays are scheduled to be performed on the system for a plurality of liquid samples, said method provides scheduling of said assays in advance of performance thereof, each assay test definition containing several timing parameters with each activity of the assay test containing time values which scheduling uses to determine which resources of the system and activity required by each of said assays and the time period that said resources need. According to a fifth embodiment of the method, the scheduling process includes scheduling of each activity to be performed in an assay before the assay is kitted, and scheduling performance of each assay activity prior to its originally scheduled execution time, thus minimizing resource idle time, the calibration procedures scheduling being preferably scheduled as a stat procedure. In the general method, the assay performed on said reaction mixture in said reaction vessel may be a homogeneous assay or a heterogeneous assay. In the general method, preferably at least two assays are immunoassays such as immunoassays comprised of MEIA and FPIA assays. In the method, the analyzing step may include optically monitoring said reaction mixtures. The reaction mixtures may be monitored by turbidimetric, colorimetric, fluorometric or luminescent means. Furthermore, partial initiation of an assay reaction sequence may be achieved simultaneously with the creating of the unit dose disposable. Also, in the method, the system may achieve simultaneous creation of unit dose disposables, transferring of a unit dose disposable reaction vessel and mixing of a reaction mixture while incubating multiple reaction mixtures and performing at least one scheduled assay and analysis simultaneously.

A further apparatus of the invention is an automated, continuous and random access analytical system apparatus capable of simultaneously effecting multiple assays of a plurality of liquid samples, comprising (a) a front end carousel assembly inclusive of a sample cup carousel, a reagent pack carousel and a reaction vessel carousel mounted concentrically and serviced by a transfer pipetting means suitable for kitting a reaction vessel, (b) a transfer station providing means for transferring a kitted reaction vessel to a process carousel, the process carousel maintained within a controlled environment, (c) a process carousel transfer pipetting means suitable for mixing reagents with the sample in a reaction well of the reaction vessel, (d) means for transferring the resulting reaction mixture to one of at least two assay reader means, (e) means for transferring a reaction vessels from the assay reader to a transfer station, and (f) means associated with said transfer station for removing the disposable reaction vessel from the system, wherein said analytical system comprises a data acquisition system comprising electronic firm wear utilizing digital signal processing and single chip analog to digital converter to improve noise performance, wherein said system is combined with digital micro-controller means to simplify communication and operation thereof. In a first embodiment of the apparatus, external FPIA optics analog signals may be provided to a DSP A/D chip which sends serial bus signals to an optic signal processor 8-bit digital microcontroller which is in communication with a computer; wherein said digital micro-controller is in communication through serial bus to said FPIA optics through high voltage power PMT supply, FPIA tungsten lamp power supply, and in electronic communication with FPIA optics; wherein external MEIA optics analog signals are provided to a second DSP A/D chip which also sends serial bus signal to said optic signal processor 8-bit digital microcontroller; and wherein said digital micro-controller presents signal to MEIA optics which is in communication through serial bus to a high voltage PMT power supply mercury lamp power supply, said high voltage PMT power supply MEIA mercury lamp power supply is in electronic communication with said MEIA optics. In a second embodiment of the apparatus, said system functions within an FPIA subsystem to acquire and convert to digital format, wherein PMT high voltage sample fluorescence intensity is under excitation by vertical and horizontal polarized light and provides liquid crystal control. Alternatively, said system may function within an MEIA reader subsystem to acquire and convert to digital format, mercury lamp intensity level and sample fluorescence intensity level, and PMT high voltage. In a further embodiment, one assay reader means is comprised of a cartridge wheel carousel containing multiple disposable cartridges, the apparatus providing means for supplying said cartridges to the cartridge wheel carousel and disposing of the cartridges from the cartridge wheel carousel. The assay reader means of the apparatus may include means for optically monitoring said assay reaction. The assay reader means may provide calibration and reader means as well as recording means for the resulting assay data. The transfer station transfer means may be comprised of a carousel rotatable about an axis, an arm with a pick for mating with a reaction vessel transfer projection means and means for pulling the reaction vessel from the front end carousel, rotating and placing the reaction vessel onto the process carousel through rotation and rack and pinion movement of the pick arm. The sample handling means and reagent handling means may include means for identifying said liquid samples and liquid reagents from coded information associated with the sample cups and the reagent packs. The apparatus may further include means to store the output readings of the assay reader. The apparatus may further include means to calculate the concentration of an analyte from the output readings of said assay reader. The reaction vessel may contain a reaction cuvette having a physical characteristic of low birefringence through an optical read region.

The invention also provides a reaction vessel loading device for loading a plurality of reaction vessels onto a reaction vessel carousel, said reaction vessel loading device comprising a semi-rigid planar cover having an upper planar surface with spaced apart vessel insert depressions which are insertable into reaction vessel openings, said depressions providing a projection for at least one reaction vessel well and cuvette opening, said projections being aligned to fit into said well and cuvette opening for affixing reaction vessels onto the reaction vessel loading device, the mounted reaction vessels on the reaction vessel loading device being appropriately spaced and aligned for insertion loading into a reaction vessel carousel, the reaction vessel loading device having a length with edges defining a portion of an arc having the same curvature as the reaction vessel carousel. In one embodiment of the reaction vessel loading device, the reaction vessel loading device protrusions are fitted into the reaction vessel cuvette on one end of the reaction vessel and on a reaction vessel well at an opposite end of the reaction vessel. In a second embodiment of the reaction vessel loading device, (i) the reaction vessel planar surface, (ii) reaction vessel insertion depressions, (iii) reaction vessel well protrusions, and (iv) the reaction cuvette protrusions, are spaced apart and aligned for insertably receiving reaction vessels whereby once the reaction vessels are mounted thereon, the reaction vessels are aligned for drop-in mounting in the reaction vessel carousel reception openings. In a third embodiment of the reaction vessel loading device, the semi-rigid planar cover comprises a flexible plastic material and the loader is a molded plastic. According to a fourth embodiment of the reaction vessel loading device, the planar surface terminates in a continuous elevated rim substantially perpendicular to the planar surface, said rim terminating in a planar surface segment substantially parallel to the planar surface of the reaction vessel loading device. In such fourth embodiment, preferably the loader rim has elevated segments on opposing ends of the loader for handling purposes.

Furthermore, according to the present invention, there is provided a reaction vessel loading device for use with an automated, continuous and random access analytical system apparatus capable of multiple assays of a plurality of liquid samples, wherein said system comprises a front end carousel assembly inclusive of a sample cup carousel, a reagent pack carousel, and a reaction vessel carousel mounted concentrically and serviced by a transfer pipetting means suitable for kitting a reaction vessel, said reaction vessel loading device comprising a semi-rigid planar cover having an upper planar surface with spaced apart vessel insert depressions which are insertable into reaction vessel openings, said depressions providing a projection for at least one reaction vessel well and reaction vessel for receiving a reaction cuvette, cuvette opening, said projections being aligned to fit into said well or cuvette opening for affixing reaction vessels onto the reaction vessel loading device, where the mounted reaction vessels on the reaction vessel loading device being appropriately spaced and aligned for insertion loading into a reaction vessel carousel, said planar surface terminating in a continuous elevated rim substantially perpendicular to the planar surface, said rim terminating in a planar surface segment being substantially parallel to the planar surface of the reaction vessel loading device, and the reaction vessel loading device having a length with edges defining a portion of an arc having the same curvature as the reaction vessel carousel. In a first embodiment of this reaction vessel loading device, the reaction vessel loading device is utilized for loading a plurality of reaction vessels onto the reaction vessel carousel, the reaction vessel loading device protrusions are fitted into the reaction vessel cuvette on one end of the reaction vessel and on a reaction vessel well at an opposite end of the reaction vessel. In a second embodiment of this reaction vessel loading device, (i) the reaction vessel planar surface, (ii) the reaction vessel insertion depressions, (iii) the reaction vessel well protrusions, and (iv) the reaction cuvette protrusions are spaced apart and aligned for insertable receiving reaction vessels whereby once the reaction vessels are mounted thereon, the reaction vessels are aligned for drop-in mounting in the reaction vessel carousel reception openings. In a third embodiment of this reaction vessel loading device, the loader rim has elevated segments on opposing ends of the loader for handling purposes. In a fourth embodiment of this reaction vessel loading device, the loading device with reaction vessels mounted thereto is positioned and snapped into the reaction vessel carousel reception openings and the reaction vessel loading device is removed from the reaction vessels by an upward motion to thereby slide the loader mounting means from contact with the open well and cuvette, whereby the reaction vessels are held in a snapped position on the reaction vessel carousel.

Another apparatus according to the invention is an automated, continuous and random access analytical system apparatus capable of multiple assays of a plurality of liquid samples comprising (a) a front end carousel assembly inclusive of a sample cup carousel, a reagent pack carousel and a reaction vessel carousel mounted concentrically and serviced by a transfer pipetting means suitable for kitting a reaction vessel, (b) a reaction vessel loading device, said reaction vessel loading device for loading a plurality of reaction vessels onto a reaction vessel carousel comprising a semi-rigid planar cover having an upper planar surface with spaced apart vessel insert depressions which are insertable into reaction vessel openings, said depressions providing a projection for at least one reaction vessel well and cuvette opening, said projections being aligned to fit into said well and cuvette opening for affixing reaction vessels onto the reaction vessel loading device, the mounted reaction vessels on the reaction vessel loading device being appropriately spaced and aligned for insertion loading into a reaction vessel carousel, the reaction vessel loading device having a length with edges defining a portion of an arc having the same curvature as the reaction vessel carousel, (c) a transfer station providing means for transferring a kitted reaction vessel to a process carousel, the process carousel maintained within a control environment, (d) process carousel transfer pipetting means suitable for mixing reagents with the sample in a reaction well of the reaction vessel, (e) means for transferring the resulting reaction mixture to one of at least two assay reader means, (f) means for transferring a reaction vessel from the assay reader to a transfer station, and (g) means associated with said transfer station for removing the disposable reaction vessel from the system.

A further method according to the invention is a method for loading a plurality of reaction vessels into a reaction vessel carousel of an automated, continuous and random access analytical system capable of simultaneously effecting multiple assays of a plurality of liquid samples, said method comprising the steps of (a) providing multiple reaction vessels removably mounted on mounting means of a reaction vessel loading device, the loader having a continuous planar surfaced with spaced apart aligned reaction vessel opening depressions wherein said depressions have protrusions suitable for fitting into the opening of at least one reaction vessel well and a reaction vessel cuvette, the reaction vessel loading device having an arc configuration of the reaction vessel depressions mateable with the reaction vessel carousel and reaction vessel receiving means, (b) positioning the reaction vessel loading device and removably mounted reaction vessels thereon over a reaction vessel carousel, (c) mating the arced reaction vessel loading device and mounted reaction vessels with the matching radius of curvature of the reaction vessel carousel, (d) inserting the plurality of reaction vessels into respective slots in the reaction vessel carousel, (e) snapping the reaction vessels into position into holding means on the reaction vessel carousel, and (f) removing the slideably mounted reaction vessels from the reaction vessel loading device by withdrawing the loader from the reaction vessels. In such method, the holding means of the reaction vessel carousel may present a sufficient holding force to overcome force required to overcome slidable disengagement of the loader from the reaction vessels.

The present invention also provides a bubble flushing aspirating and dispensing apparatus having precision and volumetric accuracy, said apparatus comprising (a) a reciprocating piston within a bore, the bore and piston having seal means at a first end and a closed end at a second end, (b) an annulus defined between the piston, the bore wall and the seal means, (c) valved fluid inlet and outlet means in communication with the annulus and bore, (d) a valved fluid source in communication with the inlet means, (e) a fluid conduit in communication with the outlet means and an open ended release tip, and (f) a drive means for reciprocating the piston within the bore. In a first embodiment of such apparatus, the fluid inlet and fluid outlet means are located proximal to the seal means and the fluid inlet and fluid outlet means are positioned about 180° apart from the seal means. In a second embodiment of the apparatus, the reciprocating piston comprises a piston head having substantially the same geometry as the inside configuration of the bore closed end. In such second embodiment, the piston head may be dome-shaped. In a third embodiment of the apparatus, the reciprocating piston disrupts any bubbles in the bore end when positioned substantially close to a contacting position with the bore end when in its full extension position. In a fourth embodiment of the apparatus, the reciprocating piston is positioned with the piston head being flush with the seal and positioned slightly beyond the fluid entry port and the fluid exit port when in its full outward extension position.

According to the invention, there is also provided a method for flushing bubbles from a fluidics system, said method comprising the steps of (a) introducing fluid into an annulus defined by a piston and bore wall, the annulus closed at a first end of the bore by seal means between the bore wall and the piston and at a second end of the bore by a closed bore end, (b) introducing fluid into the bore when the piston is withdrawn beyond fluid inlet and outlet means, (c) flowing the fluid through the bore end and annulus and around the sides of the piston and out through a fluid outlet, (d) creating a cross flow pattern proximal to the seal area across the end of the piston in one position and around the piston when the piston is moving in or out of the bore, and (e) reciprocating the piston at least once to flush bubbles from the fluidics system. In a preferred embodiment, creating the cross flow pattern proximal to the seal area across the end of the piston flushes bubbles from the end of the piston when the reciprocating piston is in a full outward extension position. In another embodiment, flowing the fluids around the sides of the piston and recapturing the fluid at a fluid outlet while the piston is moving in or out of the bore, flowing the fluid around the sides of the piston from the which is located about 180° from the fluid inlet flushes bubbles from and recapturing the fluid at a fluid outlet which is located about 180° from the fluid inlet, flushes bubbles from the fluidics system. Bubbles near or on the closed bore end may be disrupted by positioning the end of the piston sufficiently close contacting the inside bore end. Bubbles may be flushed from the fluidics system by reciprocating the piston for multiple reciprocal cycles between each aspirating and dispensing functions. Bubbles may be flushed from the fluidics system by reciprocating the piston for multiple reciprocation cycles, said cycles automatically controlled at periodic multiples of the aspirating and dispensing functions. According to one embodiment of the method, the inlet is closed and the piston is reciprocated at variable speeds. In the latter embodiment, the piston may be withdrawn at a reduced speed of about ½ or less of the full piston travel distance to thereby prevent increased vacuum and creation of bubbles. Alternatively, the piston may be reciprocated at full speed in order to dislodge formed bubbles in the end portion of the bore.

Another method according to the invention is a method for dispensing liquids in a fluidics system, said method comprising the steps of (a) introducing fluid into an annulus defined by a piston and bore wall, the annulus closed at a first end of the bore by seal means between the bore wall and the piston and at a second end of the more by a closed bore end and introducing fluid into the bore when the piston is withdrawn beyond fluid inlet and outlet, (b) flowing the fluid through the bore end and annulus and around the sides of the piston and out through a fluid outlet, (c) creating a cross flow pattern proximal to the seal area across the end of the piston in one position and around the piston when the piston is moving in or out of the bore, (d) reciprocating the piston to dispense, and (e) flushing bubbles from the fluidic system through reciprocating of the piston at least through one complete reciprocation cycle.

In addition, the invention provides an automated, continuous and random access analytical system apparatus capable of simultaneously effecting multiple assays of a plurality of liquid samples, comprising (a) a front end carousel assembly inclusive of a sample cup carousel, a reagent pack carousel and a reaction vessel carousel mounted concentrically and serviced by a transfer pipetting means suitable for kitting a reaction vessel, (b) a transfer station providing means for transferring a kitted reaction vessel to a process carousel, the process carousel maintained within a controlled environment, (c) a bubble flushing aspirating and dispensing apparatus means having a reciprocating piston with a bore, the bore and piston having seal means at a first end and a closed end at a second end, an annulus defined between the piston, the bore wall and the seal means, valved fluid inlet and outlet means in communication with the annulus and bore, a valved fluid source in communication with the inlet means, a fluid conduit in communication with the outlet means and an open ended release tip, and a drive means for reciprocating the piston within the bore, (d) a process carousel transfer pipetting means suitable for mixing reagents with the sample in a reaction well of the reaction vessel (e) means for transferring the resulting reaction mixture to one of at least two assay reader means, (f) means for transferring reaction vessels from the assay reader to a transfer station, and (g) means associated with said transfer station for removing the disposable reaction vessel from the system.

According to the present invention, there is also provided a method of operating an automated, continuous and random access analytical system capable of simultaneously effecting multiple assays of a plurality liquid samples, comprising (a) introducing sample cups, reagent packs, and reaction vessels for performing said assays onto concentric carousels of a front end carousel, the reaction vessels being introduced to an outer carousel, (b) identifying the reagent packs and sample cups, (c) scheduling the assays, (d) aligning the sample cups and reagent packs with a reaction vessel at a kitting station by rotating the respective carousels, (e) flushing bubbles from a fluidics system by introducing fluid into an annulus defined by a piston and bore wall, the annulus closed at a first end of the bore by seal means between the bore wall and the piston and at a second end of the bore by a closed bore end and introducing fluid into the bore when the piston is withdrawn beyond fluid inlet and outlet means, flowing the fluid through the bore end and annulus and around the sides of the piston and out through a fluid outlet, creating a cross flow pattern proximal to the seal area across the end of the piston in one position and around the piston when the piston is moving in or out of the bore, and reciprocating the piston at least once to flush bubbles from the fluidics system, (f) kitting a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent pack, (g) transferring the kitted reaction vessel to a process carousel which is maintained under controlled environment conditions (h) pipetting the sample and various reagents into a reaction well of the reaction vessel, the amounts of reagent, sequencing of transfer and time spacing there between being predetermined by assay scheduling (i) incubating the pipetted sample and reagent mix, (j) identifying and transferring the incubated mixture in the reaction well to one of at least two assay analytical stations, (k) performing an analysis by reading the prepared reaction mixture and calibrating the reading, and (1) recording the resulting assay reading analysis.

A still further method of the present invention is a method of operating an automated, continuous and random access analytical system capable of simultaneously effecting multiple assays of a plurality liquid samples, comprising (a) introducing sample cups, reagent packs, and reaction vessels for performing said assays onto concentric carousels of a front end carousel, the reaction vessels being introduced to an outer carousel, (b) identifying the reagent packs and sample cups, (c) scheduling the assays, (d) aligning the sample cups and-reagent packs with a reaction vessel at a kitting station by rotating the respective carousels, (e) dispensing liquids in a fluidics system by introducing fluid into an annulus defined by a piston and bore wall, the annulus closed at a first end of the bore by seal means between the bore wall and the piston and at a second end of the more by a closed bore end and introducing fluid into the bore when the piston is withdrawn beyond fluid inlet and outlet, flowing the fluid through the bore end and annulus and around the sides of the piston and out through a fluid outlet, creating a cross flow pattern approximate to the seal area across the end of the piston in one position and around the piston when the piston is moving in or out of the bore, reciprocating the piston to dispense, and flushing bubbles from the fluidic system through reciprocating of the piston at least through one complete reciprocation cycle, (f) kitting a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent pack, (g) transferring the kitted reaction vessel to a process carousel which is maintained under controlled environment conditions, (h) pipetting the sample and various reagents into a reaction well of the reaction vessel, the amounts of reagent, sequencing of transfer and time spacing there between being predetermined by assay scheduling, (i) incubating the pipetted sample and reagent mix, (j) identifying and transferring the incubated mixture in the reaction well to one of at least two assay analytical stations, (k) performing an analysis by reading the prepared reaction mixture and calibrating the reading, and (1) recording the resulting assay reading analysis.

In accordance with the embodiments of the present invention, the automated immunoassay analytical system provides apparatus, software, hardware and process technology for performing a multitude of assays continuously and with random access being available to the operator. The utilization of carousel pipettor technology for kitting and pipetting operations at either the main carousel or the process carousel, depending on the scheduled test, provides scheduling flexibilities heretofore unachievable. The inventive system allows for a commonality of kitting and pipetting for either immuno precipitation or competitive immunoassay technologies utilizing a common main carousel, transfer station, first kitting and pipetting probe and process carousel as well as a second pipetting probe before separating into respective apparatus and process requirements. Also shared is the commonality of cabinetry disposal and supply materials as well as a common computer network for scheduling, testing, kitting and pipetting.

It will be seen that multiple assays can be performed with a minimum of operator input or handling on the system and the system can be utilized for other processes and assays which have not been directly discussed but will be readily apparent to one practiced in the art in view of the above invention disclosure and the claims. It will also be appreciated that although particular embodiments of the present invention have been disclosed, various changes and adaptations to the apparatus and methods can be made without departing from the teachings of the specification and scope of the invention as set out in the following claims.

## Claims

1. A bubble flushing aspirating and dispensing syringe having precision and volumetric accuracy, comprising:
a reciprocating piston within a bore, the bore having seal means at a first end and a closed end at a second end;
an annulus defined between the piston, the bore wall and seal means;
fluid inlet and fluid outlet means in communication with the annulus and bore, the fluid inlet and fluid outlet means being located between the seal means and the bore closed end;
a fluid source in communication with the inlet means;
a fluid conduit in communication with the outlet means and an open ended release tip; and
a drive means for reciprocating the piston within the bore.

2. The bubble flushing aspirating and dispensing syringe according to Claim 1 wherein the reciprocating piston in its full outward extension position is positioned with the piston head being flush with the seal and positioned between the fluid entry port and the fluid exit port.

3. A bubble flushing aspirating and dispensing syringe having volumetric accuracy, comprising:
a syringe frame;
a linear actuator mounted to the frame;
an actuator motor for spinning a nut means in communication with a lead screw, the lead screw being affixed to the coupler which has a bearing on one side;
the bearing mounted in a groove in the frame;
the coupler is rotationally constrained by the bearing, the coupler reciprocates when the linear actuator motor spins the nut, and the piston which is affixed to the coupler provides reciprocating motion of the piston through a seal means and a bore;
the reciprocating piston mounted within the bore having a closed end spaced from the seal means;
an annulus defined by the piston and bore;
fluid inlet and fluid outlet means in communication with the annulus and bore, the fluid inlet and fluid outlet means located between the seal means and the bore closed end;
a fluid source in communication with the inlet means; and
a fluid conduit in communication with the fluid outlet means and an open ended release tip.

4. The bubble flushing aspirating and dispensing syringe according to Claim 1 or 3 wherein the fluid inlet and fluid outlet means are positioned approximal to the seal means and the fluid inlet and fluid outlet means are positioned at about 180° apart.

5. The bubble flushing aspirating and dispensing syringe according to Claim 1 or 3 wherein the reciprocating piston has a piston head of a similar geometry as the inside configuration of the bore closed end.

6. The bubble flushing aspirating and dispensing syringe according to Claim 5 wherein the piston head is domed-shaped.

7. The bubble flushing aspirating and dispensing syringe according to Claim 1 or 3 wherein the reciprocating piston in its full inward extension position is positioned sufficiently close to touching the inside bore end to disrupt any bubbles in the bore end.

8. The bubble flushing aspirating and dispensing syringe according to Claim 3 wherein the reciprocating piston has a piston head of a similar geometry as the inside configuration of the bore closed end and the reciprocating piston in its full outward extension position from the bore end is positioned with the piston head being flush with the seal and positioned between the fluid inlet and fluid outlet means.

9. A bubble flushing precision positive displacement pump syringe having precision and volumetric accuracy, comprising:
a reciprocating piston within a bore, the bore having seal means at a first end and a closed end at a second end;
an annulus defined between the piston and the bore wall;
valved fluid inlet and valved fluid outlet means in communication with the annulus and bore, the valved fluid inlet and valved fluid outlet means being located between the seal means and the bore closed end;
a fluid source in communication with the inlet means through a valve means;
a fluid conduit in communication with the outlet means and an outlet valve means; and
a drive means for reciprocating the pump within the bore.

10. A bubble flushing aspirating and dispensing apparatus having precision and volumetric accuracy, said apparatus comprising:
(a) a reciprocating piston within a bore, the bore and piston having seal means at a first end and a closed end at a second end;
(b) an annulus defined between the piston, the bore wall and the seal means;
(c) valved fluid inlet and outlet means in communication with the annulus and bore;
(d) a valved fluid source in communication with the inlet means;
(e) a fluid conduit in communication with the outlet means and an open ended release tip; and
(f) a drive means for reciprocating the piston within the bore.

11. An automated, continuous and random access analytical system apparatus capable of simultaneously effecting multiple assays of a plurality of liquid samples, comprising:
(a) a front end carousel assembly inclusive of a sample cup carousel, a reagent pack carousel and a reaction vessel carousel mounted concentrically and serviced by a transfer pipetting means suitable for kitting a reaction vessel;
(b) a transfer station providing means for transferring a kitted reaction vessel to a process carousel, the process carousel maintained within a controlled environment;
(c) a bubble flushing aspirating and dispensing apparatus means having a reciprocating piston with a bore, the bore and piston having seal means at a first end and a closed end at a second end, an annulus defined between the piston, the bore wall and the seal means, valved fluid inlet and outlet means in communication with the annulus and bore, a valved fluid source in communication with the inlet means, a fluid conduit in communication with the outlet means and an open ended release tip, and a drive means for reciprocating the piston within the bore;
(d) a process carousel transfer pipetting means suitable for mixing reagents with the sample in a reaction well of the reaction vessel;
(e) means for transferring the resulting reaction mixture to one of at least two assay reader means;
(f) means for transferring reaction vessels from the assay reader to a transfer station; and
(g) means associated with said transfer station for removing the disposable reaction vessel from the system.

12. A method for flushing bubbles from an aspirating and dispensing syringe and a fluidic system utilizing such syringe, which method comprises the steps of:
introducing fluid into an annulus defined by a piston and bore wall, the annulus closed at a first end of the bore by seal means between the bore wall and the piston and at a second end of the bore by a closed bore end;
flowing the fluid through the annulus around the sides of the piston and out through a fluid outlet;
creating a cross flow pattern approximate to the seal area across the end of the piston in one position and around the piston when the piston is moving in or out of the bore; reciprocating the piston; and
flushing bubbles from the fluidic system through reciprocating of the piston at least through one complete reciprocation cycle.

13. The method for flushing bubbles from an aspirating and dispensing syringe according to Claim 12 wherein creating the cross flow pattern approximate to the seal area across the end of the piston when the reciprocating piston is in a full outward extension position with the piston head being flush with the seal and around the piston when the piston is moving in or out of the bore, flowing the fluid around the sides of the piston and recapturing the fluid at a fluid outlet which is located approximately 180° from the fluid inlet.

14. The method for flushing bubbles from an aspirating and dispensing syringe according to Claim 12 wherein bubbles near or on the closed bore end are disrupted by the reciprocating piston in its full inward extension position by positioning the end of the piston sufficiently close to touching the inside bore end.

15. The method for flushing bubbles from an aspirating and dispensing syringe according to Claim 12 comprising flushing of the bubbles from the fluidics system through reciprocating of the piston for multiple reciprocation cycles between each aspirating and dispensing function or automatically controlled at periodic multiples of the aspirating and dispensing function.

16. A method for flushing bubbles from a fluidics system, said method comprising the steps of:
(a) introducing fluid into an annulus defined by a piston and bore wall, the annulus closed at a first end of the bore by seal means between the bore wall and the piston and at a second end of the bore by a closed bore end; and
(b) introducing fluid into the bore when the piston is withdrawn beyond fluid inlet and outlet means;
(c) flowing the fluid through the bore end and annulus and around the sides of the piston and out through a fluid outlet;
(d) creating a cross flow pattern proximal to the seal area across the end of the piston in one position and around the piston when the piston is moving in or out of the bore; and
(e) reciprocating the piston at least once to flush bubbles from the fluidics system.

17. The method according to Claim 16, wherein creating the cross flow pattern proximal to the seal area across the end of the piston flushes bubbles from the end of the piston when the reciprocating piston is in a full outward extension position.

18. The method according to Claim 16, wherein flowing the fluids around the sides of the piston and recapturing the fluid at a fluid outlet while the piston is moving in or out of the bore, flowing the fluid around the sides of the piston from the which is located about 180° from the fluid inlet flushes bubbles from and recapturing the fluid at a fluid outlet which is located about 180° from the fluid inlet, flushes bubbles from the fluidics system.

19. The method according to Claim 16, wherein bubbles near or on the closed bore end are disrupted by positioning the end of the piston sufficiently close contacting the inside bore end.

20. The method according to Claim 16, wherein bubbles are flushed from the fluidics system by reciprocating the piston for multiple reciprocal cycles between each aspirating and dispensing functions.

21. The method according to Claim 16, wherein bubbles are flushed from the fluidics system by reciprocating the piston for multiple reciprocation cycles, said cycles automatically controlled at periodic multiples of the aspirating and dispensing functions.

22. The method according to Claim 16, wherein the inlet is closed and the piston is reciprocated at variable speeds.

23. The method according to Claim 22, wherein the piston is withdrawn at a reduced speed of about 1/2 or less of the full piston travel distance to thereby prevent increased vacuum and creation of bubbles.

24. The method according to Claim 22, wherein the piston is reciprocated at full speed in order to dislodge formed bubbles in the end portion of the bore.

25. A method of operating an automated, continuous and random access analytical system capable of simultaneously effecting multiple assays of a plurality liquid samples, comprising:
(a) introducing sample cups, reagent packs, and reaction vessels for performing said assays onto concentric carousels of a front end carousel, the reaction vessels being introduced to an outer carousel;
(b) identifying the reagent packs and sample cups;
(c) scheduling the assays;
(d) aligning the sample cups and reagent packs with a reaction vessel at a kitting station by rotating the respective carousels;
(e) flushing bubbles from a fluidics system by introducing fluid into an annulus defined by a piston and bore wall, the annulus closed at a first end of the bore by seal means between the bore wall and the piston and at a second end of the bore by a closed bore end and introducing fluid into the bore when the piston is withdrawn beyond fluid inlet and outlet means, flowing the fluid through the bore end and annulus and around the sides of the piston and out through a fluid outlet, creating a cross flow pattern proximal to the seal area across the end of the piston in one position and around the piston when the piston is moving in or out of the bore, and reciprocating the piston at least once to flush bubbles from the fluidics system;
(f) kitting a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent pack;
(g) transferring the kitted reaction vessel to a process carousel which is maintained under controlled environment conditions;
(h) pipetting the sample and various reagents into a reaction well of the reaction vessel, the amounts of reagent, sequencing of transfer and time spacing there between being predetermined by assay scheduling;
(i) incubating the pipetted sample and reagent mix;
(j) identifying and transferring the incubated mixture in the reaction well to one of at least two assay analytical stations;
(k) performing an analysis by reading the prepared reaction mixture and calibrating the reading; and
(l) recording the resulting assay reading analysis.
